(19) Europäisches Patentamt / European Patent Office / Office européen des brevets

(11) EP 4 520 832 A1

(12) **EUROPEAN PATENT APPLICATION**
published in accordance with Art. 153(4) EPC

(43) Date of publication:
**12.03.2025 Bulletin 2025/11**

(21) Application number: **23799507.1**

(22) Date of filing: **02.05.2023**

(51) International Patent Classification (IPC):
***C12N 15/86*** (2006.01) ***A61K 35/76*** (2015.01)
***A61K 48/00*** (2006.01) ***A61P 43/00*** (2006.01)
***C12N 5/10*** (2006.01) ***C12N 7/01*** (2006.01)
***C12N 15/12*** (2006.01) ***C12N 15/37*** (2006.01)
***C12N 15/48*** (2006.01) ***C12N 15/52*** (2006.01)
***C12N 15/63*** (2006.01) ***C12N 15/66*** (2006.01)
***C12N 15/861*** (2006.01) ***C12N 15/863*** (2006.01)
***C12N 15/864*** (2006.01) ***C12N 15/867*** (2006.01)
***C12N 15/869*** (2006.01) ***C40B 40/08*** (2006.01)

(52) Cooperative Patent Classification (CPC):
**A61K 35/76; A61K 48/00; A61P 43/00; C07K 14/025; C07K 14/15; C07K 14/435; C12N 5/10; C12N 7/00; C12N 15/52; C12N 15/63; C12N 15/66; C12N 15/86; C12N 15/861; C12N 15/863; C12N 15/864;** (Cont.)

(86) International application number:
**PCT/JP2023/017130**

(87) International publication number:
**WO 2023/214578 (09.11.2023 Gazette 2023/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.05.2022 JP 2022076058**

(71) Applicant: **Synplogen Co., Ltd.**
**Kobe-shi, Hyogo 650-0047 (JP)**

(72) Inventors:
- **SAITO, Shunsuke**
  **Kobe-shi, Hyogo 650-0047 (JP)**
- **TSUGE, Kenji**
  **Kobe-shi, Hyogo 650-0047 (JP)**
- **NISHIMURA, Yuya**
  **Kobe-shi, Hyogo 650-0047 (JP)**

(74) Representative: **Müller-Boré & Partner Patentanwälte PartG mbB**
**Friedenheimer Brücke 21**
**80639 München (DE)**

Remarks:
The complete document including Reference Table(s) and the Sequence Listing(s) can be downloaded from the EPO website

(54) **VIRUS-DERIVED CONSTRUCT PLASMID LIBRARY AND CONSTRUCTION OF SAME**

(57) In one aspect, the present disclosure provides a method for producing various virus-derived construct plasmids from starting plasmids (for example, virus-derived construct plasmids of the present disclosure). In one embodiment, a method for producing virus-derived construct plasmids of the present disclosure includes a step of preparing a set of starting plasmids containing at least of a part of nucleic acid sequences required to construct a virus-derived construct, the set of starting plasmids including a set of corresponding alternative unit nucleic acids, and the set of corresponding alternative unit nucleic acids including at least one set of corresponding alternative unit nucleic acids containing different nucleic acid sequences; a step of treating the set of starting plasmids with a restriction enzyme and preparing a mixed solution containing cleaved alternative unit nucleic acid fragments; a step of ligating the cleaved alternative unit nucleic acid fragments to form an integrated nucleic acid; and a step of bringing the integrated nucleic acid into contact with a transformed organism to form virus-de-

EP 4 520 832 A1

rived construct plasmids.

[FIG. 1]

FIG. 1

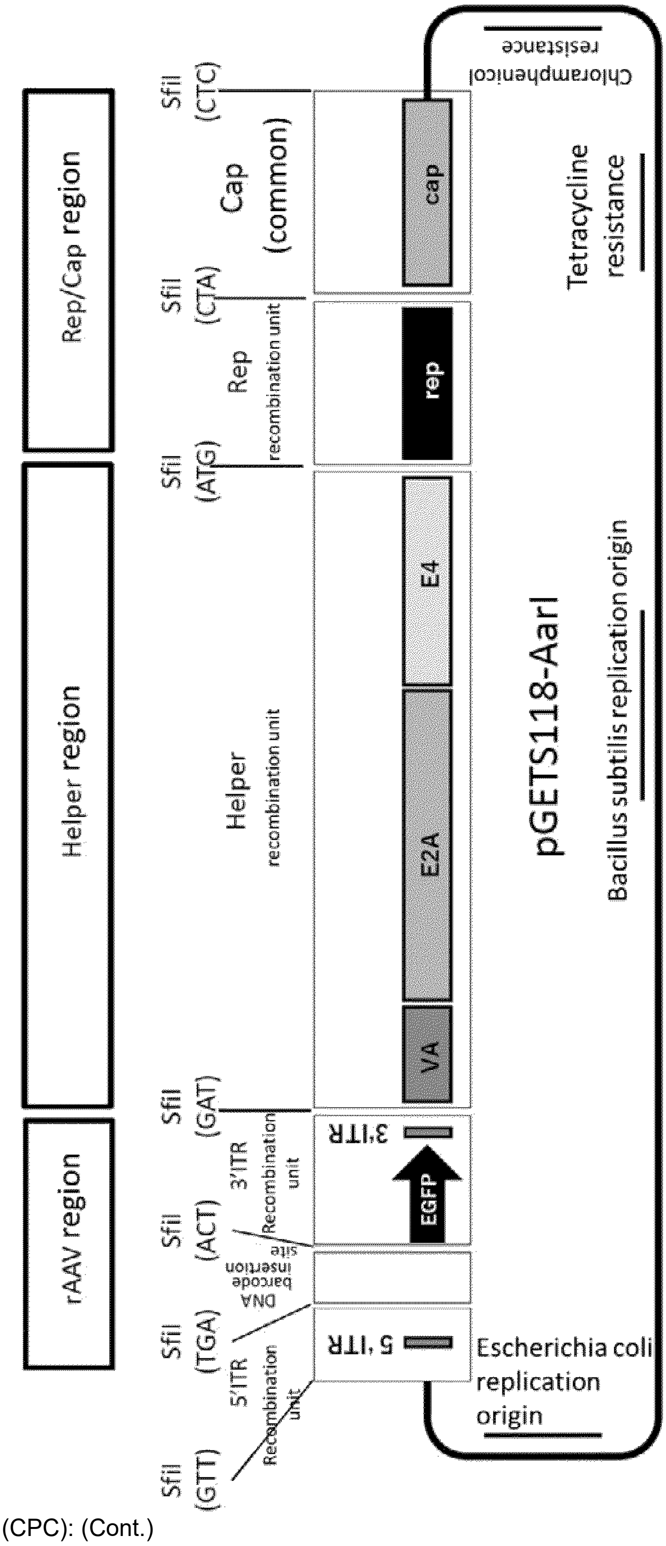


(52) Cooperative Patent Classification (CPC): (Cont.)
**C12N 15/867; C12N 15/869; C40B 40/08**

## Description

### Technical Field

The present disclosure provides a method for efficiently producing virus-derived construct plasmids. The present disclosure also provides a virus-derived construct plasmid library.

### Background Art

Various virus-derived constructs such as adenoviral vectors (Patent Literature 1) have been developed for gene therapy, vaccine therapy, and the like.

For the preparation of virus-derived constructs, it is usually necessary to introduce virus-derived construct plasmids into producer cells. Therefore, synthesis and construction of virus-derived construct plasmids are required. Virus-derived construct plasmids are usually individually designed and produced, and improvement of plasmid performance, such as the amount of virus-derived constructs produced, often involves a lot of effort.

### Citation List

### Patent Literature

Patent Literature 1: WO 2001/090392 A

### Summary of Invention

### Solution to Problem

The present inventors have found a method for efficiently producing various virus-derived construct plasmids. Based on this finding, the present disclosure provides a method for producing various virus-derived construct plasmids from starting plasmids. Furthermore, the present inventors provide a library of virus-derived construct plasmids produced by such a method.

Accordingly, the present disclosure provides the following.

(Item 1)

A method for producing a virus-derived construct plasmid, the method including:

a step of preparing a set of starting plasmids containing at least of a part of nucleic acid sequences required to construct a virus-derived construct, the set of starting plasmids including a set of corresponding alternative unit nucleic acids, and the set of corresponding alternative unit nucleic acids including at least one set of corresponding alternative unit nucleic acids containing different nucleic acid sequences;
a step of treating the set of starting plasmids with a restriction enzyme and preparing a mixed solution containing cleaved alternative unit nucleic acid fragments;
a step of ligating the cleaved alternative unit nucleic acid fragments to form an integrated nucleic acid; and
a step of bringing the integrated nucleic acid into contact with a transformed organism to form a virus-derived construct plasmid.

(Item 2)

The method according to any one of the above items, in which a population of virus-derived construct plasmids is produced.

(Item 3)

The method according to any one of the above items, in which at least one of the sets of starting plasmids contains a transcriptional start sequence that functions in an animal cell, and a virus-derived construct plasmid containing the transcriptional start sequence that functions in an animal cell is produced.

(Item 4)

The method according to any one of the above items, in which the set of starting plasmids includes three or more types of starting plasmids.

(Item 5)

The method according to any one of the above items, in which the set of corresponding alternative unit nucleic acids includes at least two sets of corresponding alternative unit nucleic acids containing different nucleic acid sequences.

(Item 6)

The method according to any one of the above items, further including a step of selecting a virus-derived construct plasmid having an excellent desired function from a population of the formed virus-derived construct plasmids.

(Item 7)

The method according to any one of the above items, in which the desired function is an amount of virus-derived construct produced.

(Item 8)

The method according to any one of the above items, in which the desired function is an amount of impurity mixed in a target virus-derived construct composition.

(Item 9)

The method according to any one of the above items, in which the impurity is selected from a coat protein that does not contain a nucleic acid sequence, a coat protein that contains an incomplete nucleic acid sequence, an incomplete coat protein, and an incomplete nucleic acid sequence.

(Item 10)

The method according to any one of the above items, in which the desired function is a purity of a target virus-derived construct in a target virus-derived construct composition.

(Item 11)

The method according to any one of the above items, further including a step of preparing a virus-derived construct plasmid containing a combination of parallel alternative unit nucleic acids of the selected virus-derived construct plasmids.

(Item 12)

The method according to any one of the above items, in which each of the sets of starting plasmids contains a nucleic acid sequence required to construct a virus-derived construct.

(Item 13)

The method according to any one of the above items, in which at least of a part of the starting plasmids are plasmids produced by the method according to Item 1.

(Item 14)

The method according to any one of the above items, in which the set of corresponding alternative unit nucleic acids includes at least two sets of corresponding alternative unit nucleic acids containing a nucleic acid sequence encoding at least of a part of different subtypes of the same gene.

(Item 15)

The method according to any one of the above items, in which each of the sets of starting plasmids contains three or more types of alternative unit nucleic acids containing at least of a part of the nucleic acid sequences required to construct a virus-derived construct.

(Item 16)

The method according to any one of the above items, in which the step of ligating to form the integrated nucleic acid includes adding a barcode nucleic acid.

(Item 17)

The method according to any one of the above items, in which each of the sets of starting plasmids contains the nucleic acid sequence required to construct a virus-derived construct of about 10 kb or more.

(Item 18)

The method according to any one of the above items, in which the nucleic acid sequence required to construct a virus-derived construct includes:

(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

(Item 19)

The method according to any one of the above items, in which the set of corresponding alternative unit nucleic acids includes a set of corresponding alternative unit nucleic acids encoding promoters whose types or directions are different.

(Item 20)

The method according to any one of the above items, in which the set of corresponding alternative unit nucleic acids includes a set of corresponding alternative unit nucleic acids containing a drug-responsive promoter and containing different nucleic acid sequences encoding a promoter.

(Item 21)

The method according to any one of the above items, in which the promoter includes a drug-responsive promoter.

(Item 22)

The method according to any one of the above items, in which the set of corresponding alternative unit nucleic acids includes at least two sets of corresponding alternative unit nucleic acids containing different nucleic acid sequences selected from:

(A) a nucleic acid sequence including a terminal repeat;
(B) a nucleic acid sequence encoding a packaging factor;
(C) a nucleic acid sequence encoding a structural protein;
(D) a nucleic acid sequence encoding a functional cofactor;
(E) a nucleic acid sequence encoding a promoter;
(F) a nucleic acid sequence encoding a desired gene; and
(G) a nucleic acid sequence involved in host cell biosynthesis.

(Item 23)

The method according to any one of the above items, in which the nucleic acid sequence involved in host cell biosynthesis contains a gene selected from the group consisting of SV40 Large T antigen (SV40LTA), TAX, Kras, Myc, MIR17HG, BCL-2, BCL-XL, PDIA2, XBP1, HSPA5, pyruvate carboxylase (PC), PDK, HIF1A, NRF2, and CPSF6.

(Item 24)

The method according to any one of the above items, in which the set of corresponding alternative unit nucleic acids includes at least two sets of corresponding alternative unit nucleic acids containing different nucleic acid sequences encoding a desired gene.

(Item 25)

The method according to any one of the above items, in which the set of corresponding alternative unit nucleic acids includes a set of corresponding alternative unit nucleic acids containing different nucleic acid sequences encoding a desired gene, and a set of corresponding alternative unit nucleic acids that are located adjacently upstream thereof and contain different nucleic acid sequences encoding a promoter.

(Item 26)

The method according to any one of the above items, in which the starting plasmid contains a nucleic acid sequence including two terminal repeats, and an alternative unit nucleic acid encoding the desired gene is present between the nucleic acid sequences including two terminal repeats.

(Item 27)

The method according to any one of the above items, in which the virus-derived construct is selected from a viral vector, a virus-like particle (VLP), an oncolytic virus, and a viral replicon.

(Item 28)

The method according to any one of the above items, in which the virus-derived construct is a virus-derived construct of a virus selected from the group consisting of an alphavirus, a vaccinia virus, a measles virus, an influenza virus, a vesicular stomatitis virus, a coronavirus, a Sindbis virus, a Semliki Forest virus, a herpes simplex virus, a retrovirus, a lentivirus, a rabies virus, a Sendai virus, an adeno-associated virus, an adenovirus, a reovirus, a coxsackievirus, and a Newcastle disease virus.

(Item 29)

The method according to any one of the above items, in which the virus-derived construct is a virus-derived construct of a virus selected from the group consisting of an adeno-associated virus, an adenovirus, a retrovirus, a lentivirus, a herpes simplex virus, and a Sendai virus.

(Item 30)

The method according to any one of the above items, in which the virus-derived construct is a virus-derived construct of a virus selected from the group consisting of an adeno-associated virus or a lentivirus.

(Item 31)

The method according to any one of the above items, in which the virus-derived construct is a viral vector of an adeno-associated virus.

(Item 32)

The method according to any one of the above items, in which the set of corresponding alternative unit nucleic acids includes a set of corresponding alternative unit nucleic acids containing different inverted terminal repeats (ITRs)

derived from any of adeno-associated virus serotypes 1 to 12 and variants thereof.

(Item 33)

The method according to any one of the above items, in which the starting plasmid contains a promoter, a desired gene, and a terminator from upstream between a 5' ITR and a 3' ITR.

(Item 34)

The method according to any one of the above items, in which the set of corresponding alternative unit nucleic acids includes a set of corresponding alternative unit nucleic acids containing different nucleic acid sequences encoding a functional cofactor derived from at least one of an adenovirus, a herpes virus, a papilloma virus, a bocavirus, and a simian virus.

(Item 35)

The method according to any one of the above items, in which the set of corresponding alternative unit nucleic acids includes a set of corresponding alternative unit nucleic acids containing different nucleic acid sequences encoding a functional cofactor derived from any of adenovirus serotypes 1 to 52 and variants thereof.

(Item 36)

The method according to any one of the above items, in which the functional cofactor includes at least one of adenoviruses E1A, E1B, E2A, E4, and VA, herpes viruses UL5, UL8, UL30, UL42, UL52, ICP0, ICP4, ICP8, and ICP22, papilloma viruses E1, E2, and E6, bocaviruses NP1, NS2, NS4, and BocaSR, and simian virus Large T antigen.

(Item 37)

The method according to any one of the above items, in which the set of corresponding alternative unit nucleic acids includes a set of corresponding alternative unit nucleic acids containing different nucleic acid sequences encoding the functional cofactor, and a set of corresponding alternative unit nucleic acids that are located adjacently upstream thereof and contain different nucleic acid sequences encoding a promoter.

(Item 38)

The method according to any one of the above items, in which the starting plasmid contains E2A, E4, and VA.

(Item 39)

The method according to any one of the above items, in which the set of corresponding alternative unit nucleic acids includes a set of corresponding alternative unit nucleic acids containing different nucleic acid sequences encoding rep.

(Item 40)

The method according to any one of the above items, in which the rep includes at least one of rep78, rep76, rep52, and rep40.

(Item 41)

The method according to any one of the above items, in which the set of corresponding alternative unit nucleic acids includes a set of corresponding alternative unit nucleic acids containing different nucleic acid sequences encoding the rep, and a set of corresponding alternative unit nucleic acids that are located adjacently upstream thereof and contain different nucleic acid sequences encoding a promoter.

(Item 42)

The method according to any one of the above items, in which the set of corresponding alternative unit nucleic

acids encoding rep includes a set of corresponding alternative unit nucleic acids containing different nucleic acid sequences derived from any of adeno-associated virus serotypes 1 to 12 and variants thereof.

(Item 43)

The method according to any one of the above items, in which the set of corresponding alternative unit nucleic acids includes a set of corresponding alternative unit nucleic acids containing different nucleic acid sequences encoding cap.

(Item 44)

The method according to any one of the above items, in which the cap includes at least one of VP1, VP2, VP3, AAP, and MAAP.

(Item 45)

The method according to any one of the above items, in which the set of corresponding alternative unit nucleic acids includes a set of corresponding alternative unit nucleic acids containing different nucleic acid sequences encoding the cap, and a set of corresponding alternative unit nucleic acids that are located adjacently upstream thereof and contain different nucleic acid sequences encoding a promoter.

(Item 46)

The method according to Item 43, in which the set of corresponding alternative unit nucleic acids encoding cap includes a set of corresponding alternative unit nucleic acids containing different nucleic acid sequences derived from any of adeno-associated virus serotypes 1 to 12 and variants thereof.

(Item 47)

The method according to any one of the above items, in which the starting plasmid contains a 5' ITR, a promoter, a desired gene, a 3' ITR, rep, cap, and a functional cofactor.

(Item 48)

The method according to any one of the above items, in which the starting plasmid contains a 5' ITR, a 3' ITR, rep, and cap, one of the rep and the cap is located downstream of a region sandwiched between the 5' ITR and the 3' ITR, and the other one of the rep and the cap is located upstream of the region sandwiched between the 5' ITR and the 3' ITR.

(Item 49)

The method according to any one of the above items, in which the starting plasmid contains two ITRs and another portion of the nucleic acid sequence required to construct a virus-derived construct, and the another portion is outside a region sandwiched between the two ITRs.

(Item 50)

The method according to any one of the above items, in which the starting plasmid contains a 5' ITR, a promoter, a desired gene, a 3' ITR, rep, cap, and a functional cofactor in this order.

(Item 51)

The method according to any one of the above items, in which the starting plasmid contains a first promoter, a first rep, a second promoter, a second rep, a third promoter, and cap in this order.

(Item 52)

The method according to any one of the above items, in which the virus-derived construct is a viral vector of a lentivirus.

(Item 53)

The method according to any one of the above items, in which the set of corresponding alternative unit nucleic acids includes a set of corresponding alternative unit nucleic acids containing different long terminal repeats (LTRs) derived from a lentivirus or variants thereof.

(Item 54)

The method according to any one of the above items, in which the starting plasmid contains a promoter, a desired gene, and a terminator from upstream between a 5' LTR and a 3' LTR.

(Item 55)

The method according to any one of the above items, in which the starting plasmid contains two or more sets of the promoter, the desired gene, and the terminator.

(Item 56)

The method according to any one of the above items, in which the set of corresponding alternative unit nucleic acids includes a set of corresponding alternative unit nucleic acids containing different nucleic acid sequences encoding a functional cofactor selected from tat and rev.

(Item 57)

The method according to any one of the above items, in which the set of corresponding alternative unit nucleic acids includes a set of corresponding alternative unit nucleic acids containing different nucleic acid sequences encoding the functional cofactor, and a set of corresponding alternative unit nucleic acids that are located adjacently upstream thereof and contain different nucleic acid sequences encoding a promoter.

(Item 58)

The method according to any one of the above items, in which the set of corresponding alternative unit nucleic acids includes a set of corresponding alternative unit nucleic acids containing different nucleic acid sequences encoding a structural protein selected from gag, pol, VSV-G, and env.

(Item 59)

The method according to any one of the above items, in which the set of corresponding alternative unit nucleic acids includes a set of corresponding alternative unit nucleic acids containing different nucleic acid sequences encoding the structural protein, and a set of corresponding alternative unit nucleic acids that are located adjacently upstream thereof and contain different nucleic acid sequences encoding a promoter.

(Item 60)

The method according to any one of the above items, in which the virus-derived construct is a viral vector of an adenovirus.

(Item 61)

The method according to any one of the above items, in which the set of corresponding alternative unit nucleic acids includes a set of corresponding alternative unit nucleic acids containing different inverted terminal repeats (ITRs) derived from an adenovirus.

(Item 62)

The method according to any one of the above items, in which the starting plasmid contains a promoter, a desired gene, and a terminator from upstream between a 5' ITR and a 3' ITR.

(Item 63)

The method according to any one of the above items, in which the starting plasmid contains two or more sets of the promoter, the desired gene, and the terminator.

(Item 64)

The method according to any one of the above items, in which the set of corresponding alternative unit nucleic acids includes a set of corresponding alternative unit nucleic acids containing different nucleic acid sequences encoding a structural protein selected from L1, L2, L3, L4, and L5.

(Item 65)

The method according to any one of the above items, in which the set of corresponding alternative unit nucleic acids includes a set of corresponding alternative unit nucleic acids containing different nucleic acid sequences encoding the structural protein, and a set of corresponding alternative unit nucleic acids that are located adjacently upstream thereof and contain different nucleic acid sequences encoding a promoter.

(Item 66)

The method according to any one of the above items, in which the set of corresponding alternative unit nucleic acids encoding a structural protein includes a set of corresponding alternative unit nucleic acids containing different nucleic acid sequences derived from any of adenovirus serotypes 1 to 52 and variants thereof.

(Item 67)

The method according to any one of the above items, in which the set of corresponding alternative unit nucleic acids includes a set of corresponding alternative unit nucleic acids containing different nucleic acid sequences encoding a functional cofactor selected from E1A, E1B, E2A, E2B, and E4.

(Item 68)

The method according to any one of the above items, in which the set of corresponding alternative unit nucleic acids includes a set of corresponding alternative unit nucleic acids containing different nucleic acid sequences encoding the functional cofactor, and a set of corresponding alternative unit nucleic acids that are located adjacently upstream thereof and contain different nucleic acid sequences encoding a promoter.

(Item 69)

The method according to any one of the above items, in which the set of corresponding alternative unit nucleic acids encoding a functional cofactor includes a set of corresponding alternative unit nucleic acids containing different nucleic acid sequences derived from any of adenovirus serotypes 1 to 52 and variants thereof.

(Item 70)

The method according to any one of the above items, in which the virus-derived construct plasmid enables production of a virus-derived construct in a producer cell into which the virus-derived construct plasmid is introduced alone.

(Item 71)

A method for constructing a virus-derived construct plasmid library by performing the method according to any one of the above items.

(Item 72)

A method for constructing a virus-derived construct plasmid library by performing the method according to any one of the above items, in which the set of corresponding alternative unit nucleic acids includes a plurality of sets of corresponding alternative unit nucleic acids containing different nucleic acid sequences, and provides 64 or more

combinations of parallel alternative unit nucleic acids.

(Item 73)

A virus-derived construct plasmid or a virus-derived construct plasmid library produced by the method according to any one of the above items.

(Item 74)

A virus-derived construct plasmid library containing a virus-derived construct plasmid containing three or more alternative unit nucleic acids as a series of parallel alternative unit nucleic acids, in which the virus-derived construct plasmid library contains a plurality of sets of corresponding alternative unit nucleic acids containing different nucleic acid sequences, and the parallel alternative unit nucleic acids on each virus-derived construct plasmid of the plurality of sets of corresponding alternative unit nucleic acids containing different nucleic acid sequences have a length of about 10 kb or more.

(Item 74A)

A virus-derived construct plasmid library containing a plurality of virus-derived construct plasmids, in which different portions in a combination in the plurality of virus-derived construct plasmids contain a nucleic acid sequence that is a promoter or a functional cofactor.

(Item 74B)

The virus-derived construct plasmid library according to any one of the above items, in which the plurality of virus-derived construct plasmids in the virus-derived construct plasmid library contain elements that constitute the different portions in the combination, the elements being differ from one another in a serotype of a virus from which they are derived.

(Item 74C)

The virus-derived construct plasmid library according to any one of the above items, in which the elements that constitute the different portions in the combination of the plurality of virus-derived construct plasmids in the virus-derived construct plasmid library contain promoters, and the promoters in the plurality of virus-derived construct plasmids include promoters operably linked to genes that are different from each other in nature.

(Item 75)

The virus-derived construct plasmid library according to any one of the above items, in which the plurality of sets of corresponding alternative unit nucleic acids containing different nucleic acid sequences include a set of corresponding alternative unit nucleic acids containing at least one different nucleic acid sequence of:

(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

(Item 76)

The virus-derived construct plasmid library according to any one of the above items, in which each virus-derived construct plasmid contains:

(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

(Item 77)

The virus-derived construct plasmid library according to any one of the above items, in which the plurality of sets of corresponding alternative unit nucleic acids containing different nucleic acid sequences contain an alternative unit nucleic acid having a GC content of 75% or more.

(Item 78)

The virus-derived construct plasmid library according to any one of the above items, in which the plurality of sets of corresponding alternative unit nucleic acids containing different nucleic acid sequences include a plurality of sets of corresponding alternative unit nucleic acids that allow for 64 or more different parallel alternative unit nucleic acid combinations, and each virus-derived construct plasmid contains the same number of parallel alternative unit nucleic acids.

(Item 79)

The virus-derived construct plasmid library according to any one of the above items, in which each virus-derived construct plasmid contains a barcode nucleic acid sequence.

(Item 80)

A virus-derived construct plasmid contained in the virus-derived construct plasmid library according to any one of the above items.

(Item 81)

A method for producing virus-derived construct, the method including:

a step of preparing a virus-derived construct plasmid by the method according to any one of the above items; and
a step of introducing the virus-derived construct plasmid into a producer cell to form a virus-derived construct.

(Item 82)

A method for producing a virus-derived construct library, the method including:

a step of preparing a population of virus-derived construct plasmids by the method according to any one of the above items; and
a step of introducing the population of virus-derived construct plasmids into a producer cell and forming a virus-derived construct to construct a virus-derived construct library.

(Item 83)

The method according to any one of the above items, in which the step of introducing the virus-derived construct plasmid into the producer cell includes introducing a single virus-derived construct plasmid into a producer cell.

(Item 84)

The method according to any one of the above items, in which at least some ofat least of a part of the nucleic acids contained in the virus-derived construct plasmid are incorporated into a chromosome of the producer cell.

(Item 85)

A producer cell prepared by the method according to any one of the above items.

(Item 86)

A virus-derived construct produced by the method according to any one of the above items.

(Item 87)

A virus-derived construct-containing composition produced by the method according to any one of the above items.

In the present disclosure, it is intended that one or a plurality of features can be provided in further combination in addition to the specified combination. Still further embodiments and advantages of the present disclosure will be appreciated by those skilled in the art upon reading and understanding the following detailed description, as necessary.

Advantageous Effects of Invention

According to the present disclosure, it is possible to efficiently produce virus-derived construct plasmids having improved performance.

Brief Description of Drawings

FIG. 1 illustrates an outline of a structure of a seed plasmid used in Examples.

FIG. 2 illustrates an outline of structures of five types of seed plasmids produced first.

FIG. 3 illustrates an outline of structures of eight types of seed plasmids to which additional seed plasmids are added.

FIG. 4 illustrates the number of genomic copies of rAAV produced from each of plasmids Control, #20, #24, and #46. The vertical axis indicates the number of genomic copies per 1 mL.

FIG. 5 illustrates a measurement result of purified rAAV produced from plasmid #46 by a mass spectrometry method. The horizontal axis indicates the measured mass (kDa) and the vertical axis indicates the counts detected at the corresponding mass.

FIG. 6 illustrates examples of construction of a plasmid assumed for a structure of a virus-derived construct plasmid.

FIG. 7 illustrates examples of construction of a plasmid assumed for a structure of a virus-derived construct plasmid.

FIG. 8 illustrates examples of construction of a plasmid assumed for a structure of a virus-derived construct plasmid.

FIG. 9 illustrates examples of construction of a plasmid assumed for a structure of a virus-derived construct plasmid.

FIG. 10 illustrates examples of construction of a plasmid assumed for a structure of a virus-derived construct plasmid.

FIG. 11 illustrates an outline of a structure of seed plasmids formed for producing a combinatorial library in Example 4.

FIG. 12 illustrates an AAV particle producing ability of the viral vector plasmid clones obtained in Example 4. The horizontal axis indicates the clone number, and the vertical axis indicates the amount of AAV particles produced (the number of genomic copies).

FIG. 13 illustrates an outline of a structure of seed plasmids formed for producing a combinatorial library in Example 5.

FIG. 14 illustrates an AAV particle producing ability of the viral vector plasmid clones obtained in Example 5. The horizontal axis indicates the clone number, and the vertical axis indicates the amount of AAV particles produced (the number of genomic copies).

Description of Embodiments

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used As used herein are to be understood as being used in the sense commonly used in the art unless otherwise stated. Therefore, unless defined otherwise, all technical and scientific terms used As used herein have the same meaning as commonly understood by those skilled in the art to which the present disclosure pertains. In the case of conflict, the present specification (including definitions) will control.

Hereinafter, the definitions of the terms and/or basic technical concepts that are particularly used As used herein will be described as appropriate.

As used herein, the term "plasmid" refers to circular DNA that is present separately from chromosomes in cells or that is present separately from chromosomes when introduced into cells. Since a method of the present disclosure can produce plasmids using plasmids as starting materials, the plasmids used as starting materials in the method may be referred to as "starting plasmids", and the produced plasmids may be referred to as "product plasmids". The totality of starting plasmids present in one reaction solution or for forming one reaction solution may be referred to as "set of starting plasmids" As used herein.

The Ordered Gene Assembly in Bacillus subtilis (OGAB) method used in the method of the present disclosure is a method of incorporating an integrated nucleic acid into a transformed organism to produce circular plasmids in the organism (details are described elsewhere As used herein). Any organism capable of incorporating acyclic long nucleic

acids and producing plasmids can be used in the OGAB method, and such an organism is referred to as "transformed organism" As used herein. The nucleic acid to be incorporated into the transformed organism is referred to as "integrated nucleic acid" As used herein, typically, the integrated nucleic acid has a tandem repeat-like structure wherein nucleic acid units having the same sequence repeatedly appear in the same direction, and the use of the integrated nucleic acid having such a structure can promote the production of plasmids in the transformed organism. As used herein, the term "unit nucleic acid" refers to a nucleic acid fragment having a portion of the sequence constituting the sequence of the integrated nucleic acid or a portion that produces such a nucleic acid fragment, and typically refers to a nucleic acid fragment containing a protruding sequence generated by a restriction enzyme treatment at both ends or a nucleic acid region sandwiched between two protruding sequences generated by a restriction enzyme treatment.

[Chem. 1]

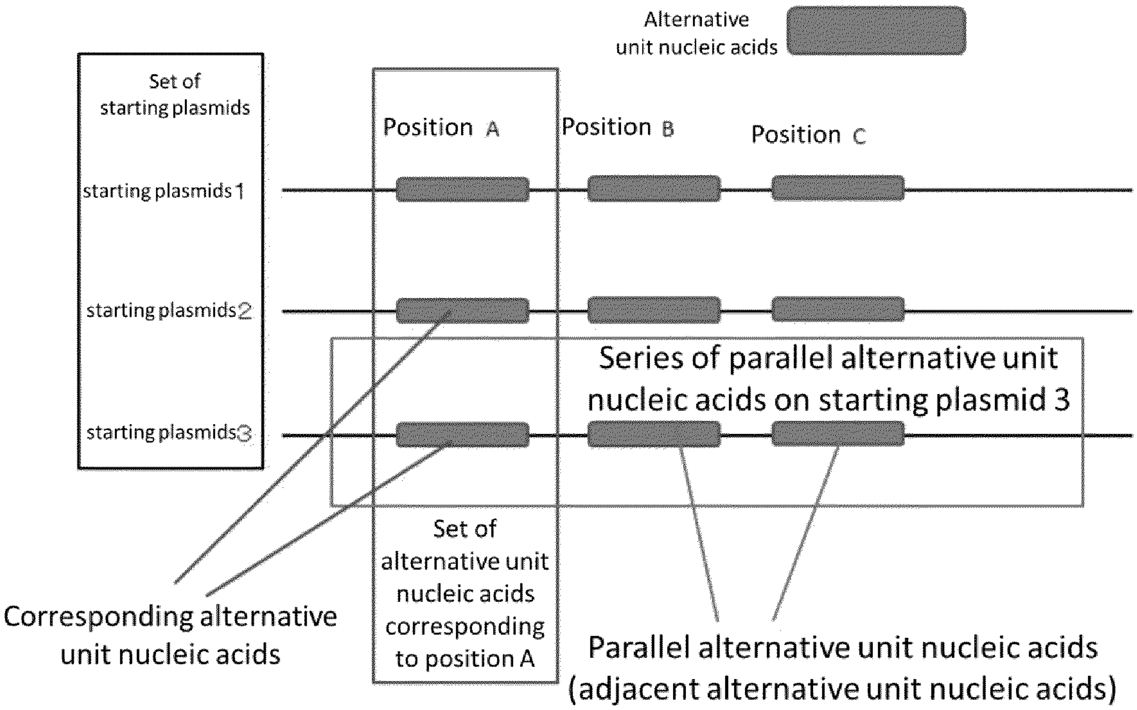


As used herein, the term "corresponding" between a plurality of unit nucleic acids refers to a relationship in which these unit nucleic acids that are nucleic acid fragments contain the same protruding sequence, or a relationship in which the same protruding sequence is generated when a plurality of nucleic acid molecules (typically, plasmids) each containing these unit nucleic acids are treated with a specific restriction enzyme. As used herein, unit nucleic acids corresponding to each other are referred to as "alternative unit nucleic acids", when fragmented alternative unit nucleic acids are integrated to produce an integrated nucleic acid and a plasmid is formed by the OGAB method, the alternative unit nucleic acids can be incorporated into the same position on the plasmid (for example, unit nucleic acids of the same order counted from the transcription initiation point, and the like). The base sequences other than the protruding sequences of the plurality of alternative unit nucleic acids may be the same or different. The totality of alternative unit nucleic acids present in one reaction solution or in a set of starting plasmids for forming one reaction solution may be referred to as "set of alternative unit nucleic acids" As used herein. The totality of alternative unit nucleic acids is also used as a set of alternative unit nucleic acids at position A or the like.

As used herein, the term "parallel" of a plurality of unit nucleic acids refers to a relationship in which these unit nucleic acids that are nucleic acid fragments contain different protruding sequences, or a relationship in which different protruding sequences are generated when nucleic acid molecules (typically, plasmids) containing these unit nucleic acids are treated with a specific restriction enzyme. Typically, the parallel unit nucleic acids are present on the same plasmid. As used herein, the phrase "two unit nucleic acids are adjacent to each other" refers to a relationship in which the unit nucleic acids contain complementary protruding sequences, or a relationship in which complementary protruding sequences are generated when nucleic acid molecules (typically, plasmids) containing these unit nucleic acids are treated with a specific restriction enzyme, and typically refers to a relationship in which the unit nucleic acids are located on both sides of a

cleavage position in a restriction enzyme treatment. The totality of parallel unit nucleic acids present on a plasmid or generated by treating a plasmid with a restriction enzyme may be referred to as "series of parallel unit nucleic acids" As used herein. The totality of parallel unit nucleic acids is also used as a series of parallel unit nucleic acids and the like on a starting plasmid 1.

As used herein, a nucleic acid that can be defined by a terminal sequence or a protruding sequence of a nucleic acid molecule, such as an alternative unit nucleic acid, can refer to any of the form of a nucleic acid fragment having a terminal portion or a protruding portion of the sequence and a partial region in the nucleic acid molecule that can potentially form the terminal portion or the protruding portion, unless otherwise specified. When described as a "nucleic acid fragment", it actually has a terminal portion or a protruding portion of the sequence.

As used herein, the term "Bacillus subtilis" refers to an aerobic, gram-positive, catalase-positive bacterium that is commonly present in soil and plants and is present in the gastrointestinal tract of ruminants and humans, and refers to any bacterium having no pathogenicity and having a natural transformation ability, including the scientific name Bacillus subtilis or Bacillus amyloliquefaciens, Bacillus licheniformis, Bacillus pumilus, or the like, which is a bacterium belonging to the genus Bacillus that is regarded as being a spore-forming bacteria, which has a size of 0.7 to 0.8 $\times$ 2.3 $\mu$m, is mesophilic, and has an optimal growth temperature of 25 to 40°C. In a preferred embodiment, Bacillus subtilis is Marburg 168 strain or its derivative strain, RM125 strain, which is a strain with a high natural transformation ability in Bacillus subtilis. The 168 strain is the most genetically studied gram-positive bacterium and is available from the American Type Culture Collection (ATCC) and the like together with the RM125 strain, and can be suitably used in the present disclosure because it is harmless to humans and animals itself and the OGAB method is known to be applicable.

As used herein, the term "library" refers to a population of homogenous substances (plasmids, viral vectors, and the like) having similar structures. Examples of the similar structure include a structure having a similar base sequence length (for example, a difference between the base lengths is within 10%), a structure in which a series of parallel unit nucleic acids has the same number of unit nucleic acids, and a structure in which different portions have the same function (for example, different serotypes of the same gene and promoters that function in the same host cell).

As used herein, the term "packaging cell" refers to a cell for producing virus-derived construct plasmids.

As used herein, the term "virus-derived construct plasmid" refers to a plasmid for producing a virus-derived construct in a producer cell containing genes loaded on the virus-derived construct. The virus-derived construct plasmid may contain a sequence of a gene (desired gene) to be expressed in a target cell of a virus-derived construct between two terminal repeats, may further contain a promoter, and may further contain other elements (for example, an enhancer, a terminator, or the like).

As used herein, the term "producer cell" means a cell capable of producing a desired virus-derived construct, and may be a cell in which a gene required for producing a virus-derived construct is expressed from a chromosome and an introduced plasmid. A desired virus-derived construct can be produced by introducing the virus-derived construct plasmids of the present disclosure into producer cells. For example, in the case of an adeno-associated virus (AAV) vector, E1A and E1B are required for production thereof, but since HEK293 has E1A and E1B, E1A and E1B can be removed from a helper plasmid. Other cells also function as producer cells when modified to have such functional cofactors.

As used herein, the term "animal cell" refers to a cell obtained from an animal (human, mouse, rat, monkey, dog, or the like) or a cell obtained by modifying a cell obtained from an animal.

As used herein, the term "virus-derived construct" refers to a construct containing a nucleic acid construct at least partially having a structure derived from a virus or a protein produced therefrom. Examples of the virus-derived construct include a viral vector, a virus-like particle (VLP), an oncolytic virus (including a virus modified to proliferate only in cancer cells), and a viral replicon (self-replicating viral genome lacking the ability to produce viral particles).

As used herein, the term "viral vector" refers to a construct that at least partially has a structure derived from a virus and is capable of introducing a nucleic acid into a target cell. Typically, the viral vector is in the form of viral particles containing nucleic acids containing viral structural proteins and heterologous genes.

As used herein, the term "nucleic acid sequence required to construct a virus-derived construct" refers to a nucleic acid sequence (for example, a combination of nucleic acid sequences) that enables a producer cell containing the nucleic acid sequence to produce a virus-derived construct. In one embodiment, the nucleic acid sequence required to construct a virus-derived construct includes a nucleic acid sequence encoding a structural protein of a virus, a nucleic acid sequence encoding a packaging factor, and a nucleic acid sequence encoding two terminal repeats of a virus and a functional cofactor. The term "nucleic acid sequence encoding a functional cofactor" may include or consist essentially of, for example, a nucleic acid sequence encoding a structural protein of a virus, a nucleic acid sequence encoding a packaging factor, a nucleic acid sequence required to construct any viral-derived construct other than two viral terminal repeats of a virus. Each of these nucleic acid sequences can vary for each virus-derived construct. These details are described elsewhere As used herein. In a preferred embodiment, as the nucleic acid sequence required to construct a virus-derived construct, an advantageous sequence common to AAV and adenovirus can be utilized, and more preferably, an advantageous sequence for AAV can be utilized. The advantageous sequence common to such AAV and adenovirus is

characterized by containing a desired gene between inverted terminal repeats (ITRs) and optionally a functional cofactor, rep, and cap, or L1, L2, L3, L4, and L5, and the advantageous sequence for AAV contains a desired gene between ITRs and optionally a functional cofactor, rep, and cap. The arrangement of the functional cofactor, rep, and cap has the characteristic that it may be outside the two ITRs. For example, examples thereof include, but are not limited to, sequences of specific functional cofactors E1A, E1B, E2A, E2B, E3, and E4.

As used herein, the term "packaging factor" includes a protein that encapsulates the genome of a virus in a structural protein, and examples thereof include rep and ψ. Without intending to be limiting, a packaging factor derived from any of adenovirus serotypes 1 to 52 or adeno-associated virus serotypes 1 to 12, or variants thereof (rh10, DJ, DJ/8, PHP.eB, PHP.S, AAV2-retro, AAV2-QuadYF, AAV2.7m8, AAV6.2, rh.74, AAV2.5, AAV-TT, Anc80, and the like) can be utilized. The protein may be either naturally occurring or artificially mutated. The "artificial mutated" protein can be produced by, for example, introducing an appropriate mutation into a naturally occurring sequence described in a known literature.

As used herein, the term "structural protein" refers to a protein produced from a gene of a virus, at least a portion of which is present on the surface of the virus or a virus-derived construct (for example, crossing an envelope). A structural protein can be responsible for infectivity into cells.

As used herein, the term "capsid" refers to a protein produced from a gene of a virus (encapsulated in an envelope, as necessary) that is present on the surface of the virus or a virus-derived construct, and is also referred to as "capsid protein".

As used herein, the term "repetitive sequence" or "tandem repeat" (a nucleic acid sequence) is a generic term for a nucleic acid sequence of a biological genome in which the same sequence is repeatedly (particularly several times or more) found. Any repetitive sequence used in the art can be used in the present disclosure. Typically, a promoter sequence appears repeatedly.

As used herein, the term "terminal repeat" is a generic term for those present at the terminal among those in which the same sequence is found repeatedly (particularly several times or more) in the nucleic acid sequence of the biological genome. Examples of the terminal repeat include, but are not limited to, an inverted terminal repeat (ITR) or a long terminal repeat (LTR). A terminal repeat derived from any of adenovirus serotypes 1 to 52 or adeno-associated virus serotypes 1 to 12, or variants thereof (rh10, DJ, DJ/8, PHP.eB, PHP.S, AAV2-retro, AAV2-QuadYF, AAV2.7m8, AAV6.2, rh.74, AAV2.5, AAV-TT, Anc80, and the like) can be utilized. The terminal repeat may be either naturally occurring or artificially mutated. The "artificial mutated" protein can be produced by, for example, introducing an appropriate mutation into a naturally occurring sequence described in a known literature.

As used herein, the term "functional cofactor" refers to a gene that supports amplification of a virus that cannot proliferate alone. In the present disclosure, examples of the functional cofactor include, but are not limited to, a nucleic acid sequence encoding a packaging factor of the virus, and a nucleic acid sequence required to construct, proliferate, enhance activity, or reduce toxicity of any virus-derived construct other than the two terminal repeats of the virus. Examples of the functional cofactor that can be used include, but are not limited to, E1A, E1B, E2A, E2B, E4, RPE, WRPE, PPT, oPRE, an enhancer, an insulator, and a silencer sequence.

As used herein, the term "loaded nucleic acid" means a nucleic acid carried by a virus-derived construct (a viral vector or the like). Whether or not it is a loaded nucleic acid can be confirmed by treating a virus-derived construct preparation with DNase to decompose the nucleic acid outside the viral particle, inactivating the DNase, and then confirming the nucleic acid extracted from the viral particle. Whether or not it is a loaded nucleic acid can also be confirmed by examining the sequence of the resulting nucleic acid product (preferably a full-length or a length close to the full-length).

As used herein, the term "host cell" refers to a cell (including the progeny of such a cell) into which an exogenous nucleic acid or protein or viral or virus-derived construct is introduced.

As used herein, the terms "protein", "polypeptide", and "peptide" are used interchangeably, and refer to an amino acid polymer of any length. The polymer may be linear, branched, or cyclic. An amino acid may be a naturally-occurring, non-naturally occurring, or modified amino acid. The term also encompasses naturally or artificially modified polymers. Examples of such a modification include disulfide bond formation, glycosylation, lipidation, acetylation, phosphorylation, and any other manipulation or modification (for example, conjugation with a labeling component).

As used herein, the terms "polynucleotide" and "nucleic acid" are used interchangeably, and refer to a polymer of nucleotides of any length. Examples of the nucleic acid include DNA, RNA, cDNA, mRNA, rRNA, tRNA, microRNA (miRNA), and lncRNA. The term also includes a "polynucleotide derivative". The "polynucleotide derivative" refers to a polynucleotide containing a nucleotide derivative or having an unusual internucleotidic linkage. The "nucleotide derivative" refers to a nucleotide having a structure different from a normal nucleotide used in natural DNA or RNA, and examples thereof include a locked nucleic acid (LNA), an ethylene nucleic acid such as a 2'-O,4'-C-ethylene-bridged nucleic acid (ENA), other bridged nucleic acids (BNAs), a hexitol nucleic acid (HNA), an amido-bridged nucleic acid (AmNA), a morpholino nucleic acid, tricyclo-DNA (tcDNA), a polyether nucleic acid (see, for example, U.S. Patent No. 5,908,845), and a cyclohexene nucleic acid (CENA). Examples of the unusual internucleotidic linkage include an internucleotidic linkage in which a phosphate diester linkage is converted to a phosphorothioate linkage, an internucleotidic linkage in

which a phosphate diester linkage is converted to an N3'-P5' phosphoramidate linkage, and an internucleotidic linkage in which ribose and a phosphate diester linkage are converted to peptide nucleic acid linkages.

Unless otherwise indicated, a particular nucleic acid sequence is also intended to encompass a conservatively modified variant (for example, a degenerate codon substituent) and a complementary sequence thereof, as well as the sequence explicitly indicated. Specifically, the degenerate codon substituent can be achieved by creating a sequence in which the third position of one or more selected (or all) codons is substituted with a mixed-base and/or deoxyinosine residue. For example, variants based on specific wild-type sequences, such as adenovirus serotypes 1 to 52 and adeno-associated virus serotypes 1 to 12, contain, in addition to variants (for example, rh10, DJ, DJ/8, PHP.eB, PHP.S, AAV2-retro, AAV2-QuadYF, AAV2.7m8, AAV6.2, rh.74, AAV2.5, AAV-TT, Anc80, and the like), nucleic acids containing a sequence having at least 70%, at least 80%, at least 85%, at least 90%, at least 95%, at least 96%, at least 97%, at least 98%, at least 99%, or at least 99.5% sequence identity to the original sequence.

As used herein, the term "gene" refers to a nucleic acid portion that performs a certain biological function. Examples of the biological function include coding a polypeptide or protein, coding protein-non-coding functional RNA (rRNA, tRNA, microRNA (miRNA), lncRNA, or the like), controlling the production of a polypeptide, protein, or protein-non-coding functional RNA, specifically binding to a specific protein, and controlling the cleavage a nucleic acid or replication. Therefore, As used herein, in addition to the nucleic acid portion encoding a protein or non-protein-coding functional RNA, the gene contains a transcriptional/translational regulatory sequence such as a promoter, a terminator, an enhancer, an insulator, a silencer, an origin of replication, or an internal ribosome entry site, and a nucleic acid portion required for packaging into viral particles. As used herein, the term "gene product" may refer to a polypeptide, protein, or non-protein coding functional RNA encoded by a gene.

As used herein, the fact that two genes are cis refers to that the genes are present on the same nucleic acid molecule or a nucleic acid molecule of a complementary strand (in the case of a double-stranded nucleic acid). In addition, As used herein, the fact that two genes are trans refers to that, in a cell (in an organism), these genes are not present on the same nucleic acid molecule or a nucleic acid molecule of a complementary strand (in the case of a double-stranded nucleic acid). For example, the gene present on the genome and the gene on the nucleic acid introduced with the virus-derived construct can be trans. As necessary, whether or not two genes are trans can be determined in a state at the time when the two genes become simultaneously present in a cell (for example, introduction of a nucleic acid into a cell).

When referring to the number of genes As used herein, one gene refers to a gene that contains a continuous sequence in a form that is normally (most frequent or 50% or more probability) present on the genome of an organism. For example, two exons encoding a protein can be two genes. For example, when the promoter sequence and the sequence encoding the protein form a continuous sequence, the nucleic acid portion containing the promoter sequence and the sequence encoding the protein can be one gene. For example, when a protein that becomes functional when cleaved is encoded by a continuous sequence on the genome, the protein can be encoded by one gene. When referring to a gene in terms of function, the nucleic acid sequence does not need to be a continuous sequence, for example, a plurality of exons encoding a protein are collectively referred to as genes of the protein.

As used herein, the term "deficiency" of a gene refers to that the nucleic acid does not contain the gene or contain a gene that is modified so as not to perform the normal function (for example, the function of producing a functional protein) of the gene.

As used herein, the term "operably linked" refers to the placement of expression (actuation) of a desired sequence under the control of a transcriptional/translational regulatory sequence (for example, a promoter, an enhancer, or the like) or a translational regulatory sequence. In order for a promoter to be operably linked to a gene, the promoter is usually arranged immediately upstream of the gene, but not necessarily adjacent.

As used herein, the term "transcriptional/translational regulatory sequence" collectively refers to a promoter sequence, a polyadenylation signal, a transcription termination sequence, an upstream regulatory domain, an origin of replication, an enhancer, IRES, and the like, which act in combination to enable the replication, transcription, and translation of a coding sequence in a recipient cell. Not all of these transcriptional/translational regulatory sequences need to be present, as long as replication, transcription, and translation of the selected coding sequence are possible in a suitable host cell. Those skilled in the art can easily identify a regulatory nucleic acid sequence from public information. Furthermore, those skilled in the art can identify a transcriptional/translational regulatory sequence that can be applied for use purposes, for example, in vivo, ex vivo, or in vitro.

As used herein, the term "promoter" refers to a segment of a nucleic acid sequence that controls transcription of an operably linked nucleic acid sequence. The promoter contains a specific sequence that is sufficient for recognition, binding, and transcription initiation by RNA polymerase. The promoter may contain a sequence that regulates recognition, binding, or transcription initiation by RNA polymerase.

As used herein, the term "enhancer" refers to a segment of a nucleic acid sequence that has the function of increasing the expression efficiency of a gene of interest.

As used herein, the term "silencer" refers to a segment of a nucleic acid sequence that has the function of reducing the expression efficiency of a gene of interest, contrary to an enhancer.

As used herein, the term "insulator" refers to a segment of a nucleic acid sequence that has the function of cis-regulation, which regulates the expression of genes at distant locations on the sequence of DNA.

As used herein, the term "terminator" refers to a segment of a nucleic acid sequence that is located downstream of a region coding a protein and is involved in the termination of transcription when the nucleic acid is transcribed into mRNA.

As used herein, the term "origin of replication" refers to a segment of a nucleic acid sequence at which replication begins by partial unwinding of the DNA double helix when a protein (for example, initiator DnaA protein or the like) that recognizes the nucleic acid sequence binds or when RNA is synthesized.

As used herein, an internal ribosome entry site ("IRES") refers to a nucleic acid segment that promotes entry or retention of ribosomes during translation of a nucleic acid sequence downstream thereof.

As used herein, the term "homology" of nucleic acids refers to a degree of identity of two or more nucleic acid sequences with respect to one another, and having "homology" generally refers to having a high degree of identity or similarity. Therefore, the identity or similarity of sequences is higher as homology of two nucleic acids is high. The "similarity" is a numerical value calculated for a similar base in addition to identity, and here, the similar base refers to a case where some of bases in a mixed base (for example, R=A+G, M =A+ C, W=A+T, S =C+ G, Y = C + T, K = G + T, H=A+T+ C, B = G + T + C, D = G + A + T, V = A + C + G, N = A + C + G + T) are identical. Whether two types of nucleic acids have homology can be investigated by a direct comparison of sequences or by a hybridization method under stringent conditions. When two nucleic acid sequences are directly compared, the genes have homology when the nucleic acid sequences are typically at least 50% homology, preferably at least 70% homology, and more preferably at least 80%, 90%, 95%, 96%, 97%, 98%, or 99% homology.

Amino acids can be mentioned As used herein by either their commonly known three letter symbols or their one character symbols recommended by the IUPAC-IUB Biochemical Nomenclature Commission. Similarly, nucleotides can be mentioned by their commonly recognized one character codes. As used herein, a comparison of similarity, identity, and homology of an amino acid sequence and a base sequence is calculated by using a sequence analysis tool BLAST and default parameters. For example, identity can be searched using BLAST 2.10.1+ (published on June 18, 2020) of the NCBI. As used herein, a value for identity generally refers to a value obtained when aligned under the default conditions using BLAST described above. However, when a higher value is obtained by changing a parameter, the highest value is set as the value of identity. In a case where identity is evaluated in a plurality of regions, the highest value in the plurality of regions is set as the value of identity. Similarity is a numerical value calculated by taking into consideration a similar amino acid in addition to identity.

As used herein, unless otherwise specified, it is understood that reference to a biological material (for example, a protein, a nucleic acid, or a gene) is also a reference to a variant of the biological material (for example, a variant having modification in an amino acid sequence) that performs a function similar to (but not to the same extent as) the biological function of the biological material. Such a variant can contain a fragment of the original molecule, and a molecule that is at least 30%, 40%, 50%, 60%, 70%, 80%, 90%, 95%, 98%, or 99% homology as compared to the sequence of the original molecule that is aligned over the amino acid sequence or nucleic acid sequence of the original biological material of the same size, or aligned by computer homology program known in the art. A variant can contain a molecule containing a modified amino acid (modification by, for example, disulfide bond formation, glycosylation, lipidation, acetylation, or phosphorylation) or a modified nucleotide (for example, modification by methylation).

As used herein, the term "stringent conditions" refers to well-known conditions commonly used in the art. As the stringent conditions, for example, the following conditions can be adopted. Incubation is performed at 37°C overnight (1) using low ionic strength and high temperature for washing (for example, at 50°C, 0.015 M sodium chloride/0.0015 M sodium citrate/0.1% sodium dodecyl sulfate), (2) using a denaturant such as formamide during hybridization (for example, at 42°C, 50% (v/v) formamide and 0.1 % bovine serum albumin/0.1 % Ficoll/0.1 % polyvinylpyrrolidone/50 mM sodium phosphate buffer with pH 6.5, and 750 mM sodium chloride, 75 mM sodium citrate), or (3) in a solution containing 20% formamide, 5 × SSC, 50 mM sodium phosphate (pH 7.6), 5 × Denhardt's solution, 10% dextran sulfate, and 20 mg/ml of denatured sheared salmon sperm DNA, and then the filter is washed at 1 × SSC at about 37 to 50°C. Note that a formamide concentration may be 50% or more. A washing time may be 5, 15, 30, 60, or 120 minutes or longer. A plurality of factors such as a temperature and a salt concentration are considered as factors affecting the stringency of the hybridization reaction, and for details, reference can be made to Ausubel et al., Current Protocols in Molecular Biology, Wiley Interscience Publishers, (1995). Examples of "highly stringent conditions" are 0.0015 M sodium chloride, 0.0015 M sodium citrate, 65 to 68°C, or 0.015 M sodium chloride, 0.0015 M sodium citrate, and 50% formamide, 42°C. Hybridization can be performed according to the methods described in experimental textbooks such as Molecular Cloning 2nd ed., Current Protocols in Molecular Biology, Supplement 1-38, DNA Cloning 1: Core Techniques, A Practical Approach, Second Edition, Oxford University Press (1995). Here, a sequence including only the A sequence or only the T sequence is preferably excluded from a sequence that hybridizes under stringent conditions. Moderately stringent conditions can be readily determined by those skilled in the art, for example, based on DNA length, and are shown in Sambrook et al., Molecular Cloning: A Laboratory Manual, # 3, Vol. 1, 7.42-7.45 Cold Spring Harbor Laboratory Press, 2001; and include,

for nitrocellulose filters, the use of a pre-wash solution of 5 × SSC, 0.5% SDS, and 1.0 mM EDTA (pH 8.0), hybridization conditions of about 50% formamide, 2 × SSC-6 × SSC (or other similar hybridization solution such as Stark's solution in about 50% formamide at about 42°C) at about 40 to 50°C, and washing conditions of 0.5 × SSC, 0.1% SDS at about 60°C. Accordingly, the polypeptide used in the present disclosure also includes a polypeptide encoded by a nucleic acid molecule that hybridizes under highly or moderately stringent conditions to the nucleic acid molecule encoding the polypeptide specifically described in the present disclosure.

As used herein, the term "corresponding" amino acid or nucleic acid refers to an amino acid or a nucleotide which has or is expected to have, in a certain polypeptide molecule or polynucleotide molecule, a similar action as a predetermined amino acid or nucleotide in a reference polypeptide or a polynucleotide for comparison, and, particularly in the case of enzyme molecules, refers to an amino acid which is present at a similar position in an active site and makes a similar contribution to catalytic activity. For example, for an antisense molecule, it can be a similar portion in an ortholog corresponding to a specific portion of the antisense molecule. A corresponding amino acid can be a specific amino acid subjected to, for example, cysteination, glutathionylation, S-S bond formation, oxidation (for example, oxidation of a methionine side chain), formylation, acetylation, phosphorylation, glycosylation, myristylation, or the like. Alternatively, a corresponding amino acid can be an amino acid responsible for dimerization. Such a "corresponding" amino acid or nucleic acid may be a region or a domain over a certain range. Accordingly, it is referred As used herein as a "corresponding" region or domain in such a case.

As used herein, the term "corresponding" gene (for example, a polynucleotide sequence or molecule) refers to a gene (for example, a polynucleotide sequence or molecule) in a certain species which has or is expected to have a similar action as a predetermined gene in a reference species for comparison. When a plurality of genes having such an action are present, the corresponding gene refers to a gene having the same evolutionary origin. Accordingly, a gene corresponding to a certain gene can be an ortholog of such a gene. For example, the cap of serotype 1 AAV can correspond to cap of serotype 2 AAV. For example, a corresponding gene in a certain virus can be found by search on a sequence database for the virus using a gene sequence of the virus serving as a reference of the corresponding gene as a query sequence.

In accordance with the present disclosure, the term "activity" refers to a function of a molecule in the broadest sense As used herein. Activity, although not intended to be limiting, generally includes a biological function, biochemical function, physical function, and chemical function of a molecule. Examples of the activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

As used herein, when referring to a certain gene or a nucleic acid molecule or a polypeptide related thereto, the term "biological function" refers to a specific function that the gene, the nucleic acid molecule, or the polypeptide can have in a living body. Examples of the biological function include, but are not limited to, ability to recognize a specific cell surface structure, enzymatic activity, and ability to bind to a specific protein. In the present disclosure, examples of the biological function include, but are not limited to, a function in which a certain promoter is recognized in a specific host cell. As used herein, the biological function can be exerted by "biological activity". As used herein, the term "biological activity" refers to an activity that a certain agent (for example, a polynucleotide, a protein, or the like) can have in a living body. Biological activity encompasses an activity of exerting a variety of functions (for example, transcription promoting activity), and also encompasses, for example, an activity of activating or inactivating another molecule by an interaction with a certain molecule. For example, when a certain factor is an enzyme, the biological activity thereof encompasses enzyme activity thereof. In another example, in a case where a certain factor is a ligand, binding to a receptor corresponding to the ligand is encompassed. Such biological activity can be measured by a technique that is well known in the art. Accordingly, the term "activity" refers to a variety of measurable indicators indicating or revealing binding (either direct or indirect); and affecting a response (that is, having a measurable effect in response to some exposure or stimulus), including, for example, the amount of upstream or downstream protein in a host cell or a measure of other similar functions.

As used in the present disclosure, the term "infectivity" of a virus or virus-derived construct refers to the ability to introduce a nucleic acid within the virus or virus-derived construct into a cell by adhesion or membrane fusion of the virus or virus-derived construct to the cell. The term "replicative ability" of a virus or virus-derived construct refers to the ability to produce infectious viral particles or virus-derived construct particles within an infected cell.

As used herein, the terms "transformation", "transduction", and "transfection" are used interchangeably, unless otherwise stated, and refer to the introduction of a nucleic acid into a host cell (as necessary, via a virus or virus-derived construct). As the transformation method, any method can be used as long as it is a method of introducing a nucleic acid into a host cell, and examples thereof include various well-known techniques such as the use of competent cells, an electroporation method, a method using a particle gun (gene gun), and a calcium phosphate method.

As used herein, the term "purified" substance or biological agent (for example, a nucleic acid, a protein, or the like) refers to a substance or a biological agent in which at least a portion of a agent naturally accompanying the biological agent is removed. Thus, the purity of the biological agent in the purified biological agent is generally higher than the purity in the normal state of the biological agent (that is, concentrated). The term "purified" As used herein refers to the presence of

preferably at least 75% by weight, more preferably at least 85% by weight, still more preferably at least 95% by weight, and most preferably at least 98% by weight of a biological agent of the same type. The substance used in the present disclosure is preferably a "purified" substance.

As used herein, the term "pharmaceutical ingredient" means an optional ingredient that can constitute a medicine, and examples thereof include an active ingredient (which itself exhibits drug efficacy) and an additional ingredient (ingredient which itself is not expected to exhibit drug efficacy, but is expected to play a certain role (for example, an excipient, a lubricant, a surfactant, or the like) when contained as a medicine). The pharmaceutical ingredient may be a single substance or a combination of a plurality of substances and agents. Any combination such as a combination of an active ingredient and an additive ingredient or a combination of an adjuvant and an active ingredient can also be included.

As used herein, the term "active ingredient" refers to an ingredient that exerts an intended medicinal effect, and can correspond to one or a plurality of ingredients.

As used herein, the term "additional ingredient" refers to an optional ingredient that is not expected to have a medicinal effect but plays a certain role when contained as a medicine, and examples of the additional ingredient include a pharmaceutically acceptable carrier, a stabilizer, an adjuvant, a solubility improving agent, a solubilizing agent, a diluent, an excipient, a buffer, a binder, a diluent, a flavoring agent, and a lubricant.

As used herein, an "agent" is usedin a broad sense, and may be any substance or other elements (for example, energy such as light, radioactivity, heat, and electricity) as long as the intended object can be achieved. Examples of such a substance include, but are not limited to, a protein, a polypeptide, an oligopeptide, a peptide, a polynucleotide, an oligonucleotide, a nucleotide, a nucleic acid (including, for example, DNA such as cDNA or genomic DNA, RNA such as mRNA), a polysaccharide, an oligosaccharide, a lipid, an organic small molecule (for example, a hormone, a ligand, an information transmitting substance, an organic small molecule, a molecule synthesized by combinatorial chemistry, a small molecule that can be used as a medicine (for example, a small molecule ligand or the like), or the like), a composite molecule thereof, and a mixture thereof.

As used herein, the term "complex" or "complex molecule" means any construct having two or more portions. For example, when one portion is a polypeptide, the other portion may be a polypeptide or a substance other than the polypeptide (for example, a substrate, a sugar, a lipid, a nucleic acid, other hydrocarbons, or the like). As used herein, two or more portions constituting the complex may be bonded by a covalent bond or may be bonded by other bonds (for example, a hydrogen bond, an ionic bond, a hydrophobic interaction, van der Waals force, and the like).

As used herein, the term "label" refers to the presence (for example, a substance, energy, an electromagnetic wave, and the like) for identifying a molecule or substance to be targeted from others. Examples of such a labeling method include a radioisotope (RI) method, a fluorescence method, a biotin method, and a chemiluminescence method. When a plurality of target proteins or agents or means for capturing the target proteins are labeled by a fluorescence method, the target proteins are labeled with fluorescent substances having different maximum fluorescence emission wavelengths. A difference in maximum fluorescence emission wavelength is preferably 10 nm or more. Any label that does not affect the function can be used, and examples of the fluorescent substance include Alexa™ Fluor. Alexa™ Fluor is a water-soluble fluorescent dye obtained by modifying coumarin, rhodamine, fluorescein, cyanine, or the like, is a series corresponding to a wide range of fluorescence wavelengths, and is significantly stable, bright, and less pH sensitive as compared with other fluorescent dyes having corresponding wavelengths. Examples of a combination of fluorescent dyes having a maximum fluorescence wavelength of 10 nm or more include a combination of Alexa™ 555 and Alexa™ 633 and a combination of Alexa™ 488 and Alexa™ 555. Examples of other fluorescent labels include a cyanine dye (for example, Cy3 and Cy5 of CyDye™ series and the like), a rhodamine 6G reagent, N-acetoxy-N2-acetylaminofluorene (AAF), and AAIF (iodine derivative of AAF). In the present disclosure, such a label can be utilized to modify the object to be targeted so that the object can be detected by detection means used. Such modifications are known in the art and those skilled in the art can carry out such methods as appropriate for the label and depending on the object to be targeted.

As used herein, the term "kit" refers to a unit generally providing portions to be provided (for example, a virus-derived construct, a manual, and the like) that are usually divided into two or more sections. The form of the kit is preferred when it is intended to provide a composition that should not be provided in a mixed state for stability or the like, but is preferably mixed immediately before use. Such a kit preferably includes an instruction or manual describing how to use the provided portions or how a reagent should be processed. When the kit is used As used herein as a reagent kit, the kit generally includes an instruction or the like describing how to use a virus-derived construct and the like.

As used herein, the term "instruction" is a document with an explanation of the method of use of the present disclosure for a physician or other users. The instruction has a description of administration of the medicine of the present disclosure and the like. In addition, the instruction may include instruction of the administration form. The instruction is prepared in accordance with a format defined by the regulatory agency of the country wherein the present disclosure is practiced (for example, the Ministry of Health, Labor and Welfare in Japan, Food and Drug Administration (FDA) in the United States or the like), with an explicit description showing approval by the regulatory agency. The instruction is a so-called package insert and is usually provided in, but is not limited to, a paper medium. For example, the instruction can also be provided in a form such as an electronic medium (for example, a web site or an e-mail provided on the Internet).

The term "about" refers to the indicated value plus or minus 10%. "About" when used for temperature refers to the indicated temperature plus or minus 5°C, and "about" when used for pH refers to the indicated pH plus or minus 0.5.

(Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description As used herein. It is also understood that the following embodiments may be used alone or in combination.

(Production of virus-derived construct plasmids)

In one aspect, the present disclosure provides a method for producing various virus-derived construct plasmids from starting plasmids (for example, virus-derived construct plasmids of the present disclosure). In one embodiment, a method for producing virus-derived construct plasmids of the present disclosure includes a step of preparing a set of starting plasmids containing at least of a part of nucleic acid sequences required to construct a virus-derived construct, the set of starting plasmids including a set of corresponding alternative unit nucleic acids, and the set of corresponding alternative unit nucleic acids including at least one set of corresponding alternative unit nucleic acids containing different nucleic acid sequences; a step of treating the set of starting plasmids with a restriction enzyme and preparing a mixed solution containing cleaved alternative unit nucleic acid fragments; a step of ligating the cleaved alternative unit nucleic acid fragments to form an integrated nucleic acid; and a step of bringing the integrated nucleic acid into contact with a transformed organism to form virus-derived construct plasmids. When the starting plasmid contains a plurality of unit nucleic acids, the unit nucleic acid fragments generated by the restriction enzyme treatment of the starting plasmid are almost equimolar, and an integrated nucleic acid that can be efficiently incorporated by the transformed organism without requiring complicated adjustment of the number of moles of the unit nucleic acid fragments can be produced. In addition, as the set of starting plasmids includes a set of corresponding alternative unit nucleic acids containing different nucleic acid sequences, alternative unit nucleic acids incorporated at particular positions in the product plasmid are randomly selected from the set of corresponding alternative unit nucleic acids, and thus, virus-derived construct plasmids having new structures are easily obtained, and in a case where a plurality of sets of corresponding alternative unit nucleic acids containing different nucleic acid sequences are present, a structural variation of the virus-derived construct plasmids produced increases synergistically. In one embodiment, in a method for producing virus-derived construct plasmids of the present disclosure, a set of corresponding alternative unit nucleic acids includes a plurality of sets of corresponding alternative unit nucleic acids containing different nucleic acid sequences to provide 16 or more, 27 or more, 32 or more, 64 or more, 81 or more, 128 or more, 243 or more, 256 or more, 512 or more, 729 or more, 1,024 or more possible combinations of different parallel alternative unit nucleic acids.

In one embodiment, the method for producing virus-derived construct plasmids of the present disclosure produces a population of virus-derived construct plasmids. In one embodiment, in the method for producing virus-derived construct plasmids of the present disclosure, at least one of the sets of starting plasmids contains a transcriptional start sequence that functions in animal cells, and virus-derived construct plasmids containing the transcriptional start sequence that functions in animal cells are produced. In one embodiment, in the method for producing virus-derived construct plasmids of the present disclosure, three or more types of starting plasmids are used. In one embodiment, in the method for producing virus-derived construct plasmids of the present disclosure, the set of corresponding alternative unit nucleic acids includes at least two sets of corresponding alternative unit nucleic acids containing different nucleic acid sequences. The virus-derived construct plasmids produced by the method for producing virus-derived construct plasmids of the present disclosure can be used as starting plasmids and subjected to the next round of the method for producing virus-derived construct plasmids of the present disclosure.

In one embodiment, the method for producing virus-derived construct plasmids of the present disclosure further includes a step of selecting a virus-derived construct plasmid having an excellent desired function from a population of the formed virus-derived construct plasmids. Examples of the desired function as the selection criterion include the amount of virus-derived construct produced per plasmid, the amount of virus-derived construct produced per producer cell, the amount of impurities generated when the virus-derived construct is produced from the plasmid, and the purity of a target virus-derived construct when the virus-derived construct is produced from the plasmid. Examples of the impurities include a coat protein that does not contain a nucleic acid sequence, a coat protein that contains an incomplete nucleic acid sequence, an incomplete coat protein, an incomplete nucleic acid sequence, and a virus having self-replication ability (including a wild-type virus-like nucleic acid sequence generated by genetic recombination during the production process) (can be determined to be incomplete by having a structure different from that of the complete nucleic acid or protein expected from the nucleic acid sequence of the virus-derived construct plasmid, such as introduction mutations and

cleavage). In one embodiment, the method for producing virus-derived construct plasmids of the present disclosure can include adding a barcode nucleic acid in the step of ligating to form the integrated nucleic acid, which can facilitate distinguishing the formed virus-derived construct plasmids.

In one embodiment, the method for producing virus-derived construct plasmids of the present disclosure can be performed to improve a partial structure of the virus-derived construct plasmid. In one embodiment, the improvement can be performed by preparing virus-derived construct plasmids containing a combination of parallel alternative unit nucleic acids of the virus-derived construct plasmids selected in the above step of virus-derived construct plasmids having an excellent desired function. For example, when virus-derived construct plasmids having an excellent desired function are found, a combination of elements on the plasmid can be considered preferable for the desired function. Therefore, a structure similar to the combination of elements may be formed on another plasmid with reference to nucleic acid sequence information of the plasmid or generated amino acid sequence information of the plasmid, or a specific region may be cut out from the plasmid and transplanted into another plasmid. In this case, it is not necessary to prepare and examine a virus-derived construct plasmid containing the entire nucleic acid sequence required to construct a virus-derived construct, and the method for producing virus-derived construct plasmids of the present disclosure can be performed in order to prepare a variation of a partial structure to be examined for improvement in the virus-derived construct plasmid.

In one embodiment, in the method for producing virus-derived construct plasmids of the present disclosure, examples of the elements for examining a structural variation include (A) a nucleic acid sequence including a terminal repeat, (B) a nucleic acid sequence encoding a packaging factor, (C) a nucleic acid sequence encoding a structural protein, (D) a nucleic acid sequence encoding a functional cofactor, (E) a nucleic acid sequence encoding a promoter, (F) a nucleic acid sequence encoding a desired gene, and (G) a nucleic acid sequence involved in host cell biosynthesis. The inclusion of variants (different serotypes, variants, different promoters that function in the same host cell, and the like) of these elements (or individual genes corresponding to the elements) in the set of corresponding alternative unit nucleic acids can facilitate the design of virus-derived construct plasmids having an excellent desired function. Those skilled in the art can appropriately determine the elements to be contained in the alternative unit nucleic acids according to the matter to be considered. For example, when an appropriate combination of a promoter and a desired gene is considered, the method for producing virus-derived construct plasmids of the present disclosure can be performed by preparing a set of corresponding alternative unit nucleic acids containing different promoter sequences, and preparing a set of corresponding alternative unit nucleic acids containing sequences of different desired genes as adjacent alternative unit nucleic acids. Subsequently, a preferred combination of a promoter, a desired gene, and an ITR can be examined by performing the method for producing virus-derived construct plasmids of the present disclosure by selecting a plurality of combinations of a promoter and a desired gene having relatively excellent performance, preparing a set of corresponding alternative unit nucleic acids as unit nucleic acids simultaneously containing the combination of the promoter and the desired gene, and performing a combination with a set of corresponding alternative unit nucleic acids containing different ITR sequences. The method for producing virus-derived construct plasmids of the present disclosure may also be performed by preparing three sets of corresponding alternative unit nucleic acids containing different promoter sequences, sequences of different desired genes, or different ITR sequences. In one embodiment, in the method for producing virus-derived construct plasmids of the present disclosure, the set of corresponding alternative unit nucleic acids includes a set of corresponding alternative unit nucleic acids containing different nucleic acid sequences encoding a desired gene, and a set of corresponding alternative unit nucleic acids that are located adjacently upstream thereof and contain different nucleic acid sequences encoding a promoter.

In one embodiment, the present disclosure provides a method for producing virus-derived construct plasmids of the present disclosure, the method including producing adeno-associated virus-derived construct plasmids in Bacillus subtilis, virus-derived construct plasmids produced by such a method, and a virus-derived construct. A virus-derived gene is often difficult to replicate by a host cell due to generation of mutation, inhibition of proliferation to the host cell, or the like. In particular, ITRs contained in AAV are likely to be mutated in Escherichia coli, making replication difficult. In addition, even with current techniques, replication of virus-derived construct plasmids is not readily achievable in any host cell. It is not easy to form and replicate the virus-derived construct plasmids by changing the host to Bacillus subtilis while various efforts are made in the replication of the virus-derived construct plasmids by Escherichia coli. Formation and production of virus-derived construct plasmids in Bacillus subtilis was enabled for the first time through the experiments of the present disclosure.

(OGAB method)

In one embodiment, the virus-derived construct plasmids of the present disclosure and/or a library thereof can be produced by the OGAB method. The Ordered Gene Assembly in Bacillus subtilis (OGAB) method is a method of incorporating an integrated nucleic acid into a transformed organism to produce circular plasmids in the organism. In the OGAB method, plasmids containing large sized nucleic acids can be conveniently prepared. The unit nucleic acid used for

preparing the integrated nucleic acid may be prepared as a unit vector as described in details below (mainly for construction of novel plasmids), or may be prepared by cleaving the constructed plasmids. Hereinafter, a procedure for producing an integrated nucleic acid to be incorporated into a transformed organism will be described.

• Preparation of unit nucleic acids

A unit nucleic acid for incorporation into an integrated nucleic acid is prepared. The following is a description mainly for construction of a novel plasmid. The unit nucleic acid can be produced by any known method, for example, by polymerase chain reaction (PCR) or chemical synthesis. The unit nucleic acid can contain any desired sequence, such as a sequence encoding a desired protein (a therapeutic protein, a protein constituting a virus-derived construct, or the like) or a portion thereof, a sequence controlling a gene (a promoter, an enhancer, or the like), or a sequence for manipulating a nucleic acid (a restriction enzyme recognition sequence or the like). The ends of each unit nucleic acid can be configured to produce a specific protruding sequence so that a plurality of types of unit nucleic acids are aligned in a particular order and/or specific direction when incorporated into an integrated nucleic acid. One or a plurality of unit nucleic acids can be designed to encode one or a plurality of genes having a long base length, as a large number of unit nucleic acids can eventually be integrated on the plasmid. Examples of the gene having a long base length include a group of genes constituting a series of metabolic pathways.

• Preparation of unit vector

A unit vector can be prepared by linking a unit nucleic acid and an additional nucleic acid different from the unit nucleic acid. The use of the unit vector can allow for easier handling of unit nucleic acids.

The additional nucleic acid may be a linear nucleic acid or a circular plasmid. In a case where a circular plasmid is used as an additional nucleic acid, a unit vector can also have a circular structure, and thus can be used for transformation of Escherichia coli and the like, for example. In one embodiment, the additional nucleic acid can contain a replication origin so that the unit vector is replicated in the introduced host. In one embodiment, all the unit nucleic acids for constructing an integrated nucleic acid can be linked to the same type of additional nucleic acid, thereby reducing a difference in size between the unit vectors and facilitating the handling of the plurality of types of unit vectors. In one embodiment, a unit nucleic acid for constructing an integrated nucleic acid may be linked to different types of additional nucleic acids. In one embodiment, for one or a plurality of unit nucleic acids for constructing an integrated nucleic acid, a ratio of (base length of unit nucleic acid)/(base length of unit vector) or an average thereof can be 50% or less, 40% or less, 30% or less, 20% or less, 15% or less, 10% or less, 7% or less, 5% or less, 2% or less, 1.5% or less, 1% or less, or 0.5% or less. As the size of the additional nucleic acid is larger than that of the unit nucleic acid, more uniform handling of different types of unit vectors can be possible. The linking between the unit nucleic acid and the additional nucleic acid can be performed by any method such as ligation using the DNA ligase or TA cloning method. In one embodiment, for one or the plurality of unit nucleic acids for constructing an integrated nucleic acid, the ratio of (base length of unit nucleic acid)/(base length of unit vector) or the average thereof can be 1% or more, 0.3% or more, 0.1% or more, 0.03% or more, 0.01% or more, 0.003% or more, or 0.001% or more, and the manipulation of the unit vector can be facilitated.

In one embodiment, the length of the unit nucleic acid can be 10 bp or more, 20 bp or more, 50 bp or more, 70 bp or more, 100 bp or more, 200 bp or more, 500 bp or more, 700 bp or more, 1,000 bp or more, or 1,500 bp or more, and 5,000 bp or less, 5,000 bp or less, 2,000 bp or less, 1,500 bp or less, 1,200 bp or less, 1,000 bp or less, 700 bp or less, or 500 bp or less.

In one embodiment, the integrated nucleic acid can be constructed from 2 kinds or more, 4 kinds or more, 6 kinds or more, 8 kinds or more, 10 kinds or more, 15 kinds or more, 20 kinds or more, 30 kinds or more, 40 kinds or more, 50 kinds or more, 60 kinds or more, 70 kinds or more, 80 kinds or more, 90 kinds or more, kinds or 100 kinds or more, and 1,000 kinds or less, 700 kinds or less, 500 kinds or less, 200 kinds or less, 120 kinds or less, 100 kinds or less, 80 kinds or less, 70 kinds or less, 60 kinds or less, 70 kinds or less, kinds or 50 kinds or less unit nucleic acids. By adjusting the numbers of moles of the respective unit nucleic acids (kinds or unit vectors) to be substantially the same, a desired integrated nucleic acid having a tandem repeat-like structure can be efficiently produced.

In one embodiment, the unit nucleic acid may have a base length obtained by approximately equally dividing a set of repetitive sequences in the integrated nucleic acid by the number of unit nucleic acids. This can facilitate a manipulation of aligning the numbers of moles of the respective unit nucleic acids (or unit vectors). In one embodiment, the unit nucleic acid can have an increased or decreased base length of 30% or less, 25% or less, 20% or less, 15% or less, 10% or less, 7% or less, or 5% or less from a base length obtained by approximately equally dividing a set of repetitive sequences in the integrated nucleic acid by the number of unit nucleic acids.

In one embodiment, the unit nucleic acid can be designed to have a non-palindromic sequence (a sequence that is not a palindromic sequence) at the end of the unit nucleic acid. The unit nucleic acid designed as described above can easily provide a structure in which the unit nucleic acids are linked to each other while maintaining the order in the

integrated nucleic acid when the non-palindromic sequence is made into a protruding sequence.

• Production of integrated nucleic acid

The integrated nucleic acid can be constructed by linking the unit nucleic acids to each other. In a case where a plurality of unit nucleic acids are present on the same starting plasmid, an integrated nucleic acid can be constructed by cleaving the starting plasmid to produce unit nucleic acid fragments and linking the unit nucleic acid fragments to each other. In one embodiment, the unit nucleic acid can be prepared by being cut out from the unit vector by a restriction enzyme or the like. An integrated nucleic acid can contain 1 or more, 2 or more, 3 or more, 4 or more, 5 or more, 6 or more, 7 or more, 8 or more, 9 or more, or 10 or more sets of repetitive sequences. The repetitive sequence in the integrated nucleic acid can include a sequence of a unit nucleic acid, and as necessary, a sequence of an integrated vector nucleic acid. The integrated nucleic acid can contain a sequence that enables replication of the nucleic acid in a transformed organism. In one embodiment, a sequence that enables replication of the nucleic acid in a transformed organism can contain a replication origin effective in the transformed organism (for example, Bacillus bacteria (Bacillus subtilis)). The sequence of the replication origin effective in Bacillus subtilis is not particularly limited, and examples of a sequence having a θ-type replication mechanism include a sequence of an origin of replication contained in plasmids such as pTB19 (Imanaka, T., et al. J. Gen. Microbioi. 130, 1399-1408 (1984)) or pLS32 (Tanaka, T and Ogra, M. FEBS Lett. 422, 243-246 (1998)), and pAMβ1 (Swinfield, T. J., et al. Gene 87, 79-90 (1990)).

The integrated nucleic acid may contain an additional base sequence as necessary in addition to the unit nucleic acid. In one embodiment, the integrated nucleic acid may contain a base sequence that controls transcription translation, such as a promoter, an operator, an activator, or a terminator. Specific examples of the promoter in the case of using Bacillus subtilis as a host include Pspac (Yansura, D. and Henner, D. J. Pro. Natl. Acad. Sci, USA 81, 439-443 (1984.)) and Pr promoter (Itaya, M. Biosci. Biotechnol. Biochem. 63, 602-604 (1999)), which can be regulated in expression by isopropyl s-D-thiogalactopyranoside (IPTG).

The unit nucleic acid can form a repetitive structure that maintains a certain order and direction in the integrated nucleic acid. In one embodiment, the unit nucleic acid is constructed so that the base sequences at the protruding ends of the unit nucleic acid cut out from the unit vector are complementary to each other, such that it is possible to form a repetitive structure in which a certain order and direction in the integrated nucleic acid are maintained. In one embodiment, by making the structure of the protruding end unique for each different unit nucleic acid, it is possible to efficiently form a repetitive structure maintaining a certain order and direction. In one embodiment, the protruding end may contain a non-palindromic sequence and may be either a 5' end protruding or a 3' end protruding.

In one embodiment, a unit nucleic acid having a protruding end can be cut out from a unit vector using a restriction enzyme. In the embodiment, the unit vector can contain one or a plurality of restriction enzyme recognition sequences. In a case where the unit vector contains a plurality of restriction enzyme recognition sequences, the respective restriction enzyme recognition sequences may be recognized by the same restriction enzyme or may be recognized by different restriction enzymes. In one embodiment, the unit vector can contain a pair of regions recognized by the same restriction enzyme so that a complete unit nucleic acid region is included between these regions. The restriction enzymes used are not particularly limited, Type IIS restriction enzymes such as AarI, BbsI, BbvI, BcoDI, BfuAI, BsaI, BsaXI, BsmAI, BsmBI, BsmFI, BspMI, BspQI, BtgZI, FokI, and SfaNI can be used, and these restriction enzymes can create a protruding end at a site away from the recognition sequence outside the recognition sequence by a certain distance. When (for example, single) Type IIS restriction enzyme is used, the sequences of the protruding ends of the cut unit nucleic acid can be different for each unit nucleic acid, which can be advantageous for assembling a plurality of unit nucleic acids in a certain order and direction. In one embodiment using Type IIS restriction enzyme, the unit vector does not contain a region recognized by Type IIS restriction enzyme in the unit nucleic acid region. In an embodiment using a restriction enzyme that cleaves the recognition region, the unit vector can contain a region recognized by the restriction enzyme at the end of the unit nucleic acid region.

In one embodiment, when the same type of restriction enzyme is used to cut out the unit nucleic acid from a plurality of unit vectors, the restriction enzyme treatment can be performed in a solution containing the plurality of unit vectors, and the efficiency of the operation can be improved. The types of restriction enzymes used to produce a certain integrated nucleic acid can be, for example, 5 or less, 4 or less, 3 or less, 2 or less, or 1, and the use of a small number of restriction enzymes can reduce variations in the number of moles between the unit nucleic acids. In one embodiment, the unit nucleic acid cut out from the unit vector can be readily purified by any known fractionation method such as agarose gel electrophoresis.

The unit nucleic acid, and as necessary, the integrated vector nucleic acids can be linked (ligated) to each other using DNA ligase or the like. Accordingly, an integrated nucleic acid can be produced. For example, the linking of the unit nucleic acids, and as necessary, the integrated vector nucleic acids can be performed in the presence of components such as polyethylene glycol (for example, PEG2000, PEG4000, PEG6000, PEG8000, and the like) and salts (for example, monovalent alkali metal, sodium chloride, and the like). A concentration of each unit nucleic acid in a ligation reaction

solution is not particularly limited, and can be 1 fmol/μl or more. A reaction temperature and time of the ligation are not particularly limited, and are, for example, 37°C and 30 minutes or longer. In one embodiment, before linking the unit nucleic acid, and as necessary, the integrated vector nucleic acid, a composition containing the unit nucleic acid, and as necessary, the integrated vector nucleic acid may be subjected to any conditions for inactivating the restriction enzyme (for example, phenol/chloroform treatment).

The unit nucleic acid can be adjusted to about the same number of moles using the method described in WO 2015/111248 A or the like. In a case where a plurality of unit nucleic acids are present on the same starting plasmid, fragments of the (parallel) unit nucleic acids generated by cleaving the starting plasmid are adjusted to the same number of moles as in nature. The total number of moles of the set of (parallel) alternative unit nucleic acids generated by mixing and cleaving a plurality of starting plasmids is adjusted to the same as in nature. By adjusting the unit nucleic acids to approximately the same number of moles, a desired integrated nucleic acid having a tandem repeat-like structure can be efficiently produced. The number of moles of the unit nucleic acid can be adjusted by measuring the concentration of the unit vector or the unit nucleic acid.

• Production of plasmids from an integrated nucleic acid(s)

Plasmids can be formed in a transformed organism by bringing an integrated nucleic acid into contact with the transformed organism. In one embodiment, examples of the transformed organism include bacteria belonging to the genus Bacillus, bacteria belonging to the genus Streptococcus, bacteria belonging to the genus Haemophilus, and bacteria belonging to the genus Neisseria. Examples of the bacteria belonging to the genus Bacillus include B. subtilis (Bacillus subtilis), B. megaterium (Bacillus megaterium), and B. stearothermophilus (Bacillus stearothermophilus). In a preferred embodiment, the transformed organism is Bacillus subtilis. In one embodiment, the transformed organism that incorporates the integrated nucleic acid is in a competent state and can actively incorporate a nucleic acid. The term "competent" refers to a state in which a cell becomes more permeable to exogenous substances (for example, nucleic acids). For example, Bacillus subtilis in a competent state cleaves a double-stranded nucleic acid as a substrate on a cell, decomposes any one strand of the double-stranded nucleic acid from the cleavage point, and incorporates the other single strand into a bacterial cell. The incorporated single strand can be repaired into a circular double-stranded nucleic acid in the bacterial cell. Any known method can be used to render the transformed organism competent, and for example, Bacillus subtilis can be rendered competent using the method described in Anagnostopoulou, C. and Spizen, J. J. Bacteriol., 81, 741-746 (1961). As a method of transformation, a known method suitable for each transformed organism can be used.

(Elements of virus-derived construct plasmids)

In one aspect, the present disclosure provides virus-derived construct plasmids. Although elements of the virus-derived construct plasmids are described below, any element or combination of elements can be contained in alternative unit nucleic acids to perform a method for producing virus-derived construct plasmids of the present disclosure The elements of the virus-derived construct plasmids described below are applied to both the starting plasmid and the product plasmid. In addition, the elements of the virus-derived construct plasmids described below are also applied to a virus-derived construct plasmid library of the present disclosure.

In one embodiment, the present disclosure provides a plasmid containing at least of a part of nucleic acid sequences required to construct a virus-derived construct in an operably linked form. In one embodiment, the nucleic acid sequence required to construct a virus-derived construct includes (A) a nucleic acid sequence including a terminal repeat (for example, derived from any of serotypes 1 to 12 or variants thereof), (B) a nucleic acid sequence encoding a packaging factor (for example, rep, which can be derived from any of adeno-associated virus serotypes 1 to 12 or variants thereof), (C) a nucleic acid sequence encoding a structural protein (for example, cap, which can be derived from any of adeno-associated virus serotypes 1 to 12 or variants thereof), and (D) a nucleic acid sequence encoding a functional cofactor (for example, at least one of E1A, E1B, E2A, E4, and VA, which can be derived from any of adenovirus serotypes 1 to 52 or variants thereof). In one embodiment, the virus-derived construct plasmid of the present disclosure contains 2, 3, or 4 of the nucleic acid sequences of (A) to (D). In one embodiment, the nucleic acid sequence required to constitute a virus is located in a continuous region on the virus-derived construct plasmid. A virus-derived construct plasmid having such a structure can be formed by incorporating nucleic acid fragments containing a nucleic acid sequence required to constitute a virus into an original plasmid. In one embodiment, the nucleic acid sequence required to constitute a virus is present in a continuous region having a base length of about 50% or less, about 40% or less, about 30% or less, about 25% or less, about 20% or less, about 15% or less, about 10% or less, or about 5% or less of the total base length of the virus-derived construct plasmids. In one embodiment, elements other than the terminal repeat of the nucleic acid sequence required to constitute a virus (for example, one or a plurality of a nucleic acid sequence encoding a structural protein of a virus, a nucleic acid sequence encoding a packaging factor, and a functional cofactor) may each be located between or outside two terminal repeats. In the virus-derived construct plasmids of the present disclosure, the elements of the nucleic acid

sequence required to constitute a virus may be arranged in any order and position, and it is understood that various arrangements other than the arrangements specifically shown in the drawings and the like can be used. In a case where the nucleic acid sequence described in a specific function such as a functional cofactor contains a plurality of elements, the order and position of each element in the virus-derived construct plasmid can be arbitrary, unless otherwise specified, and arbitrary elements (for example, VA, E2A, and E4) can be arranged in succession (without sandwiching other genes therebetween). As the functional cofactor, a gene or a combination of genes derived from a virus different from the origin virus of the virus-derived construct plasmid such as an adenovirus, a herpes virus, a papilloma virus, a bocavirus, and a simian virus may be used. In one embodiment, examples of the functional cofactor include adenoviruses E1A, E1B, E2A, E4, and VA, herpes viruses UL5, UL8, UL30, UL42, UL52, ICP0, ICP4, ICP8, and ICP22, papilloma viruses E1, E2, and E6, bocaviruses NP1, NS2, NS4, and BocaSR, and simian virus Large T antigen.

In one embodiment, the nucleic acid sequence required to constitute a virus contains two terminal repeats of the virus, and a base length of a region sandwiched between the two terminal repeats (excluding the terminal repeats themselves) can be about 1 kb or more, about 5 kb or more, about 10 kb or more, about 20 kb or more, about 30 kb or more, about 40 kb or more, about 50 kb or more, about 70 kb or more, or about 100 kb or more. In one embodiment, the nucleic acid sequence required to constitute a virus contains two terminal repeats of the virus and another portion, and the another portion is located outside the region sandwiched between the two terminal repeats. In one embodiment, the virus-derived construct plasmid of the present disclosure contains a promoter, a desired gene, and a terminator from upstream between a 5' ITR and a 3' ITR. In particular, it is intended that the nucleic acid sequence encoding a structural protein of a virus may be a variant of a wild-type sequence (for example, adeno-associated virus serotypes 1 to 12).

In one embodiment, the virus-derived construct plasmid of the present disclosure contains a promoter upstream of the element, as necessary. For example, the virus-derived construct plasmids of the present disclosure may contain a promoter upstream of one or a plurality of the nucleic acid sequences of (B), (C), and (D) or the genes contained therein of the nucleic acid sequences required to construct a virus-derived construct. For example, the virus-derived construct plasmids of the present disclosure may contain a promoter upstream of one or a plurality of desired genes. In one embodiment, the virus-derived construct plasmid of the present disclosure contains a drug-selectable marker nucleic acid sequence between any two of the nucleic acid sequences of (A) to (D). In one embodiment, the virus-derived construct plasmid of the present disclosure contains a drug-selectable marker nucleic acid sequence between any two genes encoded by the nucleic acid sequences of (A) to (D). In one embodiment, a set of alternative unit nucleic acids encoding a promoter includes a set of corresponding alternative unit nucleic acids encoding promoters whose types or directions are different. In one embodiment, the promoter includes a drug-responsive promoter.

In one embodiment, the virus-derived construct plasmids of the present disclosure are used to produce a viral vector, a virus-like particle (VLP), an oncolytic virus (including a virus modified to proliferate only in cancer cells), or a viral replicon (self-replicating viral genome lacking the ability to produce viral particles).

In one embodiment, the virus-derived construct plasmid of the present disclosure contains a nucleic acid sequence that promotes plasmid replication in a microbial host cell. In one embodiment, a nucleic acid sequence that promotes plasmid replication in Bacillus subtilis can contain an origin of replication that operates in Bacillus subtilis, and an origin of replication contained in plasmids such as oriC, pTB19 (Imanaka, T., et al. J. Gen. Microbioi. 130, 1399-1408 (1984)) or pLS32 (Tanaka, T and Ogra, M. FEBS Lett. 422, 243-246 (1998)), and pAMβ1 (Swinfield, T. J., et al. Gene 87, 79-90 (1990)). Examples of the nucleic acid sequence that promotes plasmid replication in Bacillus subtilis include a plasmid or a portion thereof, or an origin of replication or a variant thereof, which is known to activate a replication mechanism by rolling circle type, theta type, oriC, phage, or the like. In one embodiment, the nucleic acid sequence that promotes plasmid replication in Bacillus subtilis can include a sequence that is identical or similar to a known replication origin (for example, 90% or more, 95% or more, 97% or more, 98% or more, 99% or more, or 99.5% or more sequence identity). In one embodiment, the nucleic acid sequence that promotes plasmid replication in a microbial host cell may be arranged within or outside a continuous region where the nucleic acid sequence required to constitute a virus is present.

In one embodiment, the virus-derived construct plasmids of the present disclosure can be produced or used in cells of organisms other than Bacillus subtilis, and thus can contain a transcriptional/translational regulatory sequence that operates in these organisms, such as an origin of replication, a promoter, and a transcription termination sequence. The transcriptional/translational regulatory sequence for each organism is known and can be appropriately selected by those skilled in the art. In one embodiment, the virus-derived construct plasmids of the present disclosure can be produced or replicated in Escherichia coli, a yeast, and the like, and thus can contain a transcriptional/translational regulatory sequence that operates in these microorganisms. In one embodiment, the virus-derived construct plasmids of the present disclosure can be introduced into producer cells and thus can contain a transcriptional/translational regulatory sequence that operates in producer cells. In particular, since the producer cell can be an animal cell, the virus-derived construct plasmids of the present disclosure can contain a transcriptional/translational regulatory sequence that operates in an animal cell. In one embodiment, the virus-derived construct plasmids of the present disclosure do not contain the sequence of at least some genes of the entire genome of the virus.

In one embodiment, the virus-derived construct plasmid of the present disclosure contains a nucleic acid

sequence involved in host cell biosynthesis. The nucleic acid sequence involved in host cell biosynthesis is a nucleic acid sequence containing genes involved in communication (metabolism, endoplasmic reticulum processing, and the like), anti-apoptosis, oxidative stress, and cell proliferation in cells, and examples of such genes include V40 Large T antigen (SV40LTA), TAX, Kras, Myc, MIR17HG, BCL-2, BCL-XL, PDIA2, XBP1, HSPA5, pyruvate carboxylase (PC), PDK, HIF1A, NRF2, and CPSF6.

In one embodiment, the virus-derived construct plasmid of the present disclosure contains a desired gene. In one embodiment, the desired gene can be located between two terminal repeats. In one embodiment, the desired gene in the virus-derived construct plasmids of the present disclosure can ultimately be contained in the loaded nucleic acid of the virus-derived construct. Such a desired gene may be encoded by a therapeutic protein, may be encoded by a gene for gene therapy, may be encoded by a gene for gene cell therapy such as CART therapy, may be encoded by a non-protein coding functional RNA (rRNA, tRNA, microRNA (miRNA), lncRNA, or the like), or may contain a transcriptional/translational regulatory sequence such as a promoter, a terminator, an insulator, an enhancer, a silencer, and an origin of replication in combination or independently thereof. In one embodiment, the desired gene contains a viral promoter, such as a cytomegalovirus promoter, a CAG promoter, an SV40 promoter, or an RSV promoter (as necessary, upstream of the protein-coding gene). In one embodiment, the desired gene contains an IRES (as necessary, upstream of the protein-coding gene). In one embodiment, the desired gene is contained in the virus-derived construct plasmid at the position of promoter, desired gene, and terminator from upstream between a 5' LTR and a 3' LTR. In one embodiment, two or more sets of the promoter, desired gene, and terminator are contained in the virus-derived construct plasmid. In one embodiment, the desired gene can be integrated into chromosomes of a subject to whom the virus-derived construct is administered. In the embodiment, the desired gene may have a function of controlling expression of gene originally contained in the subject, or may result in long-term protein expression. For example, a virus-derived construct based on adeno-associated virus, a retrovirus, and the like can be incorporated into chromosomes of a subject. In one embodiment, the desired gene can be incorporated into a therapeutic cell (for example, chromosome) (for example, via a treatment of the cells with a virus-derived construct ex vivo). In one embodiment, the virus-derived construct plasmids of the present disclosure do not contain all the genes required for the origin virus of the virus-derived construct to proliferate, and accordingly, the virus-derived construct produced may be configured not to have proliferative ability.

In one embodiment, the desired gene in the virus-derived construct plasmid of the present disclosure can include a plurality of genes. In one embodiment, the desired gene can include 2 to 100 genes, for example, 2 or more, 3 or more, 4 or more, 5 or more, 7 or more, 10 or more, 12 or more, 15 or more, 20 or more, 25 or more, 30 or more, 40 or more, or 50 or more and 100 or less, 90 or less, 80 or less, 70 or less, 60 or less, 50 or less, 40 or less, 35 or less, 30 or more, 25 or less, 20 or less, 17 or less, 15 or less, 12 or less, or 10 or less genes. In one embodiment, the desired gene in the virus-derived construct plasmid of the present disclosure can have a base length of about 0.1 to 1,000 kbp, for example, about 0.1 kbp or more, about 0.3 kbp or more, about 1 kbp or more, about 2 kbp or more, about 5 kbp or more, about 7 kbp or more, about 10 kbp or more, about 20 kbp or more, about 50 kbp or more, or about 100 kbp or more, and about 1,000 kbp or less, about 700 kbp or less, about 500 kbp or less, about 200 kbp or less, about 100 kbp or less, about 70 kbp or less, about 50 kbp or less, about 20 kbp or less, or about 10 kbp or less. Since the virus-derived construct plasmids of the present disclosure can be constructed into a complex structure containing a plurality of genes by the OGAB method, a desired gene can also be constructed into a complex structure. Since the size of the loaded nucleic acid can be limited by the type of virus-derived construct, the type of virus-derived construct can be selected depending on the size of the desired gene. In one embodiment, the desired gene includes a plurality of genes, such that a nucleic acid having high functionality can be delivered to a subject, such as a combination of a tissue (for example, cancer tissue)-specific or time-specific promoter of a subject and a therapeutic gene operably linked thereto (therapeutic protein coding sequence, gene for gene therapy, gene for gene cell therapy, or the like), or a sequence encoding a series of enzymes that enables coordinated expression of a series of enzymes that control a metabolic cascade.

In one embodiment, examples of the protein encoded by the desired gene in the virus-derived construct plasmid of the present disclosure include a therapeutic polypeptide (for example, replacement therapy), and an immunogenic polypeptide (for example, pathogen polypeptide, cancer antigen polypeptide, or the like). Examples of the therapeutic polypeptide include cystic fibrosis transmembrane regulator protein (CFTR), dystrophin (mini-dystrophin and micro-dystrophin), myostatin propeptide, follistatin, soluble activin type II receptor, IGF-1, anti-inflammatory polypeptide, sarcospan, utrophin, mini-utrophin, a coagulation factor (for example, Factor VIII, Factor IX, Factor X, or the like), erythropoietin, angiostatin, endostatin, catalase, tyrosine hydroxylase, superoxide dismutase, leptin, LDL receptor, lipoprotein lipase, ornithine transcarbamylase, β-globin, α-globin, spectrin, α1-antitrypsin, adenosine deaminase, hypoxanthine-guanine phosphoribosyltransferase, β-glucocerebrosidase, sphingomyelinase, lysosomal hexosaminidase A, branched chain ketoacid dehydrogenase, RP65 protein, cytokine (for example, α-interferon, β-interferon, interferon-γ, interleukin-2, interleukin-4, granulocyte-macrophage colony-stimulating factor, lymphotoxin, or the like), peptide growth factor, neurotrophic factor and hormone (for example, somatotropin, insulin, insulin-like growth factors 1 and 2, platelet-derived growth factor, epidermal growth factor, fibroblast growth factor, nerve growth factor, neurotrophic factor-3 and -4, brain-derived neurotrophic factor, bone morphogenetic protein, glial-derived growth factor, transforming growth factor-α

and -β, or the like), lysosomal acid α-glucosidase, α-galactosidase A, soluble tumor necrosis growth factor α receptor, S100A1, parvalbumin, adenylyl cyclase type 6, anti-inflammatory factor, anti-myostatin protein, aspartoacylase, suicide gene product (for example, thymidine kinase, cytosine deaminase, diphtheria toxin, or tumor necrosis factor), tumor suppressor gene product (for example, p53, Rb, or Wt-1), TRAIL, and FAS-ligand.

Examples of the pathogen polypeptide include a cell surface protein of a pathogenic organism such as bacteria, fungi, or parasites, and a protein (for example, spike protein, envelope protein, capsid protein, or the like) expressed on the surface of a virus. Specific examples of the pathogen polypeptide include orthomyxovirus immunogen (for example, influenza virus hemagglutinin (HA) or nucleoprotein), lentivirus immunogen (for example, HIV or SIV envelope GP160 protein, matrix/capsid protein, gag, pol, or env gene product), arenavirus immunogen (for example, Lassa fever virus nucleocapsid protein or envelope glycoprotein), poxvirus immunogen (for example, vaccinia L1 or L8 gene product), flavivirus immunogen (for example, yellow fever virus or Japanese encephalitis virus immunogen), filovirus immunogen (for example, Ebola virus or Marburg virus immunogen such as NP and GP gene products), bunyavirus immunogen (for example, RVFV, CCHF, or SFS virus immunogen), coronavirus immunogen (for example, human coronavirus immunogen such as human coronavirus envelope glycoprotein), polio immunogen, herpes virus immunogen (for example, CMV, EBV, or HSV immunogen), mumps virus immunogen, measles virus immunogen, rubella virus immunogen, diphtheria toxin or other diphtheria immunogens, pertussis antigen, and hepatitis (for example, hepatitis A, hepatitis B, hepatitis C, or the like) immunogen.

Examples of the cancer antigen polypeptide include BRCA1 gene product, BRCA2 gene product, gp100, tyrosinase, GAGE-1/2, BAGE, RAGE, LAGE, NY-ESO-1, CDK-4, β-catenin, MUM-1, caspase-8, KIAA0205, HPVE, SART-1, PRAME, p15, melanoma tumor antigen, MART-1, gp100 MAGE-1, MAGE-2, MAGE-3, CEA, TRP-1, TRP-2, P-15, tyrosinase, HER-2/neu gene product, CA125, LK26, FB5 (endosialin), TAG72, AFP, CA19-9, NSE, DU-PAN-2, CA50, SPan-1, CA72-4, HCG, STN (sialyl In antigen), c-erbB-2 protein, PSA, L-CanAg, estrogen receptor, milk fat globulin, p53 tumor suppressor protein, mucin antigen, telomerase, nuclear matrix protein, prostatic acid phosphatase, and papilloma virus antigen.

In one embodiment, the desired gene can be a gene for treating a particular disease (for example, gene for gene therapy). The gene to be selected for a specific disease can be appropriately selected by those skilled in the art. Examples of such a disease include infections due to various types of pathogens, cystic fibrosis, hemophilia A, hemophilia B, thalassemia, anemia, Alzheimer's disease, multiple sclerosis, Parkinson's disease, Huntington's disease, amyotrophic lateral sclerosis, spinal muscular atrophy, epilepsy, cancer (melanoma, adenocarcinoma, thymoma, lymphoma, sarcoma, lung cancer, liver cancer, colon cancer, leukemia, uterine cancer, breast cancer, prostate cancer, ovarian cancer, cervical cancer, bladder cancer, kidney cancer, pancreatic cancer, brain cancer, or the like), diabetes, muscular dystrophy, Gaucher's disease, Hurler syndrome, adenosine deaminase deficiency, glycogen storage disease, congenital emphysema, Lesch-Nyhan syndrome, Niemann-Pick disease, Tay-Sachs disease, Angelman syndrome, maple syrup urine disease, age-related macular degeneration, amaurosis, diabetic retinopathy, retinal degenerative disease, astrocytomas, glioblastoma, heart failure, peripheral artery disease, arthritis, joint disorder, intimal hyperplasia, AIDS, muscle wasting, kidney deficiency, hepatitis, LDL receptor deficiency, hyperammonemia, Krabbe's disease, Batten disease, spinal cerebral ataxias, phenylketonuria, autoimmune disease, amino acid metabolic disorder, organic acid metabolic disorder, fatty acid metabolic disorder, mitochondrial disease, sugar metabolic disorder, lysosomal disease, peroxisomal disorder, metal metabolic disorder, purine pyrimidine metabolic disorder, vitamin metabolic disorder, neurotransmitter disorder, lipid metabolic disorder, connective tissue disorder, congenital porphyria, α1-antitrypsin deficiency, lysosomal storage disease, mucopolysaccharidosis disorder, Fabry disease, Canavan disease, Leigh disease, Refsum's disease, Tourette's Syndrome, primary lateral sclerosis, progressive muscular atrophy, Pick disease, muscular dystrophy, myasthenia gravis, Binswanger disease, cerebral infarction, mood disorder, depression, bipolar affective disorder, persistent affective disorder, secondary mood disorder, schizophrenia, drug dependence, anxiety, obsessional disorder, somatoform disorder, dissociative disorder, grief, post-partum depression, hallucination, delusion, dementia, paranoia, autism spectrum disorder, attention-deficit hyperactivity disorder, psychosexual disorder, sleeping disorder, pain disorder, eating disorder, weight disorder, obesity, cachexia, anorexia nervosa, and bulimia. In one embodiment, the desired gene can be incorporated into a therapeutic cell (for example, chromosome) which is used for CART therapy or the like (for example, via a treatment of the cells with a virus-derived construct ex vivo).

In one embodiment, the present disclosure provides a virus-derived construct plasmid library containing the virus-derived construct plasmids of the present disclosure. In one embodiment, the present disclosure provides a virus-derived construct plasmid library containing a virus-derived construct plasmid containing three or more alternative unit nucleic acids as a series of parallel alternative unit nucleic acids, in which the virus-derived construct plasmid library contains a plurality of sets of corresponding alternative unit nucleic acids containing different nucleic acid sequences, and the parallel alternative unit nucleic acids on each virus-derived construct plasmid of the plurality of sets of corresponding alternative unit nucleic acids containing different nucleic acid sequences have a length of about 10 kb or more. In one embodiment, the set of corresponding alternative unit nucleic acids in the virus-derived construct plasmid library includes a set of corresponding alternative unit nucleic acids containing at least one different nucleic acid sequence of (A) a nucleic

acid sequence including a terminal repeat, (B) a nucleic acid sequence encoding a packaging factor, (C) a nucleic acid sequence encoding a structural protein, and (D) a nucleic acid sequence encoding a functional cofactor. In one embodiment, each virus-derived construct plasmid in the virus-derived construct plasmid library contains (A) a nucleic acid sequence including a terminal repeat, (B) a nucleic acid sequence encoding a packaging factor, (C) a nucleic acid sequence encoding a structural protein, and (D) a nucleic acid sequence encoding a functional cofactor. In one embodiment, the set of corresponding alternative unit nucleic acids in the virus-derived construct plasmid library includes a set of corresponding alternative unit nucleic acids containing at least one different nucleic acid sequence of (A) a nucleic acid sequence encoding a desired gene located between terminal repeats, (B) a nucleic acid sequence encoding a packaging factor, (C) a nucleic acid sequence encoding a structural protein, and (D) a nucleic acid sequence encoding a functional cofactor, and a set of corresponding alternative unit nucleic acids that are located adjacently upstream thereof and contain different nucleic acid sequences encoding a promoter. In one embodiment, the plurality of sets of corresponding alternative unit nucleic acids containing different nucleic acid sequences in the virus-derived construct plasmid library contain an alternative unit nucleic acid having a GC content of 75% or more. In one embodiment, the set of corresponding alternative unit nucleic acids of the virus-derived construct plasmid library includes a plurality of sets of corresponding alternative unit nucleic acids that allow for 64 or more different parallel alternative unit nucleic acid combinations, and each virus-derived construct plasmid contains the same number of parallel alternative unit nucleic acids. In one embodiment, each virus-derived construct plasmid contains a barcode nucleic acid sequence.

The virus-derived construct plasmid library of the present disclosure can also be characterized by the structure of the virus-derived construct plasmids contained therein. For example, in the case of a promoter library with different combinations of promoter portions for a common gene, each plasmid in the library can be common in sequence and order of the portion of the gene. In one embodiment, each plasmid in the library can have a common structural characteristic of having a recognition sequence for Type II restriction enzyme at a specific position (for example, upstream or downstream of a predetermined gene). In one embodiment, each plasmid in the library can have a common structural characteristic of having a recognition sequence for Type II restriction enzyme and the same sequence between the recognition sequences at a specific position (for example, upstream or downstream of a predetermined gene). In the CombiOGAB method, the plasmid preferably contains a recognition sequence for Type II restriction enzyme, since it can be operationally efficient to use the same restriction enzyme to generate different cleavage ends. In one embodiment, the library contains or consists of plasmids having different portions in common portions and combinations. The term "different portions in a combination" refers to a combination of elements such that the same for each element constituting the combination is found on different plasmids in the library, but the same combination of elements is not found on different plasmids in the library. The "common portions" can contain a sequence found in all plasmids in the library. For example, in a case where the promoter library is composed of four types of plasmids:

$$\text{promoter 1 + gene A + promoter 3 + gene B;}$$

$$\text{promoter 2 + gene A + promoter 3 + gene B;}$$

$$\text{promoter 1 + gene A + promoter 4 + gene B;}$$

and

$$\text{promoter 2 + gene A + promoter 4 + gene B,}$$

the gene A and the gene B are common portions, and the combination of, for example, the promoter 1 and the promoter 3 are different portions in the combination. In one embodiment, the elements (for example, a promoter at a specific position) constituting different portions in a combination in a plasmid in a library can have functions (for example, a function as a promoter) similar to the elements of the corresponding positions of other (for example, all) plasmids in the same library, for example, corresponding elements of other species (for example, viruses of other serotypes) or variants produced by modification from the same elements can be used, but are not particularly limited, and have similar functions (for example, a function as a promoter) as in Examples, but biologically unrelated elements may be contained at the corresponding positions of the plasmids.

(Virus-derived construct)

The virus-derived construct plasmid of the present disclosure is used to produce a virus-derived construct. In one embodiment, the present disclosure provides a method for producing a virus-derived construct using the virus-derived

construct plasmid of the present disclosure. In one embodiment, the present disclosure provides a virus-derived construct-containing composition or virus-derived construct produced as described above. Examples of the virus-derived construct include virus-derived constructs based on an alphavirus, a vaccinia virus, a measles virus, an influenza virus, a vesicular stomatitis virus, a coronavirus, a Sindbis virus, a Semliki Forest virus, a herpes simplex virus, a retrovirus, a lentivirus, a rabies virus, a Sendai virus, an adeno-associated virus, an adenovirus, a reovirus, a coxsackievirus, and a Newcastle disease virus. In one embodiment, the virus-derived construct is a virus-derived construct based on a retrovirus, a lentivirus, an adeno-associated virus, or an adenovirus. In one embodiment, the present disclosure provides a method for producing a virus-derived construct library using the virus-derived construct plasmids of the present disclosure and a virus-derived construct library produced by the method.

In one embodiment, the virus-derived construct plasmid of the present disclosure is introduced into a producer cell to produce a virus-derived construct. Examples of the producer cell include, but are not limited to, human embryonic kidney cells (prepared by transfecting human embryonic kidney cells with DNA obtained by cleaving adenovirus 5), 911 cells, PER.C6 cells, E1-transformed amniotic membrane cells, E1-transformed A549 cells, GH329: HeLa cells, HEK293 cells, IT293SF cells, HEK293 T, HEK293 F, Vero cells, CHO cells, Sf9 cells, Freestyle (trademark) 293-F, Expi293-F (trademark), Expi293 inducible, Expi293 NGT-Viral Production Cells 1.0, Viral Production Cells 2.0 (VPC 2.0 cells), AAVpro (registered trademark) 293T Cell Line, Lenti-X (trademark) 293T Cell Line, FreeStyle (trademark) CHO-S cells, ExpiCHO-S (trademark), VirusExpress (trademark) 293 AAV producing cells, and VirusExpress (trademark) 293T lentivirus-producing cells. Any known suitable producer cell can be selected depending on the type of virus-derived construct to be produced. In one embodiment, the virus-derived construct plasmid of the present disclosure is deficient in a small number (for example, 1, 2, 3, 4, or 5) of genes of the origin virus of the virus-derived construct, and may contain all other genes. In one embodiment, the virus-derived construct plasmid of the present disclosure is deficient in a portion of a set of genes required to produce a virus-derived construct, and the genes of the set of genes deficient in the virus-derived construct plasmid are supplied in the producer cell in trans with the virus-derived construct plasmid. In one embodiment, the virus-derived construct plasmid of the present disclosure can be introduced alone into a producer cell without being combined with another plasmid to allow for the production of a virus-derived construct. In one embodiment, the virus-derived construct plasmid of the present disclosure can be introduced into a producer cell expressing all of the genes in which the virus-derived construct plasmid is deficient in the set of genes required to produce a virus-derived construct. For example, the virus-derived construct plasmids of the present disclosure containing AAV cap and rep, and adenoviruses E2A, E4, and VA can be introduced alone into HEK293 cells expressing adenoviruses E1A and E1B to produce virus-derived constructs. In one embodiment, the virus-derived construct plasmid of the present disclosure can be introduced into a producer cell together with another nucleic acid containing a gene in which the virus-derived construct plasmid is deficient in the set of genes required to produce a virus-derived construct or a product of the gene (for example, viral particles) thereof.

In one embodiment, a virus-derived construct can be produced by introducing a virus-derived construct plasmid into a producer cell infected with the origin virus of the virus-derived construct and causing homologous recombination in the cells. The virus-derived construct plasmid used in the embodiment contains nucleic acid sequences having homology to the desired gene and to any region of the genome of the origin virus (for example, regions between genes). In one embodiment, the virus-derived construct plasmid can have a structure containing a desired gene between any genes in the genome of the origin virus of the virus-derived construct to be produced. In one embodiment, at least of a part of the nucleic acids contained in the virus-derived construct plasmid are incorporated into chromosomes of the producer cell.

In one embodiment, the virus-derived construct plasmid of the present disclosure contains segments for cutting loaded nucleic acids of the origin virus of the virus-derived construct to be produced (LTR in a retrovirus, ITR in AAV, and the like), and in one embodiment, the desired gene is contained between the segments. In one embodiment, the virus-derived construct plasmid of the present disclosure contains a desired gene between the segments and a promoter and/or terminator operably linked thereto (for example, those that are operable in the subject to which the virus-derived construct is targeted). In one embodiment, the virus-derived construct plasmids of the present disclosure do not contain the genes required for replication of the origin virus of the virus-derived construct between the segments. In one embodiment, the virus-derived construct plasmid of the present disclosure contains a packaging signal of the origin virus of the virus-derived construct to be produced.

The virus-derived construct produced has a structural protein (capsid or the like), which can be involved in binding to tissue or cell. Therefore, the targeting of the virus-derived construct to tissue or cell can be modulated by modifying the structural protein to modify its binding to the tissue or cell (or a surface structure thereof). In one embodiment, the virus-derived construct plasmids of the present disclosure may contain genes encoding a structural protein, and the structural protein may be modified. For example, the modification of the structural protein that modulates targeting of tissue or cell includes substitution or fusion with other proteins (other viral capsids (for example, capsids of other serotypes of viruses, VSV-G), antigen-binding regions of antibodies, ligand proteins of the subject organism (ligands for cancer antigens), and the like). In addition, a virus-derived construct containing an envelope can also contain cell membrane components of producer cells in the envelope. Therefore, the targeting of the virus-derived construct containing an envelope to tissue or

cell can be modulated by modifying the cell membrane components of the producer cells. In the case of a virus-derived construct without a structural protein, a non-virus-derived material such as a lipid particle can be used to modulate targeting to cells. In one embodiment, the virus-derived construct may be designed to target neural cells (cells of peripheral nervous system or central nervous system, brain cells such as neurons and oligodendrocytes, and the like), lung cells, ocular cells (retinal cells, retinal pigment epithelium, corneal cells, and the like), epithelial cells (for example, intestinal or respiratory epithelial cells), muscle cells (for example, skeletal muscle cells, cardiac muscle cells, smooth muscle cells, and diaphragm muscle cells), dendritic cells, pancreatic cells (islet cells and the like), hepatocytes, cardiomyocytes, bone cells (for example, bone marrow stem cells), hematopoietic stem cells, spleen cells, keratinocytes, fibroblasts, endothelial cells, prostate cells, germ cells, cancer cells, and the like. A surface structure (a receptor or the like) specific to each cell is known, and those skilled in the art can appropriately select a protein that strongly or specifically binds to the surface structure.

In one embodiment, the virus-derived construct plasmids of the present disclosure may contain a gene encoding a protein obtained by attenuating the protein of the origin virus of the virus-derived construct to be produced. The virus-derived construct plasmids of the present disclosure can contain a gene encoding any known attenuated viral protein.

In one embodiment, the present disclosure provides a plasmid-containing composition containing a population of virus-derived construct plasmids of the present disclosure. In one embodiment, the plasmid-containing composition can have a covalently closed circular (CCC) purity of the plasmid of about 60% or more, about 70% or more, about 75% or more, about 80% or more, about 85% or more, about 90% or more, or about 95% or more. The virus-derived construct plasmids of the present disclosure, the virus-derived construct plasmid library, and the virus-derived construct plasmid-containing composition of the present disclosure produced by the method of the present disclosure can have structural characteristics that are not fully analyzable or significantly difficult to analyze, such as in the steric conformation or base modification of plasmids. Therefore, the characteristic of being produced by the method of the present disclosure is one of the characteristics of appropriately expressing the virus-derived construct plasmids, the virus-derived construct plasmid library, and the virus-derived construct plasmid-containing composition of the present disclosure.

• Adenovirus-derived construct

An adenovirus is a virus belonging to the genus Mastadenovirus in the family Adenoviridae, and the viral particle is composed of a nucleocapsid and a double-stranded linear DNA genome, and has an icosahedral structure of 90 to 100 nm. Infection of an adenovirus is initiated by adsorption of the viral capsids to the coxsackie-adenovirus receptor (CAR) on the cell surface, and then the virus can enter into the cell via the integrins on the cell surface. Thereafter, the viral genome that escapes from the lysosome can reach the nucleus, resulting in replication of the viral genome. Viral replication is initiated by first expressing E1A protein and activating expression of other early proteins E1B, E2, E3, and E4. In the viral genome, a complex, in which a terminal protein (TP) expressed from E2 and deoxycytidine are covalently bound to each other and a polymerase is further bound thereto, is formed, and replication is initiated. The genome also contains five late transcription units (L1, L2, L3, L4, and L5) encoding structural proteins, including penton (L2), hexon (L3), scaffold protein (L4), and fiber protein (L5), under the control of a single promoter. Both ends of the genome contain inverted terminal repeats (ITRs) required for virus replication. The structural proteins of the virus are translated in the cytoplasm and then migrate into the nucleus to constitute viral particles, such that the structural proteins recognize the packaging signal ($\psi$) of the viral genome and package the genome. In addition, an adenovirus produces protein-non-coding VA RNAs, which are encoded by the VA gene. The adenoviral particles can have L2 and L3 capsids.

To date, 52 human adenovirus antigenic types have been identified and classified into 6 subgroups based on hemagglutination properties and sequence homology: Subgroup A (for example, serotypes 12, 18, and 31), Subgroup B (for example, serotypes 3, 7, 11, 14, 16, 21, 34, 35, and 50), Subgroup C (for example, serotypes 1, 2, 5, and 6), Subgroup D (for example, serotypes 8, 9, 10, 13, 15, 17, 19, 20, 22 to 30, 32, 33, 36 to 39, and 42 to 48), Subgroup E (for example, serotype 4), Subgroup F (for example, serotypes 40 and 41), and unclassified serotype groups (for example, serotypes 49 and 51).

In one embodiment, the adenovirus-derived construct plasmids contain at least one of the nucleic acid sequences required to construct an adenovirus-derived construct, and a desired gene. In one embodiment, the adenovirus-derived construct plasmid of the present disclosure contains a desired gene between a 5' ITR and a 3' ITR. In one embodiment, the adenovirus-derived construct plasmids contain a desired gene, a promoter, and a terminator between the 5' ITR and the 3' ITR. In one embodiment, the adenovirus-derived construct plasmid does not contain one or a plurality (for example, all) of the nucleic acid sequences required to construct an adenovirus-derived construct between the 5' ITR and the 3' ITR. In one embodiment, the adenovirus-derived construct plasmid is configured to function in a producer cell so that the virus-derived construct plasmid and the producer cell contain the nucleic acid sequences required to construct an adenovirus-derived construct. In one embodiment, one or a plurality (for example, all) of the genes not contained in the virus-derived construct plasmid among the nucleic acid sequences required to construct an adenovirus-derived construct may be encoded by the chromosomes of the producer cell.

In one embodiment, the nucleic acid sequence required to construct an adenovirus-derived construct can contain a gene encoding E1A, E1B, E2A, E2B, E3, E4, L1, L2, L3, L4, L5, IX, and IVa2. In one embodiment, the nucleic acid sequence required to construct an adenovirus-derived construct can be all genes of adenovirus other than E3. In one embodiment, the nucleic acid sequence required to construct an adenovirus-derived construct can contain a nucleic acid sequence encoding a structural protein (L2 or L3), a nucleic acid sequence encoding a packaging factor (E1A, E1B, E2A, E2B, or E4), two terminal repeats (5' ITR and 3' ITR), and a nucleic acid sequence encoding a functional cofactor. In one embodiment, the nucleic acid sequence encoding a functional cofactor can be all adenoviral genes other than L2, L3, E1A, E1B, E2A, E3, E2B, E4, a 5' ITR, and a 3' ITR. In one embodiment, the adenovirus-derived construct plasmid may not contain one or a plurality of VA, E1A, E1B, E2A, E2B, and E4, and in one embodiment, the nucleic acid sequence required to construct an adenovirus-derived construct contains genes. In certain embodiments, the adenovirus-derived construct plasmid does not contain E1A and E1B. In one embodiment, the adenovirus-derived construct plasmid is deficient in E1A, and a desired gene may be inserted into the deficient site. VA RNA is known to interact with exportin, RISK, Dicer, and the like in the human, and the deletion of VA from the adenovirus-derived construct plasmid can reduce side effects to adenovirus-derived construct infected cells. Since E1A, E1B, E2A, E2B, and E4 can be genes required for adenovirus replication, an adenovirus-derived construct plasmid that is deficient in these genes can have reduced replicative ability in infected cells.

In one embodiment, the adenovirus-derived construct of the present disclosure can be derived from human subgroup C, for example, serotype 2 or serotype 5. In one embodiment, the adenovirus-derived construct of the present disclosure can be derived from serotype 12 (Subgroup A), serotype 7 or serotype 35 (Subgroup B), serotype 30 or serotype 36 (Subgroup D), serotype 4 (Subgroup E), or serotype 41 (Subgroup F). Examples of the producer cells for producing an adenovirus-derived construct include cells such as HEK293, HEK293T, HEK293F, Hela, and Sf9.

• Adeno-associated virus-derived construct

An adeno-associated virus (AAV) is a linear, a single-stranded DNA virus from the genus Dependovirus in the family Parvoviridae, with viral particles of 20 to 26 nm in diameter. Adenovirus elements are required for AAV proliferation. At the quantitative end of the AAV genome, there is a T-shaped hairpin structure called an inverted terminal repeat (ITR). A portion of the ITR serves as a origin of replication and serves as a primer. In addition, the ITR is also required for packaging into viral particles and integration into chromosomal DNA of a host cell. In the left half of the genome, there is a rep gene encoding a non-structural protein, that is, a regulatory protein (Rep78, Rep76, Rep52, or Rep40) responsible for replication and transcription, and in the right half of the genome, there is a cap gene encoding three capsid proteins of structural proteins (VP1, VP2, and VP3).

The life cycle of AAV is divided into latent infection and lytic infection. The former is a case of infection by itself, and is characterized by being incorporated into the AAVS1 region (19q13.3-qter) of the long arm of chromosome 19 of the host cell. The incorporation is due to non-homologous recombination and involves Rep. It has been reported that Rep78/Rep76 binds to a base sequence (GAGC repeat sequence) commonly present in the AAVS1 region and the Rep binding region of ITR. Thus, it is considered that when target cells are infected with wild-type AAV, Rep binds to the ITR and AAVS1 of AAV, and site-specific integration of AAV genome into chromosome 19 occurs via Rep. In a case where a helper virus such as an adenovirus is simultaneously infected, when a cell latently infected with AAV is further coinfected with the helper virus, replication of AAV occurs, and a large amount of virus is released due to cell destruction (lytic infection).

In one embodiment, the AAV-derived construct plasmid contains at least one of the nucleic acid sequences required to construct an AAV-derived construct, and a desired gene. In one embodiment, the AAV-derived construct plasmid of the present disclosure contains a desired gene between a 5' ITR and a 3' ITR. In one embodiment, the AAV-derived construct plasmid contains a desired gene, a promoter, and a terminator between the 5' ITR and the 3' ITR. In one embodiment, the AAV-derived construct plasmid does not contain one or a plurality (for example, all) of the nucleic acid sequences required to construct an AAV-derived construct between the 5' ITR and the 3' ITR. In one embodiment, the AAV-derived construct plasmid is configured to function in a producer cell so that the virus-derived construct plasmid and the producer cell contain the nucleic acid sequences required to construct an AAV-derived construct. In one embodiment, one or a plurality (for example, all) of the genes not contained in the virus-derived construct plasmid among the nucleic acid sequences required to construct an AAV-derived construct may be encoded by the chromosomes of the producer cell.

In one embodiment, the nucleic acid sequence required to construct an AAV-derived construct can be a 5' ITR, rep, cap, an assembly activating protein (AAP), a membrane associated accessory protein (MAAP), and a 3' ITR of AAV, and the adenovirus E1A, E1B, E2A, VA, or E4. In one embodiment, the nucleic acid sequence required to construct an AAV-derived construct can include a nucleic acid sequence encoding an AAV structural protein (cap), a nucleic acid sequence encoding a packaging factor for AAV (rep), two terminal repeats of AAV (5' ITR and 3' ITR), and a nucleic acid sequence encoding a functional cofactor (adenovirus E1A, E1B, E2A, VA, or E4). In one embodiment, the AAV-derived construct plasmid may not contain one or a plurality of rep, cap, VA, E1A, E1B, E2A, and E4, and in one embodiment, the nucleic acid sequence required to construct an adenovirus-derived construct contains these sequences.

AAV serotypes 1 to 12 based on capsid have been reported. In addition, serotypes of rh10, DJ, DJ/8, PHP.eB, PHP.S, AAV2-retro, AAV2-QuadYF, AAV2.7m8, AAV6.2, rh.74, AAV2.5, AAV-TT, and Anc80 have also been reported. The AAV-derived construct of the present disclosure may be produced based on AAV of a suitable serotype depending on the target tissue. For example, the relationship of (serotype):(target tissue) may be selected as follows: (AAV1):(mmmmuscle, liver, respiratory tract, and nerve cells), (AAV2):(muscle, liver, and nerve cells), (AAV3):(muscle, liver, and nerve cells), (AAV4):(muscle and ependymal cells), (AAV5):(muscle, liver, nerve cells, glial cells, and respiratory tract), (AAV6):(muscle, liver, respiratory tract, and nerve cells), (AAV7):(muscle and liver), (AAV8):(muscle and liver), and (AAV9):(muscle, liver, and respiratory tract). Examples of the producer cells for producing an AAV-derived construct include cells such as HEK293, HEK293T, HEK293F, Hela, and Sf9. Examples of AAV capsids include wild-type as well as capsids with targeted mutations (AAV2i8, AAV2.5, AAV-TT, AAV9.HR, and the like), capsids with random mutations (AAV-PHP.B and the like), and capsids designed in silico (Anc 80 and the like), and in one embodiment, the AAV-derived construct of the present disclosure may contain these modified capsids and the AAV-derived construct plasmids of the present disclosure may be constructed to encode these modified capsids. As used herein, in a case where a nucleic acid sequence is derived from a particular serotype, it is intended that the nucleic acid sequence may encode a wild-type capsid or a capsid modified as described above based on the wild-type capsid.

• Retrovirus-derived construct

As used herein, the term "retrovirus" generally refers to a virus of the family Retroviridae. A retrovirus is a double-stranded RNA enveloped virus primarily characterized by its ability to reverse transcribe the genome from RNA to DNA. The virion is about 100 to 120 nm in length and in diameter, and contains a dimeric genome of identical positive-stranded RNA complexed with nucleocapsid proteins. The genome is encapsulated in a capsid containing enzyme proteins required for viral infection, that is, reverse transcriptase, integrase, and protease. The matrix proteins form the outer layer of the capsid core that interacts with the envelope, which is a lipid bilayer derived from the host cell membrane, surrounding the viral nuclear particle. Viral envelope glycoproteins that recognize a specific receptor on the host cell and initiate the infection process are immobilized on the bilayer. Envelope proteins are formed by two subunits, transmembrane (TM), which immobilizes proteins within lipid membranes, and surface (SU), which binds to cellular receptors.

Examples of the retrovirus include, but are not limited to, murine leukemia virus (MLV), human immunodeficiency virus (HIV), equine infectious anemia virus (EIAV), mouse mammary tumor virus (MMTV), Rous sarcoma virus (RSV), Fujinami sarcoma virus (FSV), Moloney murine leukemia virus (Mo-MLV), FBR murine osteosarcoma virus (FBR MSV), Moloney murine sarcoma virus (Mo-MSV), Abelson murine leukemia virus (A-MLV), avian myelocytomatosis virus 29 (MC29), avian erythroblastosis virus (AEV), and a lentivirus. Lentiviruses can be divided into a "primate lentivirus" and a "non-primate lentivirus". Examples of the primate lentivirus include human immunodeficiency virus (HIV), which is the causative agent of human acquired immunodeficiency syndrome (AIDS), and simian immunodeficiency virus (SIV). The non-primate lentiviral group includes the prototype "slow virus" visna-maedi virus (VMV), and the related caprine arthritis encephalitis virus (CAEV), equine infectious anemia virus (EIAV), and the more recently described feline immunodeficiency virus (FIV) and bovine immunodeficiency virus (BIV).

During the infection process, the retrovirus first is attached to a specific cell surface receptor. Upon entry into a susceptible host cell, the retroviral RNA genome is copied into DNA by reverse transcriptase. This DNA is transported to the host cell nucleus and then integrated into the host genome, and this state is called a provirus. The provirus is stable in host chromosomes during cell division and is transcribed like other cellular proteins. The provirus encodes the proteins and packaging mechanisms required to produce more viruses, and can sprout and leave the cell. When the retrovirus emerges from host cells, the retrovirus has a host cell lipid membrane. In this way, the host cell-derived membrane protein becomes portion of the retroviral particles.

The retroviral genome contains four genes, gag (group specific antigen), pro (protease), pol (polymerase) and env (envelope). The gag sequence encodes three major structural proteins: a matrix protein, a nucleocapsid protein, and a capsid protein. The pro sequence encodes a protease responsible for cleaving Gag and Gag-Pol during particle assembly, emergence, and maturation. The pol sequence encodes the enzymes of reverse transcriptase and integrase, and the former catalyzes the reverse transcription of the viral genome from RNA to DNA during the infection process, while the latter is responsible for processing the LTR and integrating the proviral DNA into the host cell genome. The env sequence encodes both SU and TM subunits of the envelope glycoprotein. The ability of retrovirus to bind to its target host cells using specific cell surface receptors is conferred by the surface component (SU) of the Env protein, whereas the ability of retrovirus to enter cells via membrane fusion can be conferred by the membrane-anchored transmembrane component (TM). The retroviral genome contains elements required to promote gene expression, reverse transcription, and integration into the host cell chromosome, and examples of the elements include two LTRs (long terminal repeats), a packaging signal ($\psi$) sequence required for specific packaging of viral RNA into a newly formed virion, and a polypurine tract (PPT) that functions as a site to initiate positive-strand DNA synthesis during reverse transcription. Long terminal repeats (LTR) are about 600 nt in length, of which the U3 region is 450, the R sequence is 100, and the U5 region is about 70

nt in length.

The genome of a complex retrovirus, such as a lentivirus, can contain, in addition to gag, pro, pol, and env, accessory genes that regulate viral gene expression, infectious particle assembly, and modulate viral replication in infected cells. A representative lentivirus is HIV-1. The lentivirus can contain two regulatory genes, tat, and rev. For example, HIV-1 further contains vif, vpr, vpu, and nef. In addition, accessory genes such as vpx are present. These accessory genes are involved in the synthesis and processing of viral RNA and control of other replication functions. In particular, HIV also contains structural landmarks (TAR, RRE, PE, SLIP, CRS, and INS) and the like. The lentiviral particles can contain the capsid protein of p24.

In one embodiment, the retrovirus-derived construct plasmid contains at least one of the nucleic acid sequences required to construct a retrovirus-derived construct, and a desired gene. In one embodiment, the retrovirus-derived construct plasmid of the present disclosure contains a desired gene between a 5' LTR and a 3' LTR. In one embodiment, the retrovirus-derived construct plasmid may contain a primer binding site (PBS) and/or a polypurine tract (PPT) between the 5' LTR and the desired gene. In one embodiment, the retrovirus-derived construct plasmid may contain a woodchuck hepatitis virus post-transcriptional regulatory element (WPRE) between the desired gene and the 3' LTR. In one embodiment, the retrovirus-derived construct plasmid is configured to function in a producer cell so that the virus-derived construct plasmid and the producer cell contain the nucleic acid sequences required to construct a retrovirus-derived construct. In one embodiment, one or a plurality (for example, all) of the genes not contained in the virus-derived construct plasmid among the nucleic acid sequences required to construct a retrovirus-derived construct may be encoded by the chromosomes of the producer cell. In one embodiment, env may be replaced with a gene encoding VSV-G (glycoprotein G of vesicular stomatitis virus (VSV)) during construction of a retrovirus-derived construct. In one embodiment, the retrovirus-derived construct (including a lentivirus) plasmid may additionally contain the VSV-G gene. In one embodiment, when the retrovirus-derived construct (including a lentivirus) is constructed, a fusion glycoprotein (FuG-B) in which the glycoprotein gene of rabies virus (RV-G) or the intracellular domain of RV-G is replaced with that of the vesicular stomatitis virus glycoprotein (VSV-G) may be used in addition to or in place of env, and the retrovirus-derived construct (including a lentivirus) plasmids may contain these genes.

In one embodiment, the nucleic acid sequence required to construct a retrovirus-derived construct can contain a nucleic acid sequence encoding a structural protein (gag), a packaging factor ($\psi$), two terminal repeats (5' LTR and 3' LTR), and a nucleic acid sequence encoding a functional cofactor. In one embodiment, the nucleic acid sequence encoding a functional cofactor can be pro, pol, and env. In one embodiment, the retrovirus-derived construct plasmid may not contain one or a plurality of gag, pro, pol, and env, and in one embodiment, the nucleic acid sequence required to construct a retrovirus-derived construct contains these genes. Since pol can be a gene required for retrovirus replication, a retrovirus-derived construct plasmid that is deficient in these genes can have reduced replicative ability in infected cells. In one embodiment, the retrovirus-derived construct plasmid does not contain one or a plurality (for example, all) of the nucleic acid sequences required to construct a retrovirus-derived construct between the 5' LTR and the 3' LTR. In one embodiment, the retrovirus-derived construct plasmid of the present disclosure contains a desired gene between a 5' LTR and a 3' LTR. In one embodiment, the retrovirus-derived construct plasmid contains a desired gene, a promoter, and a terminator between the 5' LTR and the 3' LTR. In one embodiment, the retrovirus-derived construct plasmid may be modified to be deficient in the U3 region of the LTR. Examples of the producer cells for producing a retrovirus-derived construct include cells such as HEK293.

In one embodiment, the nucleic acid sequence required to construct a lentivirus-derived construct can contain a nucleic acid sequence encoding a structural protein (gag, pol, VSV-G, or env), a packaging factor (packaging signal ($\psi$) sequence), two terminal repeats (5' LTR and 3' LTR), and a nucleic acid sequence encoding a functional cofactor. In one embodiment, the nucleic acid sequence encoding a functional cofactor can be rev. In one embodiment, the lentivirus-derived construct plasmid may not contain one or a plurality of rev, gag, pro, pol, and env, and in one embodiment, the nucleic acid sequence required to construct a lentivirus-derived construct contains these genes. Since pol and rev can be a gene required for lentivirus replication, a lentivirus-derived construct plasmid that is deficient in these genes can have reduced replicative ability in infected cells. In one embodiment, the lentivirus-derived construct plasmid may contain one or a plurality of tat, vif, vpr, vpu, nef, vpx, TAR, RRE, PE, SLIP, CRS, INS, APP, MAAP, RPE, PPT, PRE, WRPE, and oPRE. In one embodiment, the lentivirus-derived construct plasmid does not contain one or a plurality (for example, all) of the nucleic acid sequences required to construct a lentivirus-derived construct between the 5' LTR and the 3' LTR. In one embodiment, the lentivirus-derived construct plasmid of the present disclosure contains a desired gene between a 5' LTR and a 3' LTR. In one embodiment, the lentivirus-derived construct plasmid contains a desired gene, a promoter, a terminator, and WPRE between the 5' LTR and the 3' LTR. In one embodiment, the lentivirus-derived construct plasmid may be modified to be deficient in the U3 region of the LTR and TAT. In one embodiment, the retrovirus-derived construct plasmid may be modified to be deficient in the U3 region of the LTR. Examples of the producer cells for producing a lentivirus-derived construct include cells such as HEK293T, HEK293, and Hela.

• Herpes simplex virus-derived construct

Herpes simplex virus (HSV) is a virus of the family Herpesviridae, the subfamily Alphaherpesviridae, and the genus Simplexvirus. The HSV particles have a diameter of about 100 nm and have a structure in which an icosahedral capsid is covered with an envelope. Inside the capsid, linear double-stranded DNA of about 150 kbp is packaged, and the viral DNA genome encodes at least 74 viral proteins. Genes encoding these viral proteins are classified into three groups ($\alpha$, $\beta$, and $\gamma$) depending on the expression timing, and the expression of each group is controlled in a cascade manner. The $\alpha$ gene group includes $\alpha$0, $\alpha$4, $\alpha$22, $\alpha$27, and $\alpha$47, and $\alpha$0, $\alpha$4, $\alpha$22, and $\alpha$27 encode proteins that control the expression of other viral genes. When $\alpha$ genes are expressed, these gene products activate the expression of the $\beta$ gene cluster. The $\beta$ gene group mainly encodes a protein essential for viral DNA replication, such as a DNA polymerase complex and a DNA primase/helicase complex, and an enzyme group involved in deoxyribonucleotide metabolism, such as thymidine kinase (TK) and ribonucleotide reductase. Thereafter, a group of $\gamma$ genes encoding structural proteins of the viral particles is expressed, and new infectious viruses are produced.

Viral particles that enter from sensory nerve endings ascend within the axon and reach the nucleus of the neuronal cell, and begin to proliferate or remain latent. HSV latent in sensory ganglion does not produce infectious viruses, and only a transcript called latency associated transcript (LAT) is constitutively expressed from the viral genome. A latency active promoter (LAP), which is a promoter of LAT, is a promoter that enables permanent gene expression in neurons, which can be useful in gene therapy targeting neurons. The latently infected nerve cell HSV is reactivated and centrifugally transported in the axon, and recurrent onset can occur in the skin and mucosa.

Examples of advantages of an HSV-derived construct include: (1) it can infect and proliferate in most cultured cells in a broad host range; (2) it can infect both dividing and non-dividing cells; (3) it has a high tolerance for loading exogenous genes; (4) it possesses autologous TK so that the antiviral agents acyclovir or ganciclovia are effective and can be treated even when unexpected proliferation occurs; (5) it is stably present as episomes in neuronal cells and can sustain gene expression for a long period of time depending on the promoter; (6) it exhibits similar pathogenicity to a human to a mouse and can be utilized as model animals; and the like. Also, strong cytotoxicity can be beneficial for the treatment of tumors.

In one embodiment, the HSV-derived construct plasmid contains at least one of the nucleic acid sequences required to construct an HSV-derived construct, and a desired gene. In one embodiment, the HSV-derived construct plasmid contains an origin of DNA replication (ori) upstream of the desired gene. In one embodiment, the HSV-derived construct plasmid can contain LAP (for example, between ori and the desired gene), which can result in long-term expression of the introduced gene in the host cell of the HSV-derived construct. In one embodiment, the HSV-derived construct plasmid is configured to function in a producer cell so that the virus-derived construct plasmid and the producer cell contain the nucleic acid sequences required to construct an HSV-derived construct. In one embodiment, one or a plurality (for example, all) of the genes or gene products thereof that are not contained in the virus-derived construct plasmid among the nucleic acid sequences required to construct an HSV-derived construct can be supplied in producer cells in trans with the virus-derived construct plasmid. For example, the gene not contained in the virus-derived construct plasmid may be encoded by the chromosome of the producer cell, or may be encoded by another nucleic acid molecule (for example, a plasmid) to be introduced into the producer cell, or a gene product (which may be in the form of a viral particle) of the gene not contained in the virus-derived construct plasmid may be introduced into the producer cell. In one embodiment, a nucleic acid molecule other than the virus-derived construct plasmid of the present disclosure introduced into the producer cells may be deficient in one or a plurality of the pac and the origin of DNA replication (ori), and it can be avoided that the loaded nucleic acid contains a nucleic acid derived from this nucleic acid.

In one embodiment, the nucleic acid sequence required to construct an HSV-derived construct can be all genes of HSV. In one embodiment, the nucleic acid sequence required to construct an HSV-derived construct can contain genes encoding UL9, UL19, UL26, UL35, US6, US7, and US11. In one embodiment, the nucleic acid sequence required to construct an HSV-derived construct can be all genes of HSV other than one or a plurality of $\alpha$0, $\alpha$22, and $\alpha$47. In one embodiment, the nucleic acid sequence required to construct an HSV-derived construct can be all genes of HSV other than $\alpha$22. In one embodiment, the HSV-derived construct plasmid may not contain one or a plurality of $\alpha$0, $\alpha$4, $\alpha$22, $\alpha$27, and $\alpha$47, and in one embodiment, the nucleic acid sequence required to construct an HSV-derived construct contains these genes. In one embodiment, the HSV-derived construct plasmid is deficient in one or both of the $\alpha$4 and $\alpha$27 genes. In one embodiment, the HSV-derived construct plasmid is deficient in ICP6. In one embodiment, the HSV-derived construct plasmid is deficient in $\alpha$4, $\alpha$22, and $\alpha$27.

In one embodiment, the HSV-derived construct can be modified to attenuate. In one embodiment, the HSV-derived construct plasmid can be modified to be deficient in one or a plurality of thymidine kinase (TK), ribonucleotide reductase (RR), deoxyuridine triphosphatase (dUTPase), and $\gamma$34.5. In one embodiment, the HSV-derived construct plasmid can be modified to introduce a gene encoding a cytokine (such as an immune activating cytokine) at the $\gamma$34.5 deficient site. In one embodiment, an HSV-derived construct that is deficient in these genes can be obtained by producing an HSV-derived construct without supplying these genes to producer cells containing the HSV-derived construct plasmid

that is deficient in these genes. For example, since TK can be highly expressed in cancer cells, the TK-deficient HSV-derived construct can function selectively in cancer cells. Examples of the producer cells for producing an HSV-derived construct include cells such as Hela and Vero.

• Sendai virus-derived construct

Sendai virus (SeV) is a negative-stranded RNA virus classified into the genus Respirovirus of the family Paramyxoviridae. The Sendai virus can also infect non-dividing cells, including neurons. After infection with Sendai virus, the viral genome does not integrate into the host cell chromosomes and remains in the cytoplasm in the RNA state.
Genes encoding proteins of Sendai virus include N, P, M, F, HN, and L genes. The N, P, M, F, HN, and L genes encode nucleocapsid, phospho, matrix, fusion, hemagglutinin-neuraminidase, and large protein, respectively. Generally, on the genome of the wild-type Sendai virus, following a 3' short leader region (LE), six genes encoding N (nucleocapsid protein), P (phosphoprotein), M (matrix protein), F (fusion protein), HN (hemagglutinin-neuraminidase), and L (large protein) are arranged, and the short 5' trailer region (TR) exists at the other end. Accessory proteins called C and V are also translated from the region of the P gene.
The Sendai virus expresses genes by both tubulin in the cytoplasm of the host cell and RNA polymerase (L protein) of the Sendai virus. The Sendai virus does not interact with the genome of the host cell and is not pathogenic to humans. These characteristics of the Sendai virus suggest the safety of the Sendai virus-derived construct in humans. M protein, F protein, and HN protein can be responsible for the formation of viral particles of the Sendai virus and viral infection. N protein, P protein, and L protein can be responsible for expression and replication of the viral genome.
In one embodiment, the nucleic acid transcribed from the Sendai virus-derived construct plasmid in the producer cell is the loaded nucleic acid of the Sendai virus-derived construct. Therefore, the following characteristics relating to the Sendai virus-derived construct plasmid can also be characteristics of the loaded nucleic acid of the Sendai virus-derived construct.
In one embodiment, the Sendai virus-derived construct plasmid contains at least one of the nucleic acid sequences required to construct a Sendai virus-derived construct, and a desired gene. In one embodiment, the desired gene can be located upstream and/or downstream of any of the Sendai virus genes (N, P, M, F, HN, and L genes). In one embodiment, the Sendai virus-derived construct plasmid can contain an EIS sequence (transcription end (E) sequence-intervening (I) sequence-transcription start (S) sequence) upstream or downstream of the desired gene, thereby promoting the expression of the gene upstream or downstream of the desired gene. In one embodiment, the Sendai virus-derived construct plasmid can be modified to insert a sequence having a base number of a multiple of 6 (for example, a sequence containing a desired gene). In one embodiment, the Sendai virus-derived construct plasmid is configured to function in a producer cell so that the virus-derived construct plasmid and the producer cell contain the nucleic acid sequences required to construct a Sendai virus-derived construct. In one embodiment, one or a plurality (for example, all) of the genes or gene products thereof that are not contained in the virus-derived construct plasmid among the nucleic acid sequences required to construct a Sendai virus-derived construct can be supplied in producer cells in trans with the virus-derived construct plasmid.
In one embodiment, the nucleic acid sequence required to construct a Sendai virus-derived construct can be all genes of Sendai virus. In one embodiment, the Sendai virus-derived construct plasmid contains the nucleic acid sequence required to construct a Sendai virus-derived construct between LE and TR. In one embodiment, the nucleic acid sequence required to construct a Sendai virus-derived construct can contain N, P, V, C, M, F, HN, and L genes. In one embodiment, the nucleic acid sequence required to construct a Sendai virus-derived construct can be all genes of Sendai virus other than one or a plurality of M, F, and HN. When using Sendai virus-derived construct plasmid that is deficient in one or a plurality of M, F, and HN genes, the loaded nucleic acids can lose their transferability in the host. In one embodiment, the Sendai virus-derived construct plasmid may not contain one or a plurality of M, F, and HN and in one embodiment, the nucleic acid sequence required to construct a Sendai virus-derived construct contains these genes. In one embodiment, the Sendai virus-derived construct plasmid can contain a gene encoding an RNA polymerase of bacteriophage T7. In one embodiment, the Sendai virus-derived construct plasmid can contain a self-cleaving ribozyme Rbz.
In one embodiment, the Sendai virus gene may be modified to reduce the antigenicity of Sendai virus proteins or to increase RNA transcription and replication efficiency. In one embodiment, in order to enhance the function of transcription or replication, one or a plurality of N gene, P gene, and L gene, which are replication factors, may be modified. In one embodiment, HN protein, which is a structural protein, may be modified, such that the hemagglutinin activity and/or the neuraminidase activity can be changed, the hemagglutinin activity can be weakened to improve the stability of the virus in the blood, and the neuraminidase activity can be changed to change the infectivity of the virus. In one embodiment, F protein involved in membrane fusion may be modified. In one embodiment, V gene, which is an accessory gene, may be modified so as to be deleted. Examples of the producer cells for producing a Sendai virus-derived construct include cells such as BHK/T7.

• Measles virus-derived construct

Measles virus is an RNA virus classified in the genus Paramyxoviridae. Measles virus, like Sendai virus, contains N, P, M, F, H, and L genes.

In one embodiment, the Measles virus-derived construct plasmid contains at least one of the nucleic acid sequences required to construct a Measles virus-derived construct, and a desired gene. In one embodiment, the desired gene can be located upstream and/or downstream of any of the Measles virus genes. In one embodiment, the Measles virus-derived construct plasmid is configured to function in a producer cell so that the virus-derived construct plasmid and the producer cell contain the nucleic acid sequences required to construct a Measles virus-derived construct. In one embodiment, one or a plurality (for example, all) of the genes or gene products thereof that are not contained in the virus-derived construct plasmid among the nucleic acid sequences required to construct a Measles virus-derived construct can be supplied in producer cells in trans with the virus-derived construct plasmid. In one embodiment, the nucleic acid sequence required to construct a Measles virus-derived construct can be all genes of Measles virus.

• Alphavirus-derived construct

As used herein, the term "alphavirus" refers to a virus in the genus Alphavirus in the family Togaviridae. An alphavirus is an enveloped RNA virus that can proliferate in the cytoplasm without passing through intermediates of DNA and infect various cells via a laminin receptor and the like. The genome of the alphavirus is a single-stranded messenger-sense RNA, and is modified at the 5' end with a methylation cap and is modified at the 3' end with a variable-length poly(A) tract Viral particles have a structure containing an RNA genome in an icosahedral nucleocapsid. The genome of the alphavirus contains genes encoding non-structural proteins of nsp1, nsp2, nsp3 and nsp4, and genes encoding structural proteins of capsid (C) protein and E1 and E2 glycoproteins. Examples of the alphavirus include Venezuelan equine encephalitis (VEE) virus, Semliki forest virus (SFV), Sindbis virus, Ross River virus, Western equine encephalitis virus, Eastern equine encephalitis virus, Chikungunya virus, S.A.AR86, Everglades virus, Mucambo Virus, Barmah forest virus, Middelburg virus, Pixuna virus, O'nyong'nyong virus, Getah virus, Sagiyama virus, Beval virus, Mayaro virus, Una virus, Aura virus, Whataroa virus, Babanki virus, Kyzylagach virus, Highlands J virus, Fort Morgan virus, Ndumu virus, and Buggy Creek virus.

In one embodiment, the alphavirus-derived construct plasmid contains at least one of the nucleic acid sequences required to construct an alphavirus-derived construct, and a desired gene. In one embodiment, the desired gene may be located between any of nsp1, nsp2, nsp3, and nsp4. In one embodiment, the alphavirus-derived construct plasmid is configured to function in a producer cell so that the virus-derived construct plasmid and the producer cell contain the nucleic acid sequences required to construct an alphavirus-derived construct. In one embodiment, one or a plurality (for example, all) of the genes or gene products thereof that are not contained in the virus-derived construct plasmid among the nucleic acid sequences required to construct an alphavirus-derived construct can be supplied in producer cells in trans with the virus-derived construct plasmid. For example, the gene not contained in the virus-derived construct plasmid may be encoded by the chromosome of the producer cell, or may be encoded by another nucleic acid molecule (for example, a plasmid) to be introduced into the producer cell, or a gene product (which may be in the form of a viral particle) of the gene not contained in the virus-derived construct plasmid may be introduced into the producer cell. In one embodiment, a nucleic acid molecule other than the virus-derived construct plasmid of the present disclosure introduced into the producer cells may be deficient in one or a plurality of a 5' alphavirus replication recognition sequence and a 3' alphavirus replication recognition sequence, and it can be avoided that the loaded nucleic acid contains a nucleic acid derived from this nucleic acid.

In one embodiment, the nucleic acid sequence required to construct an alphavirus-derived construct can be all genes of the alphavirus. In one embodiment, the alphavirus-derived construct plasmid may not contain one or a plurality of alphavirus genes other than the 5' alphavirus replication recognition sequence, nsp1, nsp2, nsp3, nsp4, and the 3' alphavirus replication recognition sequence, and in one embodiment, the nucleic acid sequences required to construct an alphavirus-derived construct contain these genes.

• Rabies virus-derived construct

A rabies virus is a negative-stranded single-stranded RNA virus of the genus Lyssavirus in the family Rhabdoviridae, and the viral particles are bullet-shaped and cylindrical. The cylinder has a length of about 180 nm and a diameter of about 75 nm. The viral particle contains five genes encoding L (large protein), G (glycoprotein), N (nucleoprotein), P (phosphoprotein), and M (matrix protein). Among them, G protein may be associated with infectivity. In the genome of the rabies virus, genes are arranged in the order of N, P, M, G, and L from the leader site. The expression level can increase as the gene is closer to the leader site.

In one embodiment, the rabies virus-derived construct plasmid contains at least one of the nucleic acid

sequences required to construct a rabies virus-derived construct, and a desired gene. In one embodiment, the desired gene can be located upstream and/or downstream of any of N, P, M, G, and L genes. In one embodiment, the rabies virus-derived construct plasmid is configured to function in a producer cell so that the virus-derived construct plasmid and the producer cell contain the nucleic acid sequences required to construct a rabies virus-derived construct. In one embodiment, one or a plurality (for example, all) of the genes or gene products thereof that are not contained in the virus-derived construct plasmid among the nucleic acid sequences required to construct a rabies virus-derived construct can be supplied in producer cells in trans with the virus-derived construct plasmid. In one embodiment, the nucleic acid sequences required to construct a rabies virus-derived construct may be all genes of the rabies virus. In one embodiment, the rabies virus-derived construct plasmid may not contain one or more of N, P, M, G, and L.

• Vesicular stomatitis virus-derived construct

Vesicular stomatitis virus (VSV) is an RNA virus belonging to the genus Vesiculovirus in the family Rhabdoviridae, and retains a negative single-stranded RNA genome of about 11 kb. The vesicular stomatitis virus (VSV) contains five genes encoding L (large protein), G (glycoprotein), N (nucleoprotein), P (phosphoprotein), and M (matrix protein), similar to the rabies virus.

In one embodiment, a VSV-derived construct plasmid contains at least one of the nucleic acid sequences required to construct a VSV-derived construct, and a desired gene. In one embodiment, the desired gene can be located upstream and/or downstream of any of N, P, M, G, and L genes. In one embodiment, the VSV-derived construct plasmid is configured to function in a producer cell so that the virus-derived construct plasmid and the producer cell contain the nucleic acid sequences required to construct a VSV-derived construct. In one embodiment, one or a plurality (for example, all) of the genes or gene products thereof that are not contained in the virus-derived construct plasmid among the nucleic acid sequences required to construct a VSV-derived construct can be supplied in producer cells in trans with the virus-derived construct plasmid. In one embodiment, the nucleic acid sequence required to construct a VSV-derived construct can be all genes of VSV. In one embodiment, the VSV-derived construct plasmid may not contain one or more of N, P, M, G, and L.

• Coronavirus-derived construct

A viral particle of a virus belonging to the family Coronavirus (CoV) shows a circle (spherical shape) having a diameter of about 80 to 100 nm, and in an envelope enclosing a nucleocapsid, a spike (S) protein, a membrane integral (M) protein, and an envelope (E) protein are present. In the genome of CoV, genes encoding 1a and 1b non-structural proteins from a 5' side, which are about 30 kb of (+) strand RNAs, and S, E, M, and N gene, which are structural genes located downstream thereof, are present, and genes of some non-structural proteins in the region immediately below S gene are present.

In one embodiment, the coronavirus-derived construct plasmid contains at least one of the nucleic acid sequences required to construct a coronavirus-derived construct, and a desired gene. In one embodiment, the coronavirus-derived construct plasmid of the present disclosure contains a desired gene between a 5' ITR and a 3' ITR. In one embodiment, the coronavirus-derived construct plasmid is configured to function in a producer cell so that the virus-derived construct plasmid and the producer cell contain the nucleic acid sequences required to construct a coronavirus-derived construct. In one embodiment, one or a plurality (for example, all) of the genes or gene products thereof that are not contained in the virus-derived construct plasmid among the nucleic acid sequences required to construct a coronavirus-derived construct can be supplied in producer cells in trans with the virus-derived construct plasmid.

In one embodiment, the nucleic acid sequences required to construct a coronavirus-derived construct may be all genes of the coronavirus. In one embodiment, the coronavirus-derived construct plasmid may not contain one or a plurality of coronavirus genes other than 1a and 1b, and in one embodiment, the nucleic acid sequence required to construct a coronavirus-derived construct contains these genes.

• Influenza virus-derived construct

An influenza virus is an enveloped virus having a negative-stranded RNA genome belonging to the family Orthomyxoviridae. The genome of Type A influenza virus is segmented into 8 segments, and encodes 11 types of proteins (PB1, PB2, PA, HA, NA, M1, M2, NS1, NS2/NEP, and NP). The 5'-terminal untranslated region, the 3'-terminal untranslated region, and both ends of the translated region of the influenza virus RNA can be packaging sequences.

In one embodiment, influenza virus-derived construct plasmid can contain a 5'-terminal untranslated region of the segmented influenza virus RNA, a 5'-end of the translated region, a desired gene, a 3'-end of the translated region, and a 3'-terminal untranslated region. Here, the genes of the influenza virus may be removed or maintained. In one embodiment, the retrovirus-derived construct plasmid may contain nucleic acid sequences required to construct an

influenza virus. In one embodiment, the nucleic acid sequences required to construct an influenza virus can be PB1, PB2, PA, HA, NA, M1, M2, NS1, NS2/NEP and NP. In one embodiment, the influenza virus-derived construct plasmid is configured to function in a producer cell so that the virus-derived construct plasmid and the producer cell contain the nucleic acid sequences required to construct an influenza virus-derived construct. In one embodiment, one or a plurality (for example, all) of the genes or gene products thereof that are not contained in the virus-derived construct plasmid among the nucleic acid sequences required to construct an influenza virus-derived construct can be supplied in producer cells in trans with the virus-derived construct plasmid.

• Vaccinia virus-derived construct

Vaccinia virus belongs to the family Poxviridae and is an enveloped virus containing a linear double-stranded DNA genome of about 190 Kbp. Vaccinia virus replicates only in the cytoplasm of a host cell. During viral DNA replication, vaccinia virus produces several infectious forms which differ in their outer membranes: intracellular mature virions (IMVs), intracellular enveloped virions (IEVs), cell-associated enveloped virions (CEVs), and extracellular enveloped virions (EEVs).

In one embodiment, a vaccinia virus-derived construct plasmid contains nucleic acid sequences required to construct a vaccinia virus-derived construct, and a desired gene. In one embodiment, the nucleic acid sequences required to construct a vaccinia virus-derived construct can encode D1R, D2L, D3R, D4R, D5R, D6R, D7R, D8L, D11L, and D13L genes. In one embodiment, the vaccinia virus-derived construct plasmid may be deficient in a gene encoding thymidine kinase. In one embodiment, a vaccinia virus-derived construct can be produced by introducing a virus-derived construct plasmid into a producer cell infected with a vaccinia virus and causing homologous recombination in the cells. The vaccinia virus-derived construct plasmid used in the embodiment contains nucleic acid sequences having homology to the desired gene and to any region of the genome of the vaccinia virus. In one embodiment, the vaccinia virus-derived construct plasmid can contain a desired gene between the vaccinia virus genome and any of the vaccinia virus genes.

• Reovirus-derived construct

A virus of the family Reoviridae is an RNA virus containing icosahedral virions having a diameter of about 60 to 80 nm, a genome of 10 to 12 linear double-stranded RNAs, and no envelope. Viruses of the family Reoviridae include a rotavirus, mammalian orthoreovirus (MRV), and the like. MRV is classified into four serotypes, MRV-1, MRV-2, MRV-3, and MRV-4. 10-segmented dsRNA genomes encode 8 structural proteins: λ1 (L3 gene), λ2 (L2 gene), λ3 (L1 gene), μ1 (M2 gene), μ2 (M1 gene), σ1 (S1 gene), σ2 (S2 gene), σ3 (S4 gene), and 4 non-structural proteins: μNS (M3 gene), μNSC (M3 gene), σNS (S3 gene), and σ1s (S1 gene). MRV is composed of capsids having a two-layer structure, the outer shell is composed of μ1, σ3, and σ1, and the inner shell (core structure) is composed of λ1, λ2, and σ2. The core particle contains λ3, which is an RNA-dependent RNA polymerase, and μ2, which is a polymerase cofactor, in addition to the 10-segmented genomes. The non-structural protein μNS plays a central role in the formation of VF, which is a field of replication, and binds to various MRV proteins and recruits them into VF. μNSCs may not be essential for replication in cultured cells. σNS interacts with μNS, has a high binding ability to a single-stranded RNA, and can be involved in recruitment of a positive-stranded viral RNA into VF. σ1s is not essential for virus replication, but may be involved in pathogenesis.

In one embodiment, a reovirus-derived construct plasmid contains at least one of the nucleic acid sequences required to construct a reovirus-derived construct, and a desired gene. In one embodiment, the nucleic acid sequence required to construct a reovirus-derived construct can contain L1, L2, L3, M1, M2, M3, S1, S2, S3, and S4 genes. In one embodiment, the reovirus-derived construct plasmid can contain L1, L2, L3, M1, M2, M3, S1, S2, S3, and S4 genes. In one embodiment, the reovirus-derived construct plasmid may not encode at least one of μNSC and σ1s. In one embodiment, the reovirus-derived construct plasmid is configured to function in a producer cell so that the virus-derived construct plasmid and the producer cell contain the nucleic acid sequences required to construct a reovirus-derived construct. In one embodiment, one or a plurality (for example, all) of the genes or gene products thereof that are not contained in the virus-derived construct plasmid among the nucleic acid sequences required to construct a reovirus-derived construct can be supplied in producer cells in trans with the virus-derived construct plasmid. In one embodiment, the nucleic acid sequence required to construct a reovirus-derived construct can be all genes of reovirus. In one embodiment, the reovirus-derived construct plasmid may not contain one or a plurality of L1, L2, L3, M1, M2, M3, S1, S2, S3, and S4. Examples of the producer cells for producing a reovirus-derived construct include cells such as L929.

• Coxsackievirus-derived construct

A coxsackie virus is a non-enveloped, linear, single-stranded, positive-stranded RNA virus belonging to the genus Enterovirus in the family Picornaviridae. The coxsackie virus, which is an enterovirus, is composed of genes VP0 (VP4 and VP2), VP3, VP1, 2A, 2B, 2C, 3A, 3B, 3C, and 3D from 5' to 3', VP gene encodes a capsid, and the other genes

encode non-structural proteins. VP4 behaves like the amino terminus of VP2.

In one embodiment, a coxsackie virus-derived construct plasmid contains at least one of the nucleic acid sequences required to construct a coxsackie virus-derived construct, and a desired gene. In one embodiment, the desired gene can be located upstream and/or downstream of any of VP0, VP3, VP1, 2A, 2B, 2C, 3A, 3B, 3C, and 3D genes. In one embodiment, the coxsackie virus-derived construct plasmid is configured to function in a producer cell so that the virus-derived construct plasmid and the producer cell contain the nucleic acid sequences required to construct a coxsackie virus-derived construct. In one embodiment, one or a plurality (for example, all) of the genes or gene products thereof that are not contained in the virus-derived construct plasmid among the nucleic acid sequences required to construct a coxsackie virus-derived construct can be supplied in producer cells in trans with the virus-derived construct plasmid. In one embodiment, the nucleic acid sequences required to construct a coxsackie virus-derived construct may be all genes of the coxsackie virus. In one embodiment, the nucleic acid sequences required to construct a coxsackie virus-derived construct may be 2A, 2B, 2C, 3A, 3B, 3C, 3D, VP1, VP2, VP3, and VP4. In one embodiment, the coxsackie virus-derived construct plasmid may not contain one or a plurality of VP0, VP3, VP1, 2A, 2B, 2C, 3A, 3B, 3C, and 3D. Examples of the producer cells for producing a coxsackie virus-derived construct include cells such as H1299 and HeLa.

• Newcastle disease virus-derived construct

Newcastle disease virus (NDV) is an enveloped linear, negative single-stranded RNA virus classified into the family Paramyxoviridae. From 3’ to 5’, the genomic RNA of NDV contains the genes for nucleocapsid protein (NP), phosphoprotein (P), matrix protein (M), fusion protein (F), hemagglutinin-neuraminidase (HN), and large protein (L). The genomic RNA also contains a leader sequence at the 3’ end. Fusion protein (F) is an integral membrane protein that is activated to promote fusion of the viral envelope with the host cell membrane. Matrix protein (M) is involved in a viral construct and interacts with viral membranes and nucleocapsid proteins. Nucleocapsid protein (NP) is the main protein of nucleocapsid and associates with phosphoprotein (P) and large protein (L). Phosphoprotein (P) undergoes phosphorylation and can be involved in transcriptional regulation, methylation, phosphorylation, and polyadenylation. Large protein (L) gene encodes an RNA-dependent RNA polymerase and is required for viral RNA synthesis along with P protein.

In one embodiment, an NDV-derived construct plasmid contains at least one of the nucleic acid sequences required to construct an NDV-derived construct, and a desired gene. In one embodiment, the desired gene can be located upstream and/or downstream of any of NP, P, M, F, HN, and L genes. In one embodiment, the NDV-derived construct plasmid is configured to function in a producer cell so that the virus-derived construct plasmid and the producer cell contain the nucleic acid sequences required to construct an NDV-derived construct. In one embodiment, one or a plurality (for example, all) of the genes or gene products thereof that are not contained in the virus-derived construct plasmid among the nucleic acid sequences required to construct an NDV-derived construct can be supplied in producer cells in trans with the virus-derived construct plasmid. In one embodiment, the nucleic acid sequence required to construct an NDV-derived construct can be all genes of NDV. In one embodiment, the NDV-derived construct plasmid may not contain one or more of NP, P, M, F, HN, and L.

In one embodiment, the present disclosure provides a virus-derived construct-containing composition containing a population of virus-derived constructs of the present disclosure. In one embodiment, the virus-derived construct-containing composition may have a viral vector particle number with respect to a viral vector genome copy number (VP/VG) of 35, 30, 25, 20, 15, 10, or 5 or less.

In one embodiment, the virus-derived construct plasmids produced from packaging cells can be purified using any known method, and the present disclosure also provides the thus purified virus-derived construct plasmids. In one embodiment, the purified virus-derived construct plasmids containing a desired nucleic acid sequence can be confirmed by examining the size pattern of fragments generated by restriction enzyme cleavage, a PCR method, a base sequencing method, or the like. In one embodiment, the virus-derived construct plasmid-containing composition prepared by the method for producing virus-derived construct plasmids of the present disclosure may contain trace endotoxins. In one embodiment, there is provided Bacillus subtilis containing the virus-derived construct plasmids of the present disclosure.

(Composition)

In one embodiment, the present disclosure provides a composition containing the virus-derived construct plasmids or virus-derived constructs described As used herein. The virus-derived construct of the present disclosure, the virus-derived construct library, and the virus-derived construct-containing composition of the present disclosure produced by the method of the present disclosure can have structural characteristics that are not fully analyzable or significantly difficult to analyze, such as in the conformation or surface modification of the virus-derived construct. Therefore, the characteristic of being produced by the method of the present disclosure is one of the characteristics of appropriately expressing the virus-derived construct, the virus-derived construct library, and the virus-derived construct-containing composition of the present disclosure.

(Formulation and the like)

The composition described As used herein can be provided in a variety of forms. The form of the composition may be, for example, an injection, a capsule, a tablet, a granule, an inhalant, or the like. An aqueous solution for injection may be stored, for example, in a vial or in a stainless container. In addition, the aqueous solution for injection may contain, for example, physiological saline, sugar (for example, trehalose), NaCl, NaOH, or the like.

In one embodiment, the composition of the present disclosure contains a pharmaceutically acceptable carrier or excipient. Such a carrier can be an aseptic liquid, for example, water or oil, includes a carrier derived from petroleum, an animal, a plant, or a synthetic origin, and includes, but is not limited to, peanut oil, soybean oil, mineral oil, sesame oil, and the like. When a medicine is orally administered, water is a preferred carrier. When the pharmaceutical composition is administered intravenously, saline and aqueous dextrose are preferred carriers. Preferably, an aqueous saline solution, and aqueous dextrose and a glycerol solution are used as a liquid carrier for an injectable solution. Examples of a preferred excipient include light anhydrous silicic acid, crystalline cellulose, mannitol, starch, glucose, lactose, sucrose, gelatin, malt, rice, wheat flour, chalk, silica gel, sodium stearate, glycerol monostearate, talc, sodium chloride, powdered skim milk, glycerol, propylene, glycol, water, ethanol, Carmellose calcium, Carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, poloxamer, saccharose, carboxymethylcellulose, corn starch, and an inorganic salt. The composition can contain a small amount of a wetting agent or an emulsifier, or a pH buffer, if desired. These compositions can be in the form of a solution, suspension, emulsion, tablet, pill, capsule, powder, sustained release mixture, or the like. The composition can also be formulated as a suppository using a traditional binding agent and carrier, for example, triglyceride. Oral formulation can also contain a standard carrier such as medicine grade mannitol, lactose, starch, magnesium stearate, sodium saccharin, cellulose, or magnesium carbonate. Examples of a preferred carrier are described in E. W. Martin, Remington's Pharmaceutical Sciences (Mark Publishing Company, Easton, U.S.A). In addition, for example, a surfactant, an excipient, a coloring agent, a flavoring agent, a preservative, a stabilizer, a buffer, a suspension, an isotonizing agent, a binder, a disintegrant, a lubricant, a fluidity accelerator, a corrigent, or the like may be contained. In one embodiment, the pH of any liquid composition of the present disclosure can be about 3, about 3.5, about 4, about 4.5, about 5, about 5.5, about 6, about 6.5, about 7, about 7.5, about 8, about 8.5, about 9, about 9.5, about 10, about 10.5, about 11, or a range between any two of these values.

The optional ingredient of the composition of the present disclosure can be provided as a pharmaceutically acceptable salt, and can be, for example, a salt formed with a free carboxyl group derived from hydrochloric acid, phosphoric acid, acetic acid, oxalic acid, tartaric acid, or the like, a salt formed with a free amine group derived from isopropylamine, triethylamine, 2-ethylaminoethanol, histidine, procaine, or the like, and a salt derived from sodium, potassium, ammonium, calcium, ferric hydroxide, or the like.

In a preferred embodiment, a composition can be formulated as a pharmaceutical composition adapted for administration to humans according to a known method. Such a composition can be administered by injection. A composition for use in injection administration is typically a solution in an aseptic isotonic aqueous buffer. The composition can also contain a solubilizing agent and a local anesthetic agent such as lidocaine which alleviates the pain at the site of injection as necessary. In general, the ingredients can be supplied separately or mixed and supplied together in a unit dosage form, and supplied as a lyophilized powder or water free concentrate, for example, in a sealed container such as an ampoule or sachet showing the amount of active agent. When the composition is to be administered by injection, the composition can be distributed by using an injection bottle containing aseptic agent-grade water or saline. When the composition is to be administered by injection, aseptic water or saline ampoule for injection can also be provided so that the ingredients can be mixed prior to administration.

(Use and application)

The virus-derived construct plasmid or virus-derived construct described As used herein, or a composition containing the same, can be used in a variety of applications, such as gene therapy, functional genomics, cancer vaccination, and/or antiviral vaccination.

When the virus-derived construct or the virus-derived construct-containing composition of the present disclosure is applied to a subject, the subject is not particularly limited, and may be a mammal (for example, a mouse, a rat, a hamster, a rabbit, a cat, a dog, a cow, sheep, a pig, a monkey, a human, or the like), a bird, a reptile, an amphibian, an arthropod, a fish, or the like.

The amount of the virus-derived construct or the virus-derived construct-containing composition of the present disclosure can vary depending on the nature of the disorder or condition to be treated or prevented, and can be determined by those skilled in the art by a standard clinical technique based on the descriptions As used herein. An in vitro assay can be used in some cases to assist the identification of the optimal dosing range. The precise dose to be used in a formulation can also vary depending on the administration route or the severity of the disease or disorder. Thus, the dose should be

determined in accordance with the judgment of the attending physician or the condition of each patient. A dosage of the virus-derived construct or the virus-derived construct-containing composition of the present disclosure is not particularly limited, and may be, for example, $1 \times 10^5$, 1 x $10^6$, $1 \times 10^7$, $1 \times 10^8$, $1 \times 10^9$, $1 \times 10^{10}$, $1 \times 10^{11}$, $1 \times 10^{12}$, $1 \times 10^{11}$, $1 \times 10^{14}$, or $1 \times 10^{15}$ virus-derived constructs per dose, and may be within a range of any two of these values. The dosing interval is not particularly limited, and may be, for example, 1 or 2 administrations every 1, 7, 14, 21, or 28 days or 1 or 2 administrations per range of any two of these values. The dosage, dosing interval, and dosing method may be appropriately selected depending on the age, weight, symptom, target organ, or the like of the patient.

The administration route of the virus-derived construct or the virus-derived construct-containing composition described As used herein may be, for example, intravenous, intradermal, subcutaneous, intramuscular, intraperitoneal, intrathecal, intraventricular, intraparenchymal, pulmonary, intranasal, epidural, or oral administration. In one embodiment, the composition of the present disclosure can be used together with various delivery systems. Such systems include, for example, encapsulation in liposomes, microparticles, and microcapsules, the use of receptor-mediated endocytosis, and the like. It is also possible to administer the medicine by a suitable route, for example, by injection, bolus injection, or absorption through epithelial or mucocutaneous lining (for example, oral cavity, rectum, intestinal mucosa, or the like). In addition, an inhaler or nebulizer using an aerosolizing agent can be used as necessary. In addition, other drugs can also be administered together. The administration can also be systemic or local.

The composition of the present disclosure can be provided as a kit. In one embodiment, the present disclosure provides a kit for producing a virus-derived construct, the kit containing the virus-derived construct plasmids of the present disclosure. In one embodiment, the present disclosure provides a drug pack or kit including one or more containers filled with one or more components that can be added to the composition of the present disclosure. In some cases, information indicating approval of manufacture, use, or sale for administration to humans by a government agency regulating the manufacture, use, or sale of medicines or biological products in a stipulated form can be appended to such a container.

The formulation procedure for the composition of the present disclosure as a medicine or the like is known in the art, and is described, for example, in the Japanese Pharmacopoeia, the United States Pharmacopeia, pharmacopeia of other countries, or the like. Accordingly, those skilled in the art can determine the embodiment, such as the amount to be used, without undue experimentation from the descriptions As used herein.

As used herein, "or" is used when "at least one or more" of the items listed in the text can be employed. The same applies to "or". When explicitly described As used herein as "within the range" of "two values", the range also includes the two values themselves.

Reference literatures such as scientific literatures, patents, and patent applications cited As used herein are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described As used herein, but is limited only by the claims.

## Examples

For reagents, the specific products described in the Examples were used. However, the reagent can be substituted with an equivalent product from another manufacturer (Sigma-Aldrich, Wako Pure Chemical Industries, Ltd., Nacalai Tesque INC., R&D Systems, USCN Life Science INC, or the like).

### Medium

LB medium was prepared by dissolving 10 g of Bactotryptone, 5 g of a yeast extract, and 5 g of sodium chloride in 1 L of water, and when preparing an agar plate, further adding 15 g of Bacto Agar, and performing autoclaving (121°C, 20 minutes). Carbenicillin (final concentration: 100 μg/mL) or tetracycline (final concentration: 10 μg/mL) was added thereto and used as necessary. TF-I medium and TF-II medium for Bacillus subtilis transformation were prepared as follows. First, 10 × Spizizen (140 g $K_2HPO_4$ (anhydrous), 60 g $KH_2PO_4$ (anhydrous), 20 g $(NH_4)_2SO_4$ per 1 L), 10 g $Na_3$-citric acid·$_2H_2O$), 50% glucose, 2% $MgSO_4 \cdot 7H_2O$, 2% casamino acid, and water were individually prepared by autoclaving. In addition, an aqueous solution (5 mg/mL) of each amino acid of tryptophan, arginine, leucine, and threonine was prepared by filter sterilization. 50 mL of 10 × Spizizen, 5 mL of each of 50% glucose, 2% $MgSO_4 \cdot 7H_2O$, 2% casamino acid, and 5 mg/mL of each amino of tryptophan, arginine, leucine, and threonine, and finally 415 mL of sterile water were mixed and filtered through a filter to prepare TF-I medium (500 mL), and the TF-I medium was stored at 4°C until use. TF-II medium (500 mL) was prepared by filter filtration using the same medium as the TF-I medium except that 2.5 mL of 2% casamino acid, 0.5 mL of each amino acid solution (5 mg/mL), and 435.5 mL of sterile water were used, and the medium was stored at 4°C until use.

In vitro gene manipulation

Other common DNA manipulations were performed according to standard protocols (Sambrook, J., et al., Molecular Cloning: A Laboratory Manual. Cold Spring Harbor Laboratory Press, Cold Spring Harbor, New York (1989)).

Bacillus subtilis transformation method

2 mL of LB medium was placed in a 14 mL test tube (2051) of Falcon, and Bacillus subtilis in a glycerol stock stored at -70°C was inoculated therein, and cultured at 37°C for 17 hours while rotating by a rotary culture apparatus. 900 μL of TF-I medium was dispensed into a new 14 mL test tube (Falcon 2051), 25 μL of 2% casamino acid was added, 50 μL of a preculture solution was added, and the cells were cultured at 37°C for 4 hours while being rotated by the rotary culture apparatus. Thereafter, 900 μL of TF-II medium was dispensed into a new 14 mL test tube, 100 μL of TF-I culture solution was added thereto, and the cells were cultured at 37°C for 1.5 hours while being rotated by the rotary culture apparatus. 100 μL of TF-II culture solution was placed in a 1.5 mL centrifuge tube, and 8 μL of DNA was added thereto. The cells were cultured at 37°C for 30 minutes while being rotated by a rotary culture apparatus, 300 μL of LB medium was then added, and the cells were further cultured at 37°C for 1 hour while being rotated by the rotary culture apparatus. Thereafter, the resultant was spread on an LB medium agar plate containing 10 μg/mL of tetracycline and incubated at 37°C overnight to obtain a transformant.

Mass preparation of plasmids by ultracentrifugation

After completion of the culture, the cells were dispensed into four 50 mL tubes (Falcon 2070) by 50 mL, and centrifuged at 5,000 × g for 10 minutes. The supernatant was discarded, and the bacterial pellets were completely loosened by vortexing. In the case of Escherichia coli, P1 Buffer (Qiagen N.V.) was used alone, and in the case of Bacillus subtilis, P1 Buffer (Qiagen N.V.) solution to which 10 mg/mL lysozyme and 10 mg/mL ribonuclease A were added was used, 5 mL of the solution was added to each of the four tubes containing bacteria by 5 mL, and mixing was performed well. The resultant was incubated at room temperature for 5 minutes. To each of the four tubes, 5 mL of P2 Buffer (Qiagen N.V.) was added and mixed slowly, and incubation was performed at room temperature for 5 minutes. Furthermore, P3 Buffer (Qiagen N.V.) was added by 5 mL, and mixing was performed with a strong force to some extent so that the cloudy substances were uniformly dispersed. Centrifugation was performed at 5,000 × g for 10 minutes and the supernatant was pipetted and transferred to new four 50 mL screw-cap tubes (Falcon 2070). 5 mL of phenol saturated TE was added to each tube, and mixing was performed vigorously. Centrifugation was performed at 5,000 × g for 10 minutes and the supernatant was pipetted and transferred to new four 50 mL screw-cap tubes (Falcon 2070). 100% ethanol was added by 20 mL and mixed, and the mixture was centrifuged at 5,000 × g for 10 minutes to remove the supernatant. 5.4 mL of TE was added to the precipitate and completely dissolved. Next, 6.40 g of cesium chloride was charged and completely dissolved. Further, 2.6 mL of a 1.1 g/mL cesium chloride solution (solution prepared by mixing 1.1 g of cesium chloride and 1 mL of water, volume not adjusted) was added. Finally, 600 μL of 10 mg/mL an ethidium bromide solution was added and mixed well. The resultant was transferred to one ultracentrifuge tube (Beckman 362181). Water or a 1.1 g/mL cesium chloride solution (specific gravity: about 1.5 g/mL) was added to finely adjust the weight so that the difference in weight from the balance was within 20 mg. Centrifugation was performed using an ultracentrifuge (Beckman Coulter, Inc.) under the following conditions. Temperature: 18°C, speed: 50,000 rpm, acceleration: Max, deceleration: Max. The centrifugation was performed for 15 hours or longer.

After completion of the centrifugation, the resultant was inserted into a band of a ccc type plasmid with a 1 mL syringe set with a needle (21G × 5/8") under observation with ultraviolet rays (365 nm), and the plasmid solution was recovered and transferred to a 15 mL tube. 500 μL of P3 was added thereto, and then, water was added so that the total volume was 3 mL. In addition, 9 mL of 100% ethanol was added. Centrifugation was performed at 5,000 × g for 10 minutes, and the supernatant was removed. 700 μL of TE was added to the resulting precipitate to dissolve DNA. The resultant was transferred to a 1.5 mL tube, 600 μL of 1-butanol was added and mixed, the mixture was centrifuged at 20,000 × g for about 10 seconds and separated into two layers, and the upper butanol layer was discarded. 600 μL of 1-butanol was newly added and mixed, the mixture was centrifuged at 20,000 × g for about 10 seconds and separated into two layers, and the upper butanol layer was discarded. This operation was continued until the aqueous layer became 450 μL or less. When the aqueous layer became 450 μL or less, 50 μL of P3 Buffer (Qiagen N.V.) was added, 900 μL of ethanol was further added and mixed, and then, centrifugation was performed at 20,000 × g for 10 minutes. The supernatant was removed, and the resulting pellet DNA was rinsed with 900 μL of 70% ethanol and then dissolved in 22 μL of TE.

(Example 1: Screening using DNA barcodes from collectively transfected All-in-One library)

• Design of seed plasmids for CombiOGAB method

The All-in-One genome serving as a seed plasmid was designed to have a structure in which the rAAV region (reporter gene surrounded by two ITR sequences), the Helper region (VA, E4, and E2A), and the Rep/Cap region, which are usually separately loaded on three plasmid vectors, were linked together in this order (FIG. 1). Five types of seed plasmids #1 to #5 linking sequences from different serotypes of AAV (ITR and Rep genes) and different serotypes of adenovirus (Hepler region) were designed as shown in Table 1.

[Table 1]

| Seed plasmid No. | 5TTR | 3TTR | Helper | Rep |
|---|---|---|---|---|
| #1 | Improved AAV-2 | Improved AAV-2 | Ad-2C | Improved AAV-2 |
| #2 | AAV-1 | AAV-1 | Ad-12A | AAV-1 |
| #3 | AAV-2 | AAV-2 | Ad-148 | AAV-2 |
| #4 | AAV-3 | AAV-3 | Ad-5C | AAV-3 |
| #5 | AAV-4 | AAV-4 | Ad-8D | AAV-8 |

Here, for the reporter gene (EGFP) and the Cap gene (derived from Ad2), sequences common between seed plasmids were used. That is, the target of recombination by the combinatorial library by the CombiOGAB method is four portions of 5' ITR, 3' ITR, Helper, and Rep. A restriction enzyme SfiI recognition site (5'-GGCCnNNN/nGGCC-3'; n and N are any of A, T, G, and C; capital letters are 3' end protrudings) was located at the boundary of each region. Each 3' end protruding of the three bases generated when each SfiI recognition site is cleaved is the only sequence in the plasmid, as illustrated in FIG. 1. In the combinatorial library production by the CombiOGAB method, the order and direction of the adjacent DNA fragments are specified by the complementarity of the protruding portions. Thus, the seed plasmids were designed so that, for all types of seed plasmids, unit DNAs located at the same location had the same protruding sequence as illustrated in FIG. 2. Although some genes to be used may have SfiI recognition sequences naturally, the recognition sequences on the gene encoding the protein were replaced with synonymous codons and the recognition sequences on the non-coding region such as within the ITR sequence were changed in sequence to eliminate the SfiI recognition site.

In order to confirm what combinations of unit DNAs the rAAV vector plasmids produced from the All-in-One library have, a site where a DNA barcode with random sequences of 10 bases in the rAAV region can be introduced were designed. The specific sequences of the seed plasmids are set forth in SEQ ID NOs: 1 to 5.

• Preparation of integrated nucleic acids for OGAB method

In order to construct the designed seed plasmids by the OGAB method, 18 or 19 DNA fragments were designed, respectively (SEQ ID NOs: 6 to 98). Each material DNA was basically chemically synthesized and then prepared by the MAP method (JP 2019-198236 A) except that the sequence of the portion of the seed plasmid #1 was obtained by PCR.

• Cloning of integrated fragment for OGAB into vector

The resulting DNA fragments were purified using MinElute PCR Purification Kit (Qiagen N.V.), and finally eluted from the column with 15 μL of TE buffer (Nacalai Tesque INC.). To 1.4 μL of the resulting DNA solution, 0.2 μL of 10 × Ex-Taq buffer (Takara Bio Inc.), 0.2 μL of 2 mM dNTP (Takara Bio Inc.), and 0.2 μL of 10 × A-attachment mix (Toyobo Co., Ltd.) were added, and the mixture was reacted at 60°C for 1 hour. Thereafter, 0.5 μL of fragments of 4.3 kb obtained by cleaving with 6 ng/μL of pBR-delTypeIIS-3-AarI (SEQ ID NO: 99) AarI, and 2.5 μL of DNA Ligation Kit <Mighty Mix> were added thereto, a ligation reaction was performed at 16°C for 3 hours, and transformation was performed using Escherichia coli JM109 competent cells (Takara Bio Inc.). After overnight culture, the base sequences of the colonies that appeared on an LB plate containing carbenicillin were confirmed, and a clone having a correct sequence for each fragment was obtained.

• Preparation of equimolar mixture of DNA fragments for OGAB method

Escherichia coli with these clones was cultured in 2 mL of LB medium, and plasmids were purified with automated system QIAcube by QIAcube by QIAprep Spin Miniprep Kit (Qiagen N.V.) and finally eluted into 30 μL of TE buffer. TE buffer was added so that a solution containing 3 μg of plasmid DNAs among them was 50 μL. Further, 6 μL of 10 X Plasmid

safe buffer (epicentre), 2.4 μL of 25 mM ATP, and 2 μL of Plasmid-Safe ATP-Dependent DNase (Lucigen) were added thereto, and the mixture was reacted at 37°C for 1 hour and then deactivated at 70°C for 30 minutes to decompose DNA having a structure other than a circular structure. Thereafter, the reaction solution was purified using MinElute PCR Purification Kit (Qiagen N.V.) and finally eluted from the column with 15 μL of TE buffer (Nacalai Tesque INC.). The resulting DNA solution was measured using a microspectrophotometer (Nano drop One, Thermo Fisher Scientific Inc.), and diluted by adding TE buffer so as to be 100 ng/μL. Thereafter, the concentration was measured again, and the volume required to fractionate exactly 500 ng of DNA was calculated to two decimal places in μL, and fractionated with a micropipette to adjust each plasmid to be equimolar. The fractionated DNA solutions were pooled in two or three 1.5 mL centrifuge tubes depending on the type of restriction enzyme to be subsequently used (Table A).

Thereafter, 1.94 volumes of sterilized water, 0.33 volumes of 10 × Buffer AarI (Thermo Fisher Scientific Inc.), 0.06 volumes of 50 × oligonucleotide (0.025 mM), and 0.17 volumes of AarI (Thermo Fisher Scientific Inc.) were added to the tube for cleavage with AarI when the total volume of the plasmid solution was 1 volume, and the mixture was reacted at 37°C for 2 hours. To the tube for cleavage with BsaI, 2 volumes of sterile water, 0.33 volumes of 10 X CutSmart buffer (NEB), and 0.17 volumes of BsaI-HFv2 were added when the total volume of the plasmid solution was 1 volume, and the mixture was reacted at 37°C for 2 hours. An equal amount of TE saturated phenol to the restriction enzyme reaction solution was added to each tube, and the mixture was well mixed to deactivate the restriction enzyme. Then, the mixture of the phenol and the DNA solution in the emulsified state was integrated into one 2 mL centrifuge tube, and centrifuged at 20,000 × g for 10 minutes to separate the phenol phase and the aqueous phase. The upper layer was transferred to a new tube, into which an equal amount of 1-butanol was added, and the mixture was well stirred and centrifuged at 20,000 × g for 1 minute. Thereafter, the upper layer was removed by suction with a pipette, an equal amount of 1-butanol was added again, centrifugation was performed, and the upper layer was discarded until the volume of the lower layer became 450 μL or less. Thereafter, 50 μL of P3 buffer and 900 μL of ethanol were added, and the mixture was centrifuged at 20,000 × g for 10 minutes. The supernatant was discarded so as not to lose the precipitate and then rinsed with 900 μL of 70% ethanol, and the supernatant was sucked with a pipette and discarded. Thereafter, the mixture was centrifuged again, the remaining supernatant was collected at the bottom, and the liquid was completely removed with a pipette. Immediately, 50 μL of TE was added, and the precipitate was completely dissolved by tapping for 5 minutes. Thereafter, an equimolar mixture of 18 or 19 types of fragments of about 600 to 900 bp cut out from pBR-delTypeIIS-3-AarI was size-fractionated by electrophoresis with a low melting point agarose gel, and a mixture of 18 or 19 types of fragments was purified according to the method for cutting and purifying DNA fragments from an electrophoresis gel described in (WO 2020/203496 A).

• Preparation of fragments for OGAB integration

Plasmid pGETS118-AarI (Tsuge et al., Scientific Reports, 5, 10655) was cleaved with restriction enzyme AarI and size-fractionated by low melting point agarose gel electrophoresis, and large fragments of 15 kb were cut out from gel and purified to obtain fragments for OGAB integration.

• Plasmid formation by OGAB method

TE buffer was added to a mixed solution (10 fmol) of the resulting 18 or 19 DNA fragments and the fragments for OGAB integration (10 fmol) so that the total amount was 4 μL, and 5 μL of 2 × ligation buffer (15% polyethylene glycol 6000, 500 mM NaCl, 132 mM Tris·HCl (pH 7.6), 13.2 mM MgCl2, 20 mM DTT, 0.2 mM ATP) was added thereto. Then, the mixture was well mixed, 1 μL of T4 DNA Ligase (Takara Bio Inc.) was added, and the mixture was incubated at 37°C for 5 hours, thereby preparing transforming DNA linked in tandem repeats. 100 μL of competent cells of Bacillus subtilis were added thereto, and the mixture was subjected to rotary culture at 37°C for 30 minutes using a duck rotor. Thereafter, 300 μL of LB medium was added, and rotary culture was performed at 37°C for 1 hour with a duck rotor. Thereafter, the culture solution was spread on an LB plate containing 10 μg/mL of tetracycline (Sigma-Aldrich), and culture was performed overnight at 37°C to obtain candidate transformants of the seed plasmids.

• Confirmation of plasmid structure of transformant

12 colonies were randomly selected from the resulting candidate transformants and cultured overnight in 2 mL of LB medium containing 10 μg/mL of tetracycline, and in order to amplify the number of copies of the plasmids, IPTG was added so that a final concentration was 1 mM, and culture was further performed at 37°C for 3 hours. A small amount of plasmids was extracted from the resulting bacterial cells by the following procedure. 900 μL of the bacterial solution was placed in a 1.5 mL centrifuge tube and centrifuged at 6,800 × g for 3 minutes, and the supernatant was removed with a micropipette. The resulting cell pellets were well suspended, 100 μL of P1 buffer containing 10 mg/mL of egg white lysozyme (Wako Pure Chemical Industries, Ltd.) was added, and then the cell pellets were incubated at 37°C for 5 minutes. Then, 200 μL of P2 buffer was added and mixed by inversion 4 times. Thereafter, 150 μL of P3 was added, and mixing was

performed by inversion 4 times. The resultant was centrifuged at 20,000 × g for 10 minutes to separate into a white precipitate and a supernatant. The supernatant was transferred to a new 1.5 mL centrifuge tube, into which 450 μL of TE saturated phenol (Nacalai Tesque INC.) was added, and mixed well. Then, the mixture was centrifuged at 20,000 × g for 10 minutes to separate into a phenol phase and an aqueous phase, 320 μL of the aqueous phase was transferred to a new tube, into which 900 μL of ethanol was added, and the mixture was centrifuged at 20,000 × g for 10 minutes. The supernatant was removed with a micropipette, 900 μL of 70% ethanol was added, and the entire tube was rinsed. Thereafter, 70% ethanol was removed with a micropipette, and the resulting precipitate was dissolved in 27 μL of TE buffer. 8 μL of the resulting plasmid solution was taken, 1 μL of 10 × 3.1 NEB buffer (NEB) and 1 μL of a restriction enzyme appropriate for confirming the structure of each seed plasmid were added thereto, the mixture was reacted at 37°C for 1 hour, and the cleavage pattern was confirmed by agarose gel electrophoresis, thereby obtaining one clone matching the assumed restriction enzyme cleavage pattern for each seed plasmid. These plasmids were fragmented with Illumina prep (Illumina, Inc.) and the MiSeq (Illumina, Inc.) entire base sequence was determined to obtain clones in which each seed plasmid was correctly integrated.

• Seed plasmid mass preparation method

High purity plasmid DNAs were prepared by cesium chloride-ethidium bromide density gradient ultracentrifugation. 200 mL of a mixture obtained by adding tetracycline to LB medium so as to be 10 μg/mL was prepared, 100 mL of the mixture was added to a 500 mL Erlenmeyer flask to inoculate Bacillus subtilis plasmid-retaining strain, and culture was performed at 37°C for 16 hours. Thereafter, IPTG was added so as to be 1 mM, culture was further performed for 1 day to increase the number of copies of the plasmids, and then the plasmid was purified by ultracentrifugation.

• Preparation of DNA barcode fragment

At the time of synthesis, 10 μL of 10 × ExTaq Buffer (Takara Bio Inc.), 10 μL of a 2.5 mM dNTP solution (Takara Bio Inc.), and 2 μL of ExTaq polymerase (Takara Bio Inc.) were added to 88 μL of an aqueous solution containing 1 nmol of chemically synthesized DNA (SEQ ID NO: 100) containing a random sequence synthesized by continuously introducing 10 bases of N (base of any of A, T, G, and C) and 1 nmol of primer DNA (SEQ ID NO: 101) hybridizing with the 3' end of the DNA, and then, the mixture was denatured at 99°C for 10 minutes by a PCR apparatus and then reacted at 58°C for 1 minute and 72°C for 20 minutes. The reactant was purified by MiniElute PCR Purification Kit to obtain DNA fragments dissolved in 15 μL of TE. 9 μL of water, 3 μL of 10 × CutSmart Buffer, and 3 μL of DraIII-HF were added thereto, and reacted at 37°C for 30 minutes, and then, the reactant was purified again by MiniElute PCR Purification Kit to obtain DNA barcode fragments dissolved in 15 μL of TE.

• CombiOGAB library

1 μg of five types of seed plasmids obtained by mass preparation were mixed, and water was added so as to be 85 μL, 10 μL of 10 × CutSmart buffer (NEB) and 5 μL of restriction enzyme SfiI were added, and then, the mixture was reacted at 50°C for 1 hour. After completion of the reaction, 600 μL of water and 500 μL of TE saturated phenol (Nacalai Tesque INC.) were added, and then the mixture was mixed. Then, the mixture was separated into two phases by centrifugation at 20,000 × g for 5 minute, the aqueous phase was transferred to a new 1.5 mL tube, 600 μL of 1-butanol was added and mixed, centrifugation was performed at 20,000 × g for about 10 seconds, the mixture was separated into two layers, and the upper butanol layer was discarded. 600 μL of 1-butanol was newly added and mixed, the mixture was centrifuged at 20,000 × g for about 10 seconds and separated into two layers, and the upper butanol layer was discarded. This operation was continued until the aqueous layer became 450 μL or less. When the aqueous layer became 450 μL or less, 50 μL of P3 Buffer (Qiagen N.V.) was added, 900 μL of ethanol was further added and mixed, and then, centrifugation was performed at 20,000 × g for 10 minutes. The supernatant was removed, and the resulting pellet DNA was rinsed with 900 μL of 70% ethanol and then dissolved in 10 μL of TE.

All seed plasmids are cleaved by cleavage with SfiI into 7 fragments, but the smallest DNA fragments of 17 bp are washed away and disappear without precipitation when ethanol precipitation operation is performed after the restriction enzyme cleavage as described above. Instead of these fragments, the DNA barcode fragments described above were added to construct a library. 2 μg (0.1 pmol) of the purified DNA described above and the same number of moles of DNA barcode were added to adjust the total volume to 18 μL. To 9 μL of the mixture, 10 μL of 2 × ligation buffer and 1 μL of T4DNA ligase were added, and after mixing, the mixture was reacted at room temperature for 1 hour. 100 μL of a Bacillus subtilis competent cell culture solution was added to 10 μL of a ligation product with two tubes, and the mixture was swirled and cultured for 30 minutes with a duck rotor. Thereafter, 300 μL of LB medium was added thereto, and the mixture was swirled and cultured at 37°C for 1 hour. Thereafter, the whole culture solution was smeared on 10 plates containing tetracycline, and culture was performed overnight at 37°C. As a result, 88 colonies were obtained. These colonies were

inoculated into 2 mL of LB medium containing tetracycline, and a culture solution was obtained by overnight culture to obtain plasmids. Each plasmid was fragmented using Illumina prep (Illumina, Inc.), base sequence analysis was performed using Miseq (Illumina, Inc.), and identification of original seeds plasmid and association of DNA barcodes were performed (Table 2). As a result, the ITR sequence was dominated by recombination units derived from specific seed plasmids, but for Rep and Cap recombination units, recombination units derived from various seed plasmids were included.

• Production of rAAV from All-in-One library

Plasmid DNAs (plasmids derived from Bacillus subtilis) were obtained by centrifuging 88 mL of the resulting culture solution obtained by collecting 1 mL of the culture solution from each of the culture solutions derived from the 88 colonies described above, and preparing a large amount of the Bacillus subtilis plasmid described above for the obtained bacterial pellets.

2.5 x $10^6$ 293T cells were seeded in a 6 cm-dish the day before transfection. After 22 hours, the medium was replaced with 4.75 mL of DMEM medium (2% FBS, 1% penicillin/streptomycin), and culture was performed at 37°C and 5% $CO_2$ for 2 hours. A transfection solution obtained by mixing 125 μL of DMEM medium containing 9.3 μg of the plasmids and 125 μL of DMEM medium containing PEI pro in an amount of 1-fold of DNA was added thereto.

After culturing at 37°C and 5% $CO_2$ for 72 hours, 1/80 volume of 0.5M EDTA (pH 8.0) was added, and the mixture was allowed to stand at room temperature for 10 minutes. The detached cell suspension was recovered and centrifuged at 180 × g for 5 minutes to completely remove the supernatant. The cell pellets were suspended in 400 μL of Lysis buffer (2 mM $MgCl_2$, 150 mM NaCl, 50 mM Tris, 0.1% Triton (registered trademark) X-100; pH 8.5), benzonase (75 units/mL) was further added thereto, and the mixture was reacted at 37°C for 30 minutes. Thereafter, 2/100 volume of a 1.9 M $MgSO_4$ solution was added, and the mixture was allowed to stand at room temperature for 10 minutes. The supernatant was recovered by centrifugation at 12,000 x g and 4°C for 10 minutes, and Pluronic (registered trademark) F-68 was added at a final concentration of 0.001%.

• Extraction of rAAV genomes

600 μL of water was added to 100 μL of the supernatant, 500 μL of TE saturated phenol was added thereto, and the mixture was well mixed. Then, the mixture was separated into two phases by centrifugation at 20,000 × g for 5 minute, the aqueous phase was transferred to a new 1.5 mL tube, 600 μL of 1-butanol was added and mixed, centrifugation was performed at 20,000 × g for about 10 seconds, the mixture was separated into two layers, and the upper butanol layer was discarded. 600 μL of 1-butanol was newly added and mixed, the mixture was centrifuged at 20,000 × g for about 10 seconds and separated into two layers, and the upper butanol layer was discarded. This operation was continued until the aqueous layer became 450 μL or less. When the aqueous layer became 450 μL or less, 50 μL of P3 Buffer (Qiagen N.V.) was added, 900 μL of ethanol was further added and mixed, and then, centrifugation was performed at 20,000 × g for 10 minutes. The supernatant was removed, and the resulting pellet DNA was rinsed with 900 μL of 70% ethanol and then dissolved in 10 μL of TE. Water was added to 1 μL of a 100-fold diluted solution of the DNA, 5 pmol of each of primers set forth in SEQ ID NO. 102 and SEQ ID NO. 103, 5 μL of 10 × ExTaq Buffer (Takara Bio Inc.), 5 μL of 2.5 mM dNTP (Takara Bio Inc.), and 1 μL of Ex-Taq polymerase (Takara Bio Inc.) so that the total volume was 50 μL, and after 96°C for 2 minutes, cycles of 98°C for 20 seconds, 58°C for 30 seconds, and 72°C for 30 seconds were repeated 10 times to amplify the barcode region. For the obtained DNA, the molecular abundance ratio of each DNA barcode was examined by Miseq (Illumina, Inc.). In addition, for the ALL-in-One plasmids used for transfection, the molecular abundance ratio of each DNA barcode was determined in the same manner, and the transfection ratio for each clone was determined. The results are shown in Table 2. It was determined that the production amount per unit DNA of the viral vector was different depending on the difference in the sequence of All-in-One.

• Production of rAAV by All-in-One library via Escherichia coli

The All-in-One plasmids prepared with the Bacillus subtilis were introduced into Escherichia coli, and in order to confirm whether the result of the obtained library plasmid was consistent with All-in-One derived from Bacillus subtilis, the following experiment was performed. 0.1 μL of the All-in-One plasmids prepared with the above Bacillus subtilis were introduced into electrocompetent cells (Takara Bio Inc.) of JM109 by electroporation (Bio-Rad Laboratories, Inc.), and selected on an LB plate containing 12.5 μg/mL of chloramphenicol to obtain tens of thousands of colonies. The pellets mixed with the colonies were recovered using a spreader, and plasmid DNAs (plasmids derived from Escherichia coli) were recovered by the above-described ultracentrifugation method for mass preparation.

3 x $10^6$ 293T cells were seeded in a 10 cm-dish the day before transfection. After 22 hours, the medium was replaced with 9.5 mL of DMEM medium (2% FBS, 1% penicillin/streptomycin), and culture was performed at 37°C and 5%

$CO_2$ for 2 hours. A transfection solution obtained by mixing 250 μL of DMEM medium containing 12.5 μg of the plasmids and 250 μL of DMEM medium containing PEI pro in an amount of 1-fold of DNA was added thereto.

After culturing at 37°C and 5% $CO_2$ for 72 hours, 1/80 volume of 0.5M EDTA (pH 8.0) was added, and the mixture was allowed to stand at room temperature for 10 minutes. The detached cell suspension was recovered and centrifuged at 180 × g for 5 minutes to completely remove the supernatant. The cell pellets were suspended in 400 μL of Lysis buffer (2 mM $MgCl_2$, 150 mM NaCl, 50 mM Tris, 0.1% Triton (registered trademark) X-100; pH 8.5), benzonase (75 units/mL) was further added thereto, and the mixture was reacted at 37°C for 30 minutes. Thereafter, 2/100 volume of a 1.9 M $MgSO_4$ solution was added, and the mixture was allowed to stand at room temperature for 10 minutes. The supernatant was recovered by centrifugation at 12,000 x g and 4°C for 10 minutes, and Pluronic (registered trademark) F-68 was added at a final concentration of 0.001%.

The viral genome DNA was extracted in the same manner as in the case of the plasmids derived from Bacillus subtilis, and the abundance ratio was examined by MiSeq. The results are shown in Table 2. The obtained results were almost similar to those of the plasmids derived from Bacillus subtilis.

[Table 2-1]

| | Original seed plasmid No. | | | | Out/In(relative value) | |
|---|---|---|---|---|---|---|
| Clone No. | 5ITR | 31TR | Helper | Rep | Bacillus subtilis -derived | Escherichia coli -derived |
| 1 | #1 | #5 | #5 | #3 | | |
| 2 | #1 | #5 | #5 | #3 | 1.3 | 2.6 |
| 3 | #1 | #5 | #3 | #5 | 1 | 3.3 |
| 4 | | | | | | |
| 5 | #1 | #5 | | | 1 | 1 |
| 6 | #1 | #5 | #3 | #3 | 6 | 48 |
| 7 | #1 | #5 | #5 | #5 | 4 | 8 |
| 8 | #1 | #1 | #5 | #3 | 1.6 | 0.6 |
| 9 | #1 | #5 | #5 | #5 | 1.4 | 0.2 |
| 10 | | | | #5 | | |
| 11 | #1 | #5 | #3 | #5 | 1.7 | 1 |
| 12 | #1 | #5 | #5 | #5 | 1 | 0.2 |
| 13 | #1 | #5 | #4 | #4 | 1.9 | 0.8 |
| 14 | | | | | | |
| 15 | | #5 | #5 | | | |
| 16 | #1 | #5 | #5 | #3 | 1 | 3.8 |
| 17 | #3 | | #5 | #1 | 18 | 24.8 |
| 18 | #1 | #5 | #1 | | 5.3 | 20.5 |
| 19 | #1 | #5 | #5 | #5 | 1 | 1 |
| 20 | | | | #5 | | |
| 21 | | | | | | |
| 22 | #1 | #5 | #3 | #1 | 0.4 | 0.2 |
| 23 | | | #5 | #3 | | |
| 24 | #1 | #5 | #1 | #4 | | |
| 25 | | | | #5 | | |
| 26 | #1 | #5 | #3 | #5 | | |
| 27 | | | | #5 | | |
| 28 | #1 | #1 | #5 | #5 | 0.1 | 0 |

(continued)

| | Original seed plasmid No. | | | | Out/In(relative value) | |
|---|---|---|---|---|---|---|
| Clone No. | 5ITR | 31TR | Helper | Rep | Bacillus subtilis -derived | Escherichia coli -derived |
| 29 | | | | #4 | | |
| 30 | #1 | #5 | #5 | #3 | | |
| 31 | #1 | #5 | #5 | #5 | 1.5 | 2.9 |
| 32 | #1 | #5 | #5 | #5 | 1,6 | 0.3 |
| 33 | #1 | #5 | #5 | #5 | | |
| 34 | | | | | 1 | 0 |
| 35 | #1 | #5 | #5 | #5 | 2.3 | 6 |
| 36 | | | | #3 | | |
| 37 | | | | #5 | | |
| 38 | #1 | #5 | #5 | | 1 | 0.2 |
| 39 | #1 | #5 | #5 | #5 | 1.7 | 11.3 |
| 40 | #1 | #1 | #5 | #1 | | |
| 41 | #1 | #5 | #3 | #4 | 0.9 | 1 |
| 42 | #1 | #5 | #3 | #5 | | |

[Table 2-2]

| | | | | | | |
|---|---|---|---|---|---|---|
| 43 | #5 | #3 | #5 | | | |
| 44 | #1 | #5 | #5 | #5 | 1 | 1 |
| 45 | #1 | #5 | #5 | #4 | 2.8 | 3.8 |
| 46 | | | | #4 | | |
| 47 | #1 | #5 | #5 | #5 | 1.5 | 2 |
| 48 | #1 | #5 | #5 | #4 | 3.1 | 1.3 |
| 49 | | | | #5 | | |
| 50 | #1 | #1 | #4 | #3 | 2.9 | 11 |
| 51 | #3 | #5 | #5 | #3 | | |
| 52 | | | | #1 | | |
| 53 | #4 | #5 | #5 | #5 | | |
| 54 | #1 | #5 | #5 | #3 | | |
| 55 | | | | #3 | | |
| 56 | | | | | | |
| 57 | | | | | | |
| 58 | #1 | #1 | #5 | #1 | 2.1 | 1.9 |
| 59 | #1 | #5 | #5 | #1 | 1.7 | 3.4 |
| 60 | #1 | #5 | #5 | #5 | | |
| 61 | | | | | | |
| 62 | | | | #5 | | |
| 63 | #1 | | #3 | #5 | 1 | 0.8 |
| 64 | | | | #4 | | |

(continued)

| | | | | | | |
|---|---|---|---|---|---|---|
| 65 | | | | #5 | | |
| 66 | #1 | | | #4 | | |
| 67 | #1 | #5 | #1 | #5 | 2.2 | 2.3 |
| 68 | #1 | #5 | #5 | #4 | 2.5 | **4** |
| 69 | #1 | #1 | #5 | #3 | 1 | 0.1 |
| 70 | #1 | #5 | #5 | #5 | 2.1 | 0.9 |
| 71 | #1 | #5 | #5 | #5 | 2.1 | 0 |
| 72 | #5 | #5 | #5 | #5 | **6.8** | 0 |
| 73 | #1 | #5 | #5 | #1 | 1 | 10.3 |
| 74 | | | | #3 | | |
| 75 | | | | #3 | | |

* The absence of the number of the original seed plasmid indicates that the corresponding seed plasmid cannot be detected.
* Out/In indicates a ratio of the number of genomic copies of the resulting viral vector and the number of copies of the plasmids used at the time of transfection in one transfection experiment, which is calculated based on the read number of the DNA barcode region by the next generation sequencer.

(Example 2: Screening of All-in-One by individual transfection)

• Design of CombiOGAB library

In order to expand the five types of libraries of Example 1, a total of eight types of seed plasmids were designed by adding new seed plasmids as shown in Table 3 and FIG. 3 for each of 5' ITR, 3' ITR, Helper, and Rep recombination units.

[Table 3]

| Seed plasmid No. | 5'ITR recombination unit | 3TTR recombination unit | Helper recombination unit | Rep recombination unit |
|---|---|---|---|---|
| #1 | Improved AAV-2 | AAV-5 | Ad-2 | Improved AAV-2 |
| #2 | AAV-1 | AAV-1 | Ad-12 | AAV-1 |
| #3 | AAV-2 | AAV-2 | Ad-14 | AAV-2 |
| #4 | AAV-3 | AAV-3 | Ad-5 | AAV-3 |
| #5 | AAV-4 | AAV-4 | Ad-8 | AAV-8 |
| #6 | AAV-5 | Improved AAV-2 | Ad-4 | AAV-5 |
| #7 | AAV-6 | AAV-6 | Ad--40 | AAV-10 |
| #8 | AAV-7 | AAV-7 | Ad-52 | AAV-7 |

Among them, as for #1, the 3' ITR of #1 in Example 1 was changed from AAV-2 to AAV-5. In this case, the DNA fragment obtained by newly synthesizing the third fragment of #1 of Example 1 is set forth in SEQ ID NO: 104. In addition, the 18 and 19 fragments prepared for the synthesis of #6 to #8 are set forth in SEQ ID NOs: 105 to 159. The restriction enzymes used to cut out the DNA fragments for the OGAB method are shown in Table A.

[Table A]

| Seed plasmid No. | #1 | #2 | #3 | #4 | #5 | #6 | #7 | #8 |
|---|---|---|---|---|---|---|---|---|
| First fragment | AarI | AarI | AarI | AarI | AarI | AarI | AarI | AarI |
| Second fragment | AarI | AarI | AarI | AarI | AarI | AarI | AarI | AarI |

(continued)

| Seed plasmid No. | #1 | #2 | #3 | #4 | #5 | #6 | #7 | #8 |
|---|---|---|---|---|---|---|---|---|
| Third fragment | AarI | AarI | AarI | AarI | AarI | AarI | AarI | AarI |
| Fourth fragment | BsmBI | AarI | BsaI | BsmBI | BsmBI | BsmBI | BsaI | BsaI |
| Fifth fragment | AarI | BsmBI | AarI | AarI | AarI | BsaI | AarI | AarI |
| Sixth fragment | AarI | BsmBI | BsaI | AarI | BsmBI | BsaI | BsaI | BsaI |
| Seventh fragment | AarI | AarI | BsaI | BsmBI | AarI | AarI | AarI | AarI |
| Eighth fragment | BsmBI | AarI | BsaI | BsmBI | BsmBI | BsmBI | BsaI | BsaI |
| N inth fragment | BsmBI | BsmBI | AarI | AarI | AarI | BsaI | AarI | AarI |
| Tenth fragment | AarI | BsmBI | BsaI | AarI | BsmB1 | AarI | BsaI | BsaI |
| Eleventh fragment | AarI | BsmBI | AarI | BsmBI | BsmBI | BsmBI | AarI | AarI |
| Twelfth fragment | AarI | BsmBI | AarI | AarI | BsmBI | AarI | BsaI | BsaI |
| Thirteenth fragment | BsmBI | BsmBI | AarI | BsmBI | BsmBI | BsmBI | BsaI | BsaI |
| Fourteenth fragment | BsmBI | Aart | AarI | BsmBI | BsmBI | AarI | AarI | AarI |
| Fifteenth fragment | BsmBI | AarI | BsaI | BsmBI | AarI | BsaI | Aart | AarI |
| Sixteenth fragment | BsmBI | AarI | BsaI | AarI | AarI | BsaI | AarI | AarI |
| Seventeenth fragment | AarI | AarI | AarI | AarI | AarI | AarI | AarI | AarI |
| Eighteenth fragment | AarI | AarI | AarI | AarI | AarI | AarI | AarI | AarI |
| Nineteenth fragment | AarI |  | AarI | AarI |  | AarI |  |  |

Plasmids were once produced by the OGAB method in the same manner as that of Example 1, and then, in order to eliminate the SfiI (GTT) recognition site present in the plasmids, each plasmid was digested with NotI. Thereafter, a short NotI fragment of about 100 bp desired to be eliminated was removed by electrophoresis, and the remaining 2 fragments were cyclized. By introducing the fragments into Escherichia coli JM109, a seed plasmid having one SfiI fragment less than that in Example 1 was constructed. The base sequences of the plasmids #1 to #8 are set forth in SEQ ID NOs: 160 to 167.

• Construction of CombiOGAB library

Escherichia coli with various plasmids was cultured overnight in LB medium containing 200 mL of chloramphenicol (Nacalai Tesque INC.) at a concentration of 12.5 μg/mL, and a seed plasmid mass preparation method was performed by ultracentrifugation. 1 μg of each of eight types of seed plasmids prepared by mass preparation was cleaved by SfiI in the same manner as that of Example 1, and the fragments were purified. Other than that, ligation and transformation were performed in the same manner as that of Example 1. As a result, 233 transformants were obtained. All colonies were cultured in 2 mL of LB medium containing 10 μg/mL of tetracycline and 1 mM IPTG, and then centrifuged at 6,800 × g for 3 minutes to recover cell pellets. The cell pellets were reacted with 200 μL of P1 Buffer containing lysozyme at room temperature for 5 minutes, and then, the pellets were recovered again under the same centrifugation conditions. Then, the cell pellets were purified by QIAquick Miniprep kit (Qiagen N.V.) using a QIAcube (Qiagen N.V.) automation device. The resulting plasmids were fragmented with Illumina prep (Illumina, Inc.) and analyzed with Miseq (Illumina, Inc.) to identify a seed plasmid from which each plasmid was derived. The results of 48 of these clones are shown in Table 4. As in Example 1, the construction of the plasmid library containing various combinations of Helper recombination unit and Rep recombination unit was confirmed.

• Production of rAAV

5 × $10^4$ 293T cells were seeded in a 96-well plate the day before transfection. After 22 hours, the medium was replaced with 90 μL of DMEM medium (2% FBS, 1% penicillin/streptomycin), and culture was performed at 37°C and 5% $CO_2$ for 2 hours. Each transfection solution obtained by mixing a solution containing 185 ng of each plasmid and DMEM medium containing PEI pro in an amount of 1-fold of DNA was added thereto.

After culturing at 37°C and 5% $CO_2$ for 72 hours, a solution obtained by adding Benzonase (200 units/mL) to 10 × Lysis buffer (20 mM $MgCl_2$, 1.5M NaCl, 500 mM Tris, 1% Triton (registered trademark) X-100; pH 7.5) was added at 10

v/v%, and the mixture was reacted at 37°C for 60 minutes. Thereafter, 2/100 volume of a 1.9 M $MgSO_4$ solution was added, and the mixture was allowed to stand at room temperature for 10 minutes. The supernatant was recovered by centrifugation at 3,750 rpm and room temperature for 10 minutes.

In order to quantify the number of genomic copies of rAAV, DNaseI treatment was performed for 30 minutes, and a sample reacted at 95°C for 10 minutes was diluted 1,000 times and subjected to qPCR. The target was ITR2, and the primer sequences used were SEQ ID NO: 168 and SEQ ID NO: 169. As the reaction conditions, after 10 minutes at 95°C, 40 cycles of 95°C/15 seconds, 55°C/5 seconds, and 72°C/30 seconds were repeated. The results are shown in Table 4. In the individual screening system, All-in-One plasmids having different viral production amounts were screened.

[Table 4-1]

| Original seed plasmid No. | | | | | |
|---|---|---|---|---|---|
| Clone No. | 5TTR | 3TTR | Helper | Rep | Amount of viral vector (VG/ml) |
| 1 | #1 | #6 | #5 | #1 | 2.49E+08 |
| 2 | | | #2 | #8 | 1.70E+08 |
| 3 | #1 | #6 | #6 | #1 | 2.51E+09 |
| 4 | #1 | #6 | #6 | #7 | 1.68E+09 |
| 5 | #1 | #6 | #3 | #6 | 1.69E+09 |
| 6 | #1 | #6 | #7 | #1 | 1.07E+08 |
| 1 | | | #1 | #4 | 6.71E+08 |
| 8 | #1 | #6 | #1 | #6 | 1.41E+09 |
| 9 | #1 | #6 | #6 | #1 | 3.78E+08 |
| 10 | #1 | #6 | #7 | #6 | 1.59E+08 |
| 11 | #1 | #6 | #6 | #1 | 8.94E+07 |
| 12 | #1 | #6 | #1 | #1 | 3.14E+08 |
| 13 | #1 | #6 | #5 | #5 | 8.35E+08 |
| 14 | #1 | #6 | #6 | #8 | 1.20E+09 |
| 15 | #1 | #6 | #1 | #1 | 8.34E+08 |
| 16 | #1 | #6 | | #1 | 2.23E+09 |
| 17 | #1 | #6 | #4 | #4 | 1.93E+09 |
| 18 | #1 | #6 | #1 | #6 | 2.11E+09 |
| 19 | #1 | #6 | #5 | #5 | 1.03E+09 |
| 20 | #1 | #6 | #6 | #2 | 1.60E+10 |
| 21 | #1 | #6 | #5 | #7 | 5.58E+08 |
| 22 | #1 | #6 | #2 | #6 | 1.45E+09 |
| 23 | #1 | #6 | #7 | #4 | 2.02E+09 |
| 24 | #1 | #6 | #2 | #1 | 5.42E+09 |
| 25 | #1 | #5 | #7 | #4 | 1.23E+09 |
| 26 | #1 | #6 | #6 | #2 | 1.37E+09 |
| 27 | #1 | #5 | #5 | #6 | 1.42E+09 |
| 28 | #1 | #6 | #4 | #6 | 1.50E+09 |
| 29 | #1 | #6 | #8 | #2 | 2.17E+09 |
| 30 | #1 | #6 | #1 | #5 | 1.81E+09 |
| 31 | #1 | #6 | #2 | #6 | 6.59E+08 |
| 32 | #1 | #6 | #6 | #1 | 1.59E+09 |

(continued)

| Original seed plasmid No. | | | | | |
|---|---|---|---|---|---|
| Clone No. | 5TTR | 3TTR | Helper | Rep | Amount of viral vector (VG/ml) |
| 33 | #1 | #6 | #2 | #6 | 2.18E+09 |
| 34 | #1 | #6 | #7 | #2 | 1.06E+09 |
| 35 | #1 | #6 | #7 | #6 | 5.96E+08 |
| 36 | #1 | #6 | #8 | #6 | 2.37E+08 |

[Table 4-2]

| | | | | | |
|---|---|---|---|---|---|
| 37 | #1 | #6 | #8 | #1 | 1.23E+09 |
| 38 | #3 | #6 | #6 | #6 | 2.64E+09 |
| 39 | #1 | #5 | #6 | #1 | 1.84E+09 |
| 40 | #3 | #6 | #8 | #2 | 3.34E+09 |
| 41 | #1 | #6 | #6 | #1 | 3.94E+09 |
| 42 | | #6 | #5 | #6 | 2.04E+09 |
| 43 | | | | #2 | 1.28E+09 |
| 44 | #1 | #6 | #2 | #6 | 2.24E+09 |
| 45 | #1 | #5 | #6 | #7 | 1.68E+09 |
| 46 | #1 | #6 | #1 | #5 | 8.52E+09 |
| 47 | #1 | #6 | #7 | #2 | 9.82E+08 |
| 48 | #1 | #6 | #6 | #2 | 2.71E+09 |

• Evaluation of rAAV

1 × $10^6$ 293T cells were seeded in a 6-well plate the day before transfection. After 22 hours, the medium was replaced with 2.85 mL of DMEM medium (2% FBS, 1% penicillin/streptomycin), and culture was performed at 37°C and 5% $CO_2$ for 2 hours. Each T transfection solution obtained by mixing a solution containing 4 μL of each plasmid (Control, #20, #24, or #46) and DMEM medium containing PEI pro in an amount of 1-fold of DNA was added thereto.

After culturing at 37°C and 5% $CO_2$ for 72 hours, 1/80 volume of 0.5M EDTA (pH 8.0) was added, and the mixture was allowed to stand at room temperature for 10 minutes. The detached cell suspension was recovered and centrifuged at 180 × g for 5 minutes to completely remove the supernatant. The cell pellets were suspended in 300 μL of Lysis buffer (2 mM $MgCl_2$, 150 mM NaCl, 50 mM Tris, 0.1% Triton (registered trademark) X-100; pH 8.5), benzonase (83 units/mL) was further added thereto, and the mixture was reacted at 37°C for 30 minutes. Thereafter, 2/100 volume of a 1.9 M $MgSO_4$ solution was added, and the mixture was allowed to stand at room temperature for 10 minutes. The supernatant was recovered by centrifugation at 12,000 x g and 4°C for 10 minutes, and Pluronic (registered trademark) F-68 was added at a final concentration of 0.001%.

In order to quantify the number of genomic copies of rAAV, DNaseI treatment was performed for 30 minutes, and a sample reacted at 95°C for 10 minutes was diluted 1,000 times and subjected to qPCR. The target was the 3' ITR of AAV-2, and the primer sequences used were SEQ ID NO: 168 and SEQ ID NO: 169. As the reaction conditions, after 10 minutes at 95°C, 40 cycles of 95°C/15 seconds, 55°C/5 seconds, and 72°C/30 seconds were repeated. The results are illustrated in FIG. 4. The AAV2 production amount of each of Control (pGETS103-AAV-Helper-RC2, SEQ ID NO: 170), #20, #24, and #46 was 1.55 × $10^{10}$ GC/mL, 1.62 × $10^9$ GC/mL, 4.07 × $10^9$ GC/mL, and 2.44 × $10^{10}$ GC/mL, and the production amount of #46 was high.

Purification of rAAV was performed to evaluate the Empty/Full rate of #46. First, three types of cesium chloride solutions (1.35 g/$cm^3$, 1.27 g/$cm^3$, and 1.25 g/$cm^3$) were overlaid in 0.5 mL, 2.5 mL, and 1.0 mL, respectively, and the cell extract of rAAV was overlaid thereon. Density gradient centrifugation at 226,000 x g and 18°C for 1.5 hours was performed to recover about 2 mL of the solution around 1.27 g/$cm^3$, and Pluronic (registered trademark) F-68 was added at a final concentration of 0.001%. Next, in order to exchange the solvent, the solution was replaced with TBS (0.005% Pluronic (registered trademark) F-68, 10% Sucrose, 100 mM Tris, 200 mM NaCl, pH 7.4) using a 30 K ultrafiltration filter (Merck;

Amicon Ultra-0.5). In order to evaluate the Full rate of the rAAV sample, measurement was performed by a mass spectrometry method using Refeyn One (Life Sciences Solutions). The results are illustrated in FIG. 5. The Full rate was 61.3%.

(Example 3: Assumed seed plasmid structure)

FIGS. 6 to 10 illustrate examples of the assumed seed plasmid structure. Each functional unit illustrated in the schematic diagram may be separately included in one alternative unit nucleic acid, or a plurality of adjacent functional units may be included in one alternative unit nucleic acid. These seed plasmids are used to perform the method for producing virus-derived construct plasmids of the present disclosure to obtain plasmids having an excellent desired function (for example, the production amount of a virus-derived constituent).

* Promoters (weak, medium, and strong) are indicated by shading.
* Promoters (arrows) indicate positive and negative directions.

(FIG. 6: Examples for AAV vector plasmid of the All-in-One structure)

- FIG. 6A: Example of order of ITR → Rep → Cap → Helper
- FIG 6B: Example of order of Rep → ITR → Cap → Helper (structure in which positions of Rep and Cap are exchanged is also intended)
- FIG 6C: Example in which Helper genes are divided and arranged

(FIG. 7: Examination example of variation of Rep/Cap)

- FIG. 7A: Examination example of arrangement obtained by dividing Rep gene and variation in expression intensity of promoter
- FIG. 7B: Examination example of arrangement obtained by dividing Cep gene and variation in expression intensity of promoter

* Promoters (weak, medium, and strong) are indicated by shading.

(FIG. 8: Examination example of variation of Helper)

- FIG. 8A: Examination example of arrangement obtained by dividing Helper gene and variation in expression intensity of promoter
- FIG. 8B: Examination example of combination of AdV-derived Helper gene and Helper gene derived from another virus and variation in positive and negative directions of promoter
- FIG. 8C: Examination example of combination of AdV-derived Helper gene and nucleic acid sequence involved in biosynthesis and variation in positive and negative directions of promoter

(FIG. 9: Example for lentiviral vector plasmid)

- FIG. 9A: Examination example of variation in expression intensity of promoter of LV vector plasmid having All-in-One structure
- FIG. 9B: Examination example of variation in expression intensity of plurality of desired genes

(FIG. 10: Example for adenoviral vector plasmid)

- FIG. 10A: Examination example of variation in structural protein of AdV vector plasmid having All-in-One structure
- FIG. 10B: Examination example of variation in expression intensity of plurality of desired genes

(Example 4: Combinatorial promoter library of helper gene expression plasmids)

• Design of seed plasmids for CombiOGAB method

Five types of pHelper seed plasmids (SEQ ID NOs: 237 to 241) loading E1A and E1B regions in addition to the VA, E4 and E2A regions usually used for helper plasmids were designed as shown in Table 5. When these plasmids are used, AAV production in producer cells other than HEK293 and HEK293T cells is possible. In addition to the wild-type promoter of each of E2A, E4orf6, E1A and E1B, any of the sequences of three types of promoters (UBC promoter, mPGK promoter, and EF1A promoter) having different expression intensities was linked to each gene region (see also FIG. 11).

[Table 5]

| | 1st | 2nd | 3rd | 4th | 5th |
|---|---|---|---|---|---|
| Seed plasmid No. | VA | E2A-SV40 polyA signal | E4orf6-bGH polyA signal | E1A-hGH polyA signal | E18-HSV polyA signal |
| #9 | WT_endogenous promoter | WT_E2A promoter | WT_E4 promoter | WT_E1A promoter | WT_E1B promoter |
| #10 | WT_endogenous promoter | WT_E2A promoter | EF1A promoter | mPGK promoter | UBC promoter |
| #11 | WT_endogenous promoter | UBC promoter | WT_E4 promoter | EF1A promoter | mPGK promoter |
| #12 | WT_endogenous promoter | mPGK promoter | UBC promoter | WT_E1A promoter | EF1A promoter |
| #13 | WT_endogenous promoter | EF1A promoter | mPGK promoter | UBC promoter | WT_E1B promoter |

Here, VA, each gene, and the polyA signal sequence downstream thereof are commonly designed between seed plasmids, and the target of the combinatorial library by the CombiOGAB method is a promoter portion. A restriction enzyme SfiI recognition site (5'-GGCCnNNN/nGGCC-3'; n and N are any of A, T, G, and C; capital letters N are 3' end protruding portions) was located at the boundary of each region. Each 3' end protruding of the three bases generated when each SfiI recognition site was cleaved was designed to be the only sequence in the plasmid. In the combinatorial library production by the CombiOGAB method, the order and direction of the adjacent DNA fragments are specified by the complementarity of the protruding portions. Thus, the seed plasmids were designed so that, for all types of seed plasmids, the corresponding unit DNAs at the positions of the respective genes had the same protruding sequence as illustrated in FIG. 11. Although some genes to be used naturally contain SfiI recognition sequences, such SfiI recognition sequences were deleted by replacing them with synonymous codons in the case of protein coding sequences and changing the sequences in the case of non-coding regions such as within ITR sequences.

• Preparation of integrated nucleic acids for OGAB method

Five cloning plasmids pBR-delTypeIIS-1st, pBR-delTypeIIS-2nd, pBR-delTypeIIS-3rd, pBR-delTypeIIS-4th, and pBR-delTypeIIS-5th, which are plasmid vectors constructed so that unit DNAs containing a promoter-ORF-polyA signal can be cut out by SfiI, were constructed by cleaving pBR-delTypeIIS-3-AarI (SEQ ID NO: 99) with AarI, and then linking linker DNAs constructed by annealing synthetic DNAs of SEQ ID NOs: 171 and 172, SEQ ID NOs: 173 and 174, SEQ ID NOs: 175 and 176, SEQ ID NOs: 177 and 178, and SEQ ID NOs: 179 and 180, respectively.

Other necessary DNAs were obtained from appropriate template DNAs by a PCR method. The reaction was performed by adding 13.3 μL of water, 2 μL of 10 × Ex-Taq buffer (Takara Bio Inc.), 1.6 μL of 2.5 mM dNTP (Takara Bio Inc.), 0.1 μL of Ex-Taq, and 1 μL of each of the following primers at 3.2 μmol/μL were added to 1 μL of a template DNA solution (about 0.01 ng/μl), and subjecting the mixture to 30 cycles of reactions at 98°C for 2 minutes, at 98°C for 20 seconds, at 58°C for 30 seconds, and at 72°C for 1 minute per 1kb. Thereafter, purification was performed using MinElute PCR Purification Kit (Qiagen N.V.), 0.5 μL of 6 ng/μL of pTAKN-2 (BioDynamics Laboratory Inc.) and 2.5 μL of DNA Ligation Kit <Mighty Mix> were added thereto, a ligation reaction was performed at 16°C for 3 hours, and transformation

was performed using Escherichia coli JM109 competent cells (Takara Bio Inc.). After overnight culture, the base sequences of the colonies that appeared on an LB plate containing 25 μg/ml of kanamycin were confirmed, and a clone having a correct sequence for each fragment was obtained. For confirmation and amplification of promoter fragments for WT_E2A, WT_E4, WT_E1A, WT_E1B, UBC, mPGK, and EF1A, respectively, the sets of primers set forth in SEQ ID NOs: 181 and 182, SEQ ID NOs: 183 and 184, SEQ ID NOs: 185 and 186, SEQ ID NOs: 187 and 188, SEQ ID NOs: 189 and 190, SEQ ID NOs: 191 and 192, and SEQ ID NOs: 193 and 194, respectively, were used. For confirmation and amplification of the genes (VA) and ORFs of VA (including WT_ endogenous promoter), WT_E2A, WT_E4, WT_E1A, and WT_E1B, the sets of primers set forth in SEQ ID NOs: 195 and 196, SEQ ID NOs: 197 and 198, SEQ ID NOs: 199 and 200, SEQ ID NOs: 201 and 202, and SEQ ID NOs: 203 and 204, respectively, were used. For confirmation and amplification of SV40 polyA signal, bGH polyA signal, hGH polyA signal, and HSV polyA signal, the sets of primers set forth in SEQ ID NOs: 205 and 206, SEQ ID NOs: 207 and 208, SEQ ID NOs: 209 and 210, and SEQ ID NOs: 211 and 212, respectively, were used. Note that, a recognition site of BspQI, which is TypeIIS restriction enzyme capable of generating an arbitrary 3-base sequence, was introduced into the 5' ends of these primers, and the resulting DNA fragments were cleaved with BspQI to design protruding sequences so that the promoter-ORF-polyA signal can be accumulated in five cloning plasmid vectors from pBR-delTypeIIS-1st to -5th in this order. These amplified DNAs were ligated to pTAKN-2 to construct 16 types of plasmids having respective fragments. These plasmids and 5 cloning plasmid vectors from pBR-delTypeIIS-1st to -5th were combined and mixed according to Table 1, and 17 types of OGAB block plasmids required to construct seed plasmids were constructed by the Golden Gate method. Specifically, 12 μL of water, 2 μL of 10 × 3.1 NEB buffer (NEB), 0.5 μL of BspQI (NEB), and 1.5 μL of T4-Ligase were added to 1 μL of each DNA solution (100 ng/μl), the mixture was subjected to 30 cycles of reactions at 37°C for 1 minute and at 16°C for 1 minute, and thereafter, the mixture was left at 16°C for 3 hours or longer. JM109 competent cells (Takara Bio Inc.) were transformed using the obtained resultant, smeared on an LB plate containing 100 μg/ml of carbenicillin, and cultured overnight at 37°C to obtain transformants.

• Preparation of equimolar mixture of DNA fragments for OGAB method

Escherichia coli with the OGAB block plasmids was cultured in 2 mL of LB medium, and plasmids were purified with automated system QIAcube by QIAcube by QIAprep Spin Miniprep Kit (Qiagen N.V.) and finally eluted into 30 μL of TE buffer. The resulting plasmids were cleaved with restriction enzymes SfiI (NEB) and ApaLI (NEB), and 17 types of fragments of about 0.8 kb to 3.2 kb were recovered by electrophoresis with a low-melting point agarose gel. After the DNA concentration was measured by a fluorometer (Qubit, Thermo Fisher Scientific Inc.), the volume required for accurately fractionating equimolar DNA was calculated to two decimal places in μL, and each fragment was fractionated with a micropipette to prepare a solution in which 5 types of DNA fragments required for each seed plasmid were mixed in equimolar amounts.

• Preparation of fragments for OGAB integration

Plasmid pGETS161 was cleaved with restriction enzyme SfiI and size-fractionated by low melting point agarose gel electrophoresis, and large fragments of 5.8 kb were cut out from gel and purified to obtain fragments for OGAB integration.

• Plasmid formation by OGAB method

TE buffer was added to a mixed solution (6.8 fmol) of the resulting 5 DNA fragments and the fragments for OGAB integration (6.8 fmol) so that the total amount was 8 μL, and 10 μL of 2 × ligation buffer (15% polyethylene glycol 6000, 500 mM NaCl, 132 mM Tris·HCl (pH 7.6), 13.2 mM $MgCl_2$, 20 mM DTT, 0.2 mM ATP) was added thereto. Then, the mixture was well mixed, 2 μL of T4 DNA Ligase (Takara Bio Inc.) was added, and the mixture was incubated at 37°C for 0.5 hours, thereby preparing transforming DNA linked in tandem repeats. 100 μL of competent cells of Bacillus subtilis were added thereto, and the mixture was subjected to rotary culture at 37°C for 30 minutes using a duck rotor. Thereafter, 300 μL of LB medium was added, and rotary culture was performed at 37°C for 1 hour with a duck rotor. Thereafter, the culture solution was spread on an LB plate containing 10 μg/mL of tetracycline (Sigma-Aldrich), and culture was performed overnight at 37°C to obtain candidate transformants of the seed plasmids.

• Confirmation of plasmid structure of transformant

Six colonies were randomly selected from the resulting candidate transformants, cultured in 2 mL of LB medium containing 10 μg/mL of tetracycline for 5 hours, and a small amount of plasmids was extracted from the resulting bacterial cells by the following procedure. 2 mL of the bacterial solution was placed in a 1.5 mL centrifuge tube and centrifuged at 6,800 × g for 3 minutes, and the supernatant was removed with a micropipette. The resulting cell pellets were well

suspended, 100 μL of P1 buffer containing 10 mg/mL of egg white lysozyme (Wako Pure Chemical Industries, Ltd.) was added, and then the cell pellets were incubated at 37°C for 5 minutes. Then, 200 μL of P2 buffer was added and mixed by inversion 4 times. Thereafter, 150 μL of P3 was added, and mixing was performed by inversion 4 times. The resultant was centrifuged at 20,000 × g for 5 minutes to separate into a white precipitate and a supernatant. The supernatant was transferred to a new 1.5 mL centrifuge tube, into which 450 μL of TE saturated phenol (Nacalai Tesque INC.) was added, and mixed well. Then, the mixture was centrifuged at 20,000 × g for 10 minutes to separate into a phenol phase and an aqueous phase, 320 μL of the aqueous phase was transferred to a new tube, into which 900 μL of ethanol was added, and the mixture was centrifuged at 20,000 × g for 5 minutes. The supernatant was removed with a micropipette, 900 μL of 70% ethanol was added, and the entire tube was rinsed. Thereafter, 70% ethanol was removed with a micropipette, and the resulting precipitate was dissolved in 25 μL of TE buffer. 8 μL of the resulting plasmid solution was taken, 1 μL of 10 × CutSmart buffer (NEB) and 1 μL of a restriction enzyme appropriate for confirming the structure of each seed plasmid were added thereto, the mixture was reacted at 37°C for 1 hour, and the cleavage pattern was confirmed by agarose gel electrophoresis, thereby obtaining one clone matching the assumed restriction enzyme cleavage pattern for each seed plasmid.

• Seed plasmid mass preparation method

High purity plasmid DNAs were prepared by cesium chloride-ethidium bromide density gradient ultracentrifugation. 200 mL of a mixture obtained by adding tetracycline to LB medium so as to be 10 μg/mL was prepared, 100 mL of the mixture was added to a 500 mL Erlenmeyer flask to inoculate Bacillus subtilis plasmid-retaining strain, culture was performed at 37°C for 16 hours, and then plasmids were purified by ultracentrifugation. These plasmids were fragmented with Illumina prep (Illumina, Inc.) and the MiSeq (Illumina, Inc.) entire base sequence was determined to obtain clones in which each seed plasmid was correctly integrated.

• CombiOGAB library

To 2 μg of four types of seed plasmids obtained by mass preparation, water was added so as to be 85 μL, 10 μL of 10 × CutSmart buffer (NEB) and 5 μL of restriction enzyme SfiI were added, and then, the mixture was reacted at 50°C for 1 hour. After completion of the reaction, 50 μL of water and 150 μL of TE saturated phenol (Nacalai Tesque INC.) were added, and then the mixture was mixed. Then, the mixture was separated into two phases by centrifugation at 20,000 × g for 5 minute, the aqueous phase was transferred to a new 1.5 mL tube, 500 μL of 1-butanol was added and mixed, centrifugation was performed at 20,000 × g for about 10 seconds, the mixture was separated into two layers, and the upper butanol layer was discarded. 500 μL of 1-butanol was newly added and mixed, the mixture was centrifuged at 20,000 × g for about 10 seconds and separated into two layers, and the upper butanol layer was discarded. This operation was continued until the aqueous layer became 450 μL or less. When the aqueous layer became 450 μL or less, 50 μL of P3 Buffer (Qiagen N.V.) was added, 900 μL of ethanol was further added and mixed, and then, centrifugation was performed at 20,000 × g for 10 minutes. The supernatant was removed, and the resulting pellet DNA was rinsed with 900 μL of 70% ethanol and then dissolved in 20 μL of TE.

About 1 μg of the purified DNA described above was adjusted to 12 μL. 15 μL of 2 × ligation buffer and 3 μL of T4DNA ligase were added thereto, and after mixing, the mixture was reacted at room temperature for 4 hours. 100 μL of a Bacillus subtilis competent cell culture solution was added to 10 μL of a ligation product with three tubes, and the mixture was swirled and cultured for 30 minutes with a duck rotor. Thereafter, 300 μL of LB medium was added thereto, and the mixture was swirled and cultured at 37°C for 1 hour. Thereafter, the whole culture solution was smeared on 12 plates containing tetracycline, and culture was performed overnight at 37°C. As a result, about 30,000 colonies were obtained.

• Production of rAAV from pHelper promoter library

96 of these colonies were randomly selected, inoculated into 2 mL of LB medium containing tetracycline, and cultured overnight. A small amount of plasmids was extracted from the resulting culture solution by the following procedure. 2 mL of the bacterial solution was placed in a 1.5 mL centrifuge tube and centrifuged at 6,800 × g for 3 minutes, and the supernatant was removed with a micropipette. The resulting cell pellets were well suspended, 100 μL of P1 buffer containing 10 mg/mL of egg white lysozyme (Wako Pure Chemical Industries, Ltd.) was added, and then the cell pellets were incubated at 37°C for 5 minutes. Centrifugation was performed at 6,800 × g for 3 minutes, the supernatant was removed with a micropipette, and the plasmids were purified with automated system QIAcube by QIAprep Spin Miniprep Kit (Qiagen N.V.) and finally eluted into 30 μL of TE buffer.

A total of 97 types of plasmid DNAs (plasmids derived from Bacillus subtilis) including the plasmids derived from the 96 colonies described above and the seed plasmid #9 were obtained. These plasmids were introduced into HeLa cells to evaluate viral particle production.

$0.17 \times 10^6$ HeLa cells were seeded in a 24-well plate the day before transfection. After 22 hours, the medium was replaced with 0.38 mL of DMEM medium (2% FBS, 1% penicillin/streptomycin), and culture was performed at 37°C and 5% $CO_2$ for 2 hours. 0.10 μg of pAAV-GFP plasmid, 0.13 μg of pR2C1 plasmid, 0.33 μg of pHelper library plasmid, and 20 μL of a transfection solution obtained by mixing each of PEI pro and DMEM medium in an amount of 1-fold were added thereto. After culturing at 37°C and 5% $CO_2$ for 72 hours, the supernatant was recovered and the number of genomic copies of rAAV was quantified.

• Evaluation of rAAV

A sample prepared by subjecting the supernatant to DNaseI treatment for 30 minutes and reacting the treated supernatant at 95°C for 10 minutes was diluted 1,000 times and subjected to qPCR. Here, primer sequences of 5'-CATCAATGGGCGTGGATAGC-3' (SEQ ID NO: 213) and 5'-GGAGTTGTTACGACATTTTGGAAA-3' (SEQ ID NO: 314) targeting the CMV promoter were used. As the reaction conditions, after 10 minutes at 95°C, 40 cycles of 95°C/15 seconds, 55°C/5 seconds, and 72°C/30 seconds were repeated.

The results are illustrated in FIG. 12. Clones with improved Titer were identified compared to the seed plasmid #9 composed of the WT promoter.

• Identification of selected rAAV promoters

The clone (pHelper clone #60) having the largest viral genome amount was selected, and the base sequence of the plasmid used for the viral vector production was determined as follows to identify the combination of promoters. Plasmid DNAs were fragmented using Illumina prep (Illumina, Inc.), and base sequence analysis was performed using Miseq (Illumina, Inc.).

(Example 5: Promoter library of pDual (helper/Rep/Cap) gene expression plasmids)

• Design of seed plasmids for CombiOGAB method

In the same manner as that of Example 4, five types of pDual seed plasmids (SEQ ID NOs: 242 to 246) were designed as shown in Table 6, each pDual seed plasmid loading the VA, E4, and E2A regions, the serotype 2 Rep gene, and the serotype 1 Cap, which are commonly used for helper plasmids. These plasmids are capable of producing AAV in HEK293 and HEK293T cells. In addition to the wild-type promoter of each of E2A, E4orf6, Rep2, and Cap1, any of the sequences obtained from three types of promoters (UBC promoter, mPGK promoter, and EF1A promoter) having different expression intensities was linked to each gene region (see also FIG. 13).

[Table 6]

| | 1 st | 2nd | 3rd | 4th | 5th |
|---|---|---|---|---|---|
| Seed plasmid No. | VA | E2A-SV40 polyA signal | E4orf6-bGH polyA signal | Cap1-hGH polyA signal | Rep2-HSV polyA signal |
| #14 | WT_endogenous promoter | WT_E2A promoter | WT_E4 promoter | WT_Cap1 promoter | WT_Rep2 promoter |
| #15 | WT_endogenous promoter | WT_E2A promoter | EF1A promoter | mPGK promoter | UBC promoter |
| #16 | WT_endogenous promoter | UBC promoter | WT_E4 promoter | EF1A promoter | mPGK promoter |
| #17 | WT_endogenous promoter | mPGK promoter | UBC promoter | WT_Cap1 promoter | EF1A promoter |
| #18 | WT_endogenous promoter | EF1A promoter | mPGK promoter | UBC promoter | WT_Rep2 promoter |

Here, VA, each gene, and the polyA signal sequence downstream thereof are commonly designed between seed plasmids, and the target of the combinatorial library by the CombiOGAB method is a promoter portion. A restriction enzyme SfiI recognition site (5'-GGCCnNNN/nGGCC-3'; n and N are any of A, T, G, and C; capital letters N are 3' end protruding portions) was located at the boundary of each region. Each 3' end protruding of the three bases generated when each SfiI recognition site was cleaved was designed to be the only sequence in the plasmid. In the combinatorial library production

by the CombiOGAB method, the order and direction of the adjacent DNA fragments are specified by the complementarity of the protruding portions. Thus, the seed plasmids were designed so that, for all types of seed plasmids, the corresponding unit DNAs at the positions of the respective genes had the same protruding sequence as illustrated in FIG. 13. Although some genes to be used naturally contain SfiI recognition sequences, such SfiI recognition sequences were deleted by replacing them with synonymous codons in the case of protein coding sequences and changing the sequences in the case of non-coding regions such as within ITR sequences.

• Preparation of integrated nucleic acids for OGAB method

The five cloning plasmids pBR-delTypeIIS-1st, pBR-delTypeIIS-2nd, pBR-delTypeIIS-3rd, pBR-delTypeIIS-4th, and pBR-delTypeIIS-5th constructed in Example 4 were used.

Among other necessary DNAs, DNAs constructed in Example 4 were used as they were. In the same manner as that of Example 4, DNAs not described in Example 4 were obtained from appropriate template DNA by a PCR method, and then cloned into pTAKN-2. For confirmation and amplification of each of promoter fragments of Cap2 and Rep1, the sets of primers set forth in SEQ ID NOs: 215 and 216 and SEQ ID NOs: 217 and 218 were used, respectively. For confirmation and amplification of ORFs of Cap2 and Rep1, the sets of primers set forth in SEQ ID NOs: 219 and 220 and SEQ ID NOs: 221 and 222 were used, respectively. As in Example 4, OGAB block plasmids were constructed to prepare the DNA fragments listed in Table 6.

• Construction of seed plasmids

The reaction was performed by the OGAB method in the same manner as that of Example 4. Plasmids with the correct structure were constructed and prepared in large quantities by ultracentrifugation.

• CombiOGAB library

Four seed plasmids #15, #16, #17, and #18 obtained by mass preparation were cleaved with the restriction enzyme SfiI, T4DNA ligase was added, the mixture was mixed and then reacted at 37°C for 2 hours, and then the resultant was used for transformation. As a result of culturing overnight at 37°C at an LB portion rate containing tetracycline, about 30,000 colonies were obtained.

• Production of rAAV from pDual promoter library

As in Example 4, 96 of these colonies were randomly selected and the plasmids were purified. A total of 97 types of plasmid DNAs (plasmids derived from Bacillus subtilis) obtained by adding seed plasmid #14 thereto were introduced into HEK293 cells, and viral particle production was evaluated.

$0.225 \times 10^6$ HEK293 cells were seeded in a 24-well plate the day before transfection. After 22 hours, the medium was replaced with 0.38 mL of DMEM medium (2% FBS, 1% penicillin/streptomycin), and culture was performed at 37°C and 5% $CO_2$ for 2 hours. 0.10 μg of pAAV-GFP plasmid, 0.34 μg of pDual library plasmid, and 20 μL of a transfection solution obtained by mixing each of PEI pro and DMEM medium in an amount of 1-fold were added thereto. As a control, 0.10 μg of pAAV-GFP plasmid, 0.12 μg of pR2C1 plasmid, 0.22 μg of pHelper plasmid, and 20 μL of a transfection solution obtained by mixing each of PEI pro and DMEM medium in an amount of 1-fold were added. After culturing at 37°C and 5% $CO_2$ for 72 hours, the supernatant was recovered and the number of genomic copies of rAAV was quantified.

• Evaluation of rAAV

A sample prepared by subjecting the supernatant to DNaseI treatment for 30 minutes and reacting the treated supernatant at 95°C for 10 minutes was diluted 1,000 times and subjected to qPCR. Here, primer sequences of 5'-CATCAATGGGCGTGGATAGC-3' (SEQ ID NO: 213) and 5'-GGAGTTGTTACGACATTTTGGAAA-3' (SEQ ID NO: 214) targeting the CMV promoter were used. As the reaction conditions, after 10 minutes at 95°C, 40 cycles of 95°C/15 seconds, 55°C/5 seconds, and 72°C/30 seconds were repeated.

The results are illustrated in FIG. 14. Clones with improved Titer were identified compared to the seed plasmid #14 composed of the WT promoter.

• Identification of selected rAAV promoters

The clone (pDual clone #15) having the largest viral genome amount was selected, and the base sequence of the plasmid used for the viral vector production was determined as follows to identify the combination of promoters. Plasmid

DNAs were fragmented using Illumina prep (Illumina, Inc.), and base sequence analysis was performed using Miseq (Illumina, Inc.).

Each plasmid used in Examples above was obtained or produced as follows.

- pAAV-GFP
  (pAAV[Exp]-CMV>EGFP:WPRE) purchased from VectorBuilder Inc.

- VB3_pAAV-EF1A-dTomato-T2A-Hygro(VB)
  (pAAV[Exp]-EF1A>dTomato(ns):T2A:Hygro) purchased from VectorBuilder Inc.

- VB5_pAAV-mPGK-mCherry-oPRE(VB)
  (pAAV[Exp]-mPGK>mCherry:oPRE) purchased from VectorBuilder Inc.

- VB6_pAAV-UBC-EBFP(VB)
  (pAAV[Exp]-UBC>EBFP) purchased from VectorBuilder Inc.

- VB7_VB211117-TRE-tTA
  (pRP[Exp]-TRE>tTA) purchased from VectorBuilder Inc.

- 6230_pAAV-CMV
  (6230_pAAV-CMV) purchased from Takara Bio Inc.

- SYNp34_pHelper
  pUC19 vector plasmid (Addgene: #500005) was treated with restriction enzymes (AatII, PciI). The following three fragments (VA fragment, E4 fragment, and E2A fragment) were inserted thereinto using InFusion. VA fragments were prepared using the genome of Ad5 (see ATCC VR-1516) as a template and using the following primers:primer sequences 5’-ttagtaagcttccctcctgacgcggtaggaggaggggagggtgccctgcatg-3’ (SEQ ID NO: 223) and 5’-ccttttgctcacatgtcaattggtagatgtacctggacatccaggtgatgccggcg-3’ (SEQ ID NO: 224). E4 fragments were prepared using the genome of Ad5 as a template and using the following primers:primer sequences 5’-tgtacaGGCGCGCCgaattcgttttagggcggagtaacttgtatgtgttgggaattgtag-3’ (SEQ ID NO: 225) and 5’-agggaagcttactaaacggtacacaggaaacaggagacacaactccaagtg-3’ (SEQ ID NO: 226). E2A fragments were prepared using the genome of Ad5 as a template and using the following primers:primer sequences 5’-gaaaagtgccacctgacgtcgcggccgcGCGATCGCggtacccaactccatgctcaacagtccccaggtacagccc-3’ (SEQ ID NO: 227) and 5’-gaattcGGCGCGCCtgtacagcccgggcgaccgcaccctgtgacgaaagccgcccgcaag-3’ (SEQ ID NO: 228).

- SYNp21_pR2C1
  pUC19 vector plasmid (Addgene: #500005) was treated with restriction enzymes (AatII, PciI). The following three fragments (p5 gene fragment, AAV2 Rep fragment, and SV40 ori fragment) were inserted thereinto using InFusion. The p5 gene fragment was prepared using the following primers:primer sequences 5’-gaaaagtgccacctgacgtcgcggccgcggaggggtggagtcgtgacgtgaattacgtcatagggttagggaggtcctgtattag aggtcacgtgagtgttttgcgac-3’ (SEQ ID NO: 229) and 5’-gttcaaacctcccgcttcaaaatggagaccctgcgtgctcactcgggcttaaatacccagcgtgaccacatggtgtcgcaaaatgtc gcaaaacactca-3’ (SEQ ID NO: 230). The AAV2 Rep fragment was prepared using pRC2-mi342 (Takara Bio Inc.) as a template using the following primers:primer sequences 5’-gcgggaggtttgaacgcgcagccgccATGCCGGGGTTTTAC-3’ (SEQ **ID** NO: 231) and 5’-GATCGCAGCGCTatttaaatcatTTATTGTTCAAAGAT-3’ (SEQ ID NO: 232). The SV40 ori fragment was prepared using SV40 ori gene sequence (Toyobo Co., Ltd.) as a template and using the following primers:primer sequences 5’-aatAGCGCTGCGATCGCggcctccaaaaaagc-3’ (SEQ ID NO: 233) and 5’-cctttgctca-catgtcaattgatcccgcccctaact-3’ (SEQ ID NO: 234). The resulting plasmids were treated with restriction enzymes (SwaI, AfeI). An AAV1Cap fragment was inserted thereinto using InFusion. The AAV1 Cap fragment was prepared using pRC1 (Takara Bio Inc.) as a template using the following primers:primer sequences 5’-AACAATAAatgatttaaatcagg-3’ (SEQ ID NO: 235) and 5’-ggccGCGATCGCAGCGCTgtagccatggaaactagata-3’ (SEQ ID NO: 236).

- SYNp177_AIO_Helper-Ad5_R2C1_EGFP_wtE1_km

The plasmids were produced by gene synthesis and the OGAB method. The genes contained in these plasmids and their order are shown below:5’ ITR2 (VectorBuilder Inc.), CMV promoter (VectorBuilder Inc.), EGFP (VectorBuilder Inc.), WPRE (VectorBuilder Inc.), hGH polyA signal (Takara Bio Inc.), 3’ ITR2 (VectorBuilder Inc.), E2A (Ad5), E4 (Ad5), VA (Ad5), Cap (AAV1), Rep (AAV2), p5 (AAV2), E1A (Ad5), E1B (Ad5), pUC ori (pUC19), kanamycin resistance gene (VectorBuilder Inc.), HSV TK PolyA signal (Takara Bio Inc.), puromycin resistance gene (VectorBuilder Inc.), and mPGK

promoter (VectorBuilder Inc.). Here, an exemplary procedure of the OGAB method is shown below. The target introduced nucleic acid is divided into fragments of about 1kb or less. The ends of each fragment are designed to be unique and complementary only to the particular fragment. Primers are designed for each of the fragments thus designed, and each fragment is prepared by PCR. Each fragment is cloned into a vector plasmid, and Escherichia coli transformation is performed using each vector plasmid to obtain a clone containing the correct sequence for each fragment. Plasmids are purified from these Escherichia coli clones and the DNA concentration of the plasmid solution is determined. The respective plasmid solutions are fractionated and mixed so that the amounts of the plasmids of the respective fragments are equal. A restriction enzyme is added to the mixed solution to generate DNA fragments each having an end that is unique and complementary only to a specific fragment. Thereafter, the OGAB integration vector cleaved with the same restriction enzyme is added to the solution in such an amount that the molar concentration is matched with that of each fragment, ligation is performed, and pairs of fragments having complementary ends (including fragments of the OGAB accumulation vector) are linked. The resultant is added to Bacillus subtilis competent cells and cultured, and then, transformed colonies are obtained. The plasmids having the target structure are recovered from these colonies.

- SYNp44-2_pHelper(Ad5ΔSfiI)_km

The plasmids were produced by gene synthesis and the OGAB method. The genes contained in these plasmids and their order are shown below:E4 (Ad5), E2A (Ad5) ΔSfiI, VA (Ad5), pUC ori (pUC19), kanamycin resistance gene (VectorBuilder Inc.), and AmpR promoter (pUC19),

(Note)

Although the present disclosure has been illustrated using preferred embodiments of the present disclosure as described above, it is understood that the scope of the present disclosure should be interpreted only by the claims. It is understood that the patents, patent applications, and documents cited As used herein are to be incorporated by reference As used herein in the same manner as the contents are specifically described As used herein.

The present application claims priority to Japanese Patent Application No. 2022-76058 filed to the Japan Patent Office on May 2, 2022. The entire content thereof is incorporated herein by reference.

Industrial Applicability

According to the present disclosure, it is possible to efficiently produce various virus-derived construct plasmids, and virus-derived construct plasmids having improved performance can be efficiently obtained.

[Sequence Listing]

## Claims

**1.** A method for producing a virus-derived construct plasmid, the method comprising:

a step of preparing a set of starting plasmids containing at least of a part of nucleic acid sequences required to construct a virus-derived construct, the set of starting plasmids including a set of corresponding alternative unit nucleic acids, and the set of corresponding alternative unit nucleic acids including at least one set of corresponding alternative unit nucleic acids containing different nucleic acid sequences;
a step of treating the set of starting plasmids with a restriction enzyme and preparing a mixed solution containing cleaved alternative unit nucleic acid fragments;
a step of ligating the cleaved alternative unit nucleic acid fragments to form an integrated nucleic acid; and
a step of bringing the integrated nucleic acid into contact with a transformed organism to form a virus-derived construct plasmid.

**2.** The method according to claim 1, wherein a population of virus-derived construct plasmids is produced.

**3.** The method according to claim 1, wherein at least one of the sets of starting plasmids contains a transcriptional start sequence that functions in an animal cell, and a virus-derived construct plasmid containing the transcriptional start sequence that functions in an animal cell is produced.

**4.** The method according to claim 1, wherein the set of starting plasmids includes three or more types of starting

plasmids.

5. The method according to claim 1, wherein the set of corresponding alternative unit nucleic acids includes at least two sets of corresponding alternative unit nucleic acids containing different nucleic acid sequences.

6. The method according to claim 1, further comprising a step of selecting a virus-derived construct plasmid having an excellent desired function from a population of the formed virus-derived construct plasmids.

7. The method according to claim 6, wherein the desired function is an amount of virus-derived construct produced.

8. The method according to claim 6, wherein the desired function is an amount of impurity mixed in a target virus-derived construct composition.

9. The method according to claim 8, wherein the impurity is selected from a coat protein that does not contain a nucleic acid sequence, a coat protein that contains an incomplete nucleic acid sequence, an incomplete coat protein, and an incomplete nucleic acid sequence.

10. The method according to claim 6, wherein the desired function is a purity of a target virus-derived construct in a target virus-derived construct composition.

11. The method according to claim 6, further comprising a step of preparing a virus-derived construct plasmid containing a combination of parallel alternative unit nucleic acids of the selected virus-derived construct plasmid.

12. The method according to claim 1, wherein each of the sets of starting plasmids contains a nucleic acid sequence required to construct a virus-derived construct.

13. The method according to claim 1, wherein at least of a part of the starting plasmids are plasmids produced by the method according to claim 1.

14. The method according to claim 1, wherein the set of corresponding alternative unit nucleic acids includes at least two sets of corresponding alternative unit nucleic acids containing a nucleic acid sequence encoding at least of a part of different subtypes of the same gene.

15. The method according to claim 1, wherein each of the sets of starting plasmids contains three or more types of alternative unit nucleic acids containing at least of a part of the nucleic acid sequences required to construct a virus-derived construct.

16. The method according to claim 1, wherein the step of ligating to form the integrated nucleic acid includes adding a barcode nucleic acid.

17. The method according to claim 1, wherein each of the sets of starting plasmids contains the nucleic acid sequence required to construct a virus-derived construct of about 10 kb or more.

18. The method according to claim 1, wherein the nucleic acid sequence required to construct a virus-derived construct includes:

(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

19. The method according to claim 1, wherein the set of corresponding alternative unit nucleic acids includes a set of corresponding alternative unit nucleic acids encoding promoters whose types or directions are different.

20. The method according to claim 1, wherein the set of corresponding alternative unit nucleic acids includes a set of corresponding alternative unit nucleic acids containing a drug-responsive promoter and containing different nucleic acid sequences encoding a promoter.

21. The method according to claim 20, wherein the promoter includes a drug-responsive promoter.

22. The method according to claim 1, wherein the set of corresponding alternative unit nucleic acids includes at least two sets of corresponding alternative unit nucleic acids containing different nucleic acid sequences selected from:

(A) a nucleic acid sequence including a terminal repeat;
(B) a nucleic acid sequence encoding a packaging factor;
(C) a nucleic acid sequence encoding a structural protein;
(D) a nucleic acid sequence encoding a functional cofactor;
(E) a nucleic acid sequence encoding a promoter;
(F) a nucleic acid sequence encoding a desired gene; and
(G) a nucleic acid sequence involved in host cell biosynthesis.

23. The method according to claim 22, wherein the nucleic acid sequence involved in host cell biosynthesis contains a gene selected from the group consisting of SV40 Large T antigen (SV40LTA), TAX, Kras, Myc, MIR17HG, BCL-2, BCL-XL, PDIA2, XBP1, HSPA5, pyruvate carboxylase (PC), PDK, HIF1A, NRF2, and CPSF6.

24. The method according to claim 1, wherein the set of corresponding alternative unit nucleic acids includes at least two sets of corresponding alternative unit nucleic acids containing different nucleic acid sequences encoding a desired gene.

25. The method according to claim 24, wherein the set of corresponding alternative unit nucleic acids includes a set of corresponding alternative unit nucleic acids containing different nucleic acid sequences encoding a desired gene, and a set of corresponding alternative unit nucleic acids that are located adjacently upstream thereof and contain different nucleic acid sequences encoding a promoter.

26. The method according to claim 24, wherein the starting plasmid contains a nucleic acid sequence including two terminal repeats, and an alternative unit nucleic acid encoding the desired gene is present between the nucleic acid sequences including two terminal repeats.

27. The method according to claim 1, wherein the virus-derived construct is selected from a viral vector, a virus-like particle (VLP), an oncolytic virus, and a viral replicon.

28. The method according to claim 1, wherein the virus-derived construct is a virus-derived construct of a virus selected from the group consisting of an alphavirus, a vaccinia virus, a measles virus, an influenza virus, a vesicular stomatitis virus, a coronavirus, a Sindbis virus, a Semliki Forest virus, a herpes simplex virus, a retrovirus, a lentivirus, a rabies virus, a Sendai virus, an adeno-associated virus, an adenovirus, a reovirus, a coxsackievirus, and a Newcastle disease virus.

29. The method according to claim 1, wherein the virus-derived construct is a virus-derived construct of a virus selected from the group consisting of an adeno-associated virus, an adenovirus, a retrovirus, a lentivirus, a herpes simplex virus, and a Sendai virus.

30. The method according to claim 1, wherein the virus-derived construct is a virus-derived construct of a virus selected from the group consisting of an adeno-associated virus or a lentivirus.

31. The method according to claim 1, wherein the virus-derived construct is a viral vector of an adeno-associated virus.

32. The method according to claim 31, wherein the set of corresponding alternative unit nucleic acids includes a set of corresponding alternative unit nucleic acids containing different inverted terminal repeats (ITRs) derived from any of adeno-associated virus serotypes 1 to 12 and variants thereof.

33. The method according to claim 31, wherein the starting plasmid contains a promoter, a desired gene, and a terminator from upstream between a 5' ITR and a 3' ITR.

34. The method according to claim 31, wherein the set of corresponding alternative unit nucleic acids includes a set of corresponding alternative unit nucleic acids containing different nucleic acid sequences encoding a functional cofactor derived from at least one of an adenovirus, a herpes virus, a papilloma virus, a bocavirus, and a simian virus.

**35.** The method according to claim 34, wherein the set of corresponding alternative unit nucleic acids includes a set of corresponding alternative unit nucleic acids containing different nucleic acid sequences encoding a functional cofactor derived from any of adenovirus serotypes 1 to 52 and variants thereof.

**36.** The method according to claim 34, wherein the functional cofactor includes at least one of adenoviruses E1A, E1B, E2A, E4, and VA, herpes viruses UL5, UL8, UL30, UL42, UL52, ICP0, ICP4, ICP8, and ICP22, papilloma viruses E1, E2, and E6, bocaviruses NP1, NS2, NS4, and BocaSR, and simian virus Large T antigen.

**37.** The method according to claim 34, wherein the set of corresponding alternative unit nucleic acids includes a set of corresponding alternative unit nucleic acids containing different nucleic acid sequences encoding the functional cofactor, and a set of corresponding alternative unit nucleic acids that are located adjacently upstream thereof and contain different nucleic acid sequences encoding a promoter.

**38.** The method according to claim 31, wherein the starting plasmid contains E2A, E4, and VA.

**39.** The method according to claim 31, wherein the set of corresponding alternative unit nucleic acids includes a set of corresponding alternative unit nucleic acids containing different nucleic acid sequences encoding rep.

**40.** The method according to claim 39, wherein the rep includes at least one of rep78, rep76, rep52, and rep40.

**41.** The method according to claim 39, wherein the set of corresponding alternative unit nucleic acids includes a set of corresponding alternative unit nucleic acids containing different nucleic acid sequences encoding the rep, and a set of corresponding alternative unit nucleic acids that are located adjacently upstream thereof and contain different nucleic acid sequences encoding a promoter.

**42.** The method according to claim 39, wherein the set of corresponding alternative unit nucleic acids encoding rep includes a set of corresponding alternative unit nucleic acids containing different nucleic acid sequences derived from any of adeno-associated virus serotypes 1 to 12 and variants thereof.

**43.** The method according to claim 31, wherein the set of corresponding alternative unit nucleic acids includes a set of corresponding alternative unit nucleic acids containing different nucleic acid sequences encoding cap.

**44.** The method according to claim 43, wherein the cap includes at least one of VP1, VP2, VP3, AAP, and MAAP.

**45.** The method according to claim 43, wherein the set of corresponding alternative unit nucleic acids includes a set of corresponding alternative unit nucleic acids containing different nucleic acid sequences encoding the cap, and a set of corresponding alternative unit nucleic acids that are located adjacently upstream thereof and contain different nucleic acid sequences encoding a promoter.

**46.** The method according to claim 43, wherein the set of corresponding alternative unit nucleic acids encoding cap includes a set of corresponding alternative unit nucleic acids containing different nucleic acid sequences derived from any of adeno-associated virus serotypes 1 to 12 and variants thereof.

**47.** The method according to claim 31, wherein the starting plasmid contains a 5' ITR, a promoter, a desired gene, a 3' ITR, rep, cap, and a functional cofactor.

**48.** The method according to claim 31, wherein the starting plasmid contains a 5' ITR, a 3' ITR, rep, and cap, one of the rep and the cap is located downstream of a region sandwiched between the 5' ITR and the 3' ITR, and the other one of the rep and the cap is located upstream of the region sandwiched between the 5' ITR and the 3' ITR.

**49.** The method according to claim 31, wherein the starting plasmid contains two ITRs and another portion of the nucleic acid sequence required to construct a virus-derived construct, and the another portion is outside a region sandwiched between the two ITRs.

**50.** The method according to claim 31, wherein the starting plasmid contains a 5' ITR, a promoter, a desired gene, a 3' ITR, rep, cap, and a functional cofactor in this order.

**51.** The method according to claim 31, wherein the starting plasmid contains a first promoter, a first rep, a second

promoter, a second rep, a third promoter, and cap in this order.

**52.** The method according to claim 1, wherein the virus-derived construct is a viral vector of a lentivirus.

**53.** The method according to claim 52, wherein the set of corresponding alternative unit nucleic acids includes a set of corresponding alternative unit nucleic acids containing different long terminal repeats (LTRs) derived from a lentivirus or variants thereof.

**54.** The method according to claim 52, wherein the starting plasmid contains a promoter, a desired gene, and a terminator from upstream between a 5' LTR and a 3' LTR.

**55.** The method according to claim 54, wherein the starting plasmid contains two or more sets of the promoter, the desired gene, and the terminator.

**56.** The method according to claim 52, wherein the set of corresponding alternative unit nucleic acids includes a set of corresponding alternative unit nucleic acids containing different nucleic acid sequences encoding a functional cofactor selected from tat and rev.

**57.** The method according to claim 56, wherein the set of corresponding alternative unit nucleic acids includes a set of corresponding alternative unit nucleic acids containing different nucleic acid sequences encoding the functional cofactor, and a set of corresponding alternative unit nucleic acids that are located adjacently upstream thereof and contain different nucleic acid sequences encoding a promoter.

**58.** The method according to claim 52, wherein the set of corresponding alternative unit nucleic acids includes a set of corresponding alternative unit nucleic acids containing different nucleic acid sequences encoding a structural protein selected from gag, pol, VSV-G, and env.

**59.** The method according to claim 58, wherein the set of corresponding alternative unit nucleic acids includes a set of corresponding alternative unit nucleic acids containing different nucleic acid sequences encoding the structural protein, and a set of corresponding alternative unit nucleic acids that are located adjacently upstream thereof and contain different nucleic acid sequences encoding a promoter.

**60.** The method according to claim 1, wherein the virus-derived construct is a viral vector of an adenovirus.

**61.** The method according to claim 60, wherein the set of corresponding alternative unit nucleic acids includes a set of corresponding alternative unit nucleic acids containing different inverted terminal repeats (ITRs) derived from an adenovirus.

**62.** The method according to claim 60, wherein the starting plasmid contains a promoter, a desired gene, and a terminator from upstream between a 5' ITR and a 3' ITR.

**63.** The method according to claim 60, wherein the starting plasmid contains two or more sets of the promoter, the desired gene, and the terminator.

**64.** The method according to claim 60, wherein the set of corresponding alternative unit nucleic acids includes a set of corresponding alternative unit nucleic acids containing different nucleic acid sequences encoding a structural protein selected from L1, L2, L3, L4, and L5.

**65.** The method according to claim 64, wherein the set of corresponding alternative unit nucleic acids includes a set of corresponding alternative unit nucleic acids containing different nucleic acid sequences encoding the structural protein, and a set of corresponding alternative unit nucleic acids that are located adjacently upstream thereof and contain different nucleic acid sequences encoding a promoter.

**66.** The method according to claim 64, wherein the set of corresponding alternative unit nucleic acids encoding a structural protein includes a set of corresponding alternative unit nucleic acids containing different nucleic acid sequences derived from any of adenovirus serotypes 1 to 52 and variants thereof.

**67.** The method according to claim 60, wherein the set of corresponding alternative unit nucleic acids includes a set of

corresponding alternative unit nucleic acids containing different nucleic acid sequences encoding a functional cofactor selected from E1A, E1B, E2A, E2B, and E4.

**68.** The method according to claim 67, wherein the set of corresponding alternative unit nucleic acids includes a set of corresponding alternative unit nucleic acids containing different nucleic acid sequences encoding the functional cofactor, and a set of corresponding alternative unit nucleic acids that are located adjacently upstream thereof and contain different nucleic acid sequences encoding a promoter.

**69.** The method according to claim 67, wherein the set of corresponding alternative unit nucleic acids encoding a functional cofactor includes a set of corresponding alternative unit nucleic acids containing different nucleic acid sequences derived from any of adenovirus serotypes 1 to 52 and variants thereof.

**70.** The method according to any one of claims 1 to 69, wherein the virus-derived construct plasmid enables production of a virus-derived construct in a producer cell into which the virus-derived construct plasmid is introduced alone.

**71.** A method for constructing a virus-derived construct plasmid library by performing the method according to any one of claims 1 to 69.

**72.** A method for constructing a virus-derived construct plasmid library by performing the method according to any one of claims 1 to 69, wherein the set of corresponding alternative unit nucleic acids includes a plurality of sets of corresponding alternative unit nucleic acids containing different nucleic acid sequences, and provides 64 or more combinations of parallel alternative unit nucleic acids.

**73.** A virus-derived construct plasmid or a virus-derived construct plasmid library produced by the method according to any one of claims 1 to 69.

**74.** A virus-derived construct plasmid library comprising a virus-derived construct plasmid containing three or more alternative unit nucleic acids as a series of parallel alternative unit nucleic acids,
wherein the virus-derived construct plasmid library contains a plurality of sets of corresponding alternative unit nucleic acids containing different nucleic acid sequences, and the parallel alternative unit nucleic acids on each virus-derived construct plasmid of the plurality of sets of corresponding alternative unit nucleic acids containing different nucleic acid sequences have a length of about 10 kb or more.

**75.** A virus-derived construct plasmid library comprising a plurality of virus-derived construct plasmids, wherein different portions in a combination in the plurality of virus-derived construct plasmids contain a nucleic acid sequence that is a promoter or a functional cofactor.

**76.** The virus-derived construct plasmid library according to any one of claims 73 to 75, wherein the plurality of virus-derived construct plasmids in the virus-derived construct plasmid library contain elements that constitute the different portions in the combination, the elements being differ from one another in a serotype of a virus from which they are derived.

**77.** The virus-derived construct plasmid library according to any one of claims 73 to 76, wherein the elements that constitute the different portions in the combination of the plurality of virus-derived construct plasmids in the virus-derived construct plasmid library contain promoters, and the promoters in the plurality of virus-derived construct plasmids include promoters operably linked to genes that are different from each other in nature.

**78.** The virus-derived construct plasmid library according to claim 74, wherein the plurality of sets of corresponding alternative unit nucleic acids containing different nucleic acid sequences include a set of corresponding alternative unit nucleic acids containing at least one different nucleic acid sequence of:

(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

**79.** The virus-derived construct plasmid library according to claim 74, wherein each virus-derived construct plasmid contains:

(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

**80.** The virus-derived construct plasmid library according to claim 74, wherein the plurality of sets of corresponding alternative unit nucleic acids containing different nucleic acid sequences contain an alternative unit nucleic acid having a GC content of 75% or more.

**81.** The virus-derived construct plasmid library according to claim 74, wherein the plurality of sets of corresponding alternative unit nucleic acids containing different nucleic acid sequences include a plurality of sets of corresponding alternative unit nucleic acids that allow for 64 or more different parallel alternative unit nucleic acid combinations, and each virus-derived construct plasmid contains the same number of parallel alternative unit nucleic acids.

**82.** The virus-derived construct plasmid library according to claim 74, wherein each virus-derived construct plasmid contains a barcode nucleic acid sequence.

**83.** A virus-derived construct plasmid contained in the virus-derived construct plasmid library according to claim 74.

**84.** A method for producing a virus-derived construct, the method comprising:

a step of preparing a virus-derived construct plasmid by the method according to any one of claims 1 to 69; and
a step of introducing the virus-derived construct plasmid into a producer cell to form a virus-derived construct.

**85.** A method for producing a virus-derived construct library, the method comprising:

a step of preparing a population of virus-derived construct plasmids by the method according to any one of claims 1 to 69; and
a step of introducing the population of virus-derived construct plasmids into a producer cell and forming a virus-derived construct to construct a virus-derived construct library.

**86.** The method according to claim 85, wherein the step of introducing the virus-derived construct plasmid into the producer cell includes introducing a single virus-derived construct plasmid into a producer cell.

**87.** The method according to claim 85, wherein at least of a part of the nucleic acids contained in the virus-derived construct plasmid are incorporated into a chromosome of the producer cell.

**88.** A producer cell prepared by the method according to claim 85.

**89.** A virus-derived construct produced by the method according to claim 85.

**90.** A virus-derived construct-containing composition produced by the method according to claim 85.

[FIG. 1]

FIG. 1

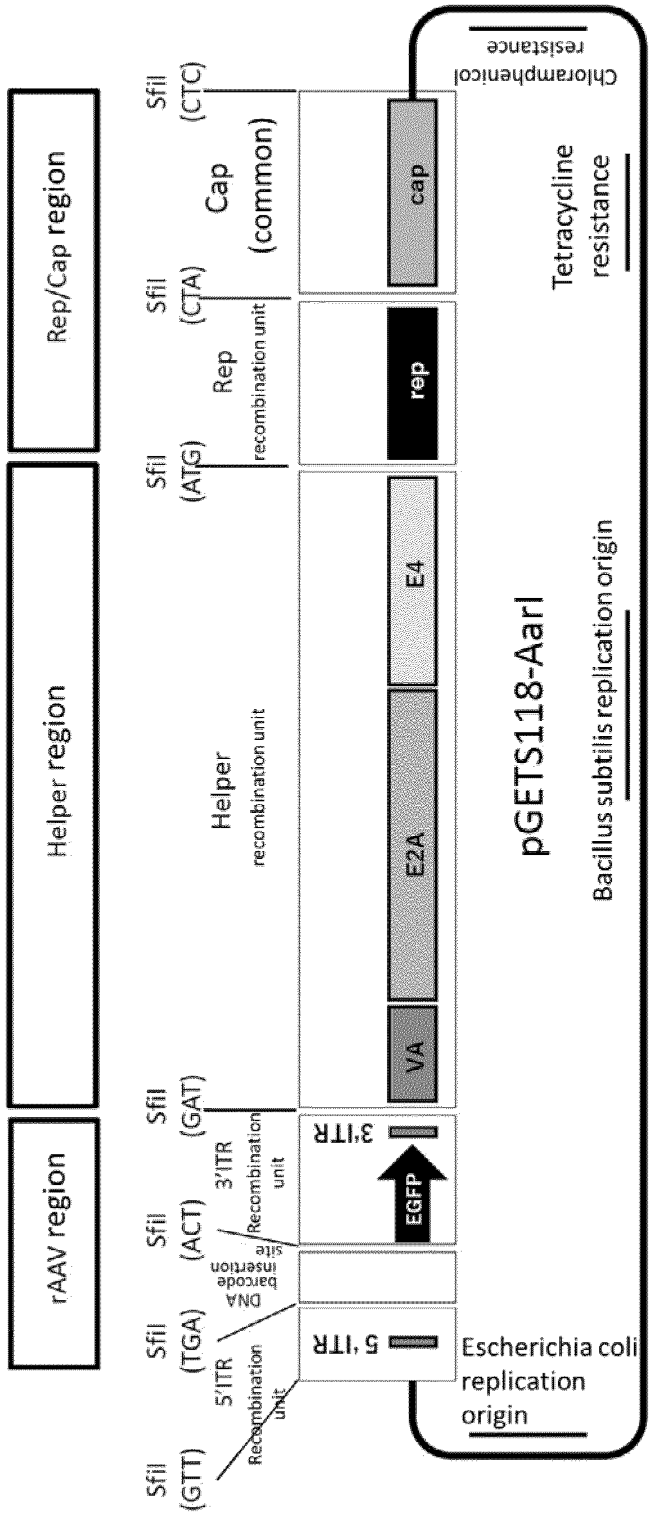
rAAV region
Helper region
Rep/Cap region
SfiI (GTT)
SfiI (TGA)
SfiI (ACT)
SfiI (GAT)
SfiI (ATG)
SfiI (CTA)
SfiI (CTC)
5'ITR Recombination unit
DNA barcode insertion site
3'ITR Recombination unit
Helper recombination unit
Rep recombination unit
Cap (common)
5'ITR
EGFP
3'ITR
VA
E2A
E4
rep
cap
Escherichia coli replication origin
pGETS118-AarI
Bacillus subtilis replication origin
Tetracycline resistance
Chloramphenicol resistance

[FIG. 2]

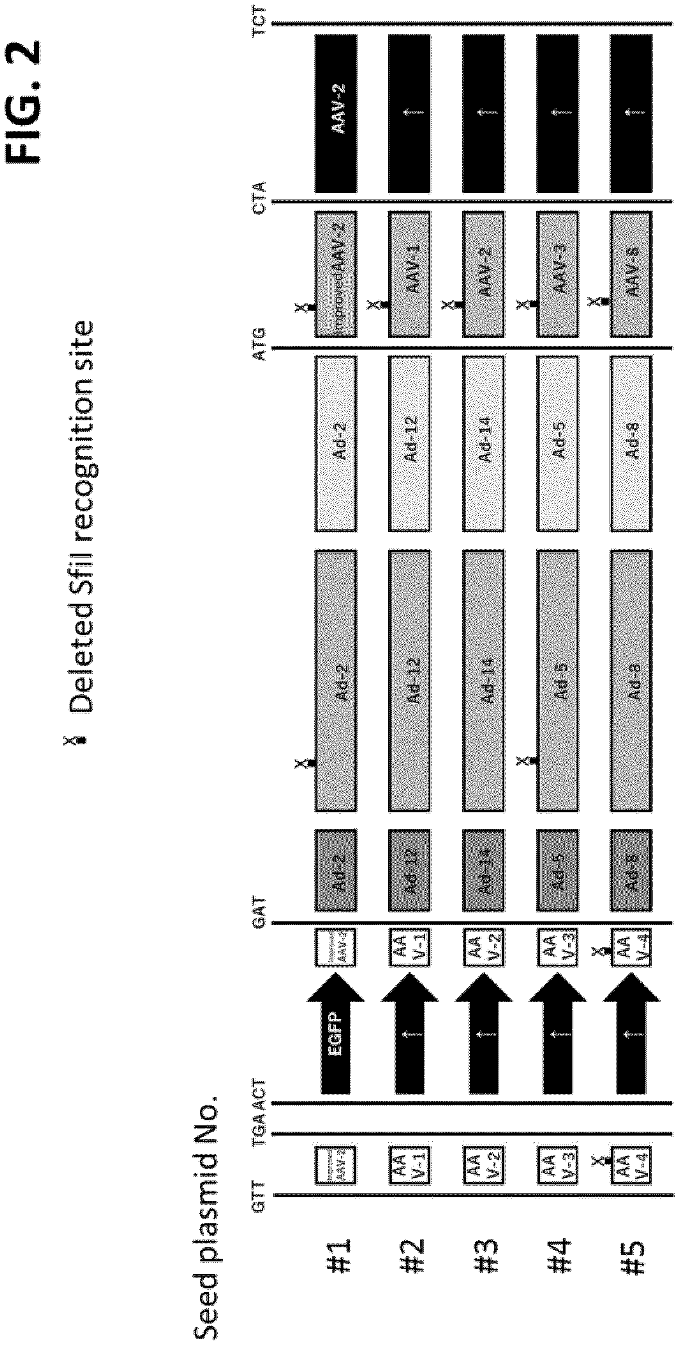
FIG. 2
Deleted SfiI recognition site
Seed plasmid No.
#1
#2
#3
#4
#5
GTT
TGA
ACT
GAT
ATG
CTA
TCT
Improved AAV-2
AA V-1
AA V-2
AA V-3
AA V-4
EGFP
Ad-2
Ad-12
Ad-14
Ad-5
Ad-8
ImprovedAAV-2
AAV-1
AAV-2
AAV-3
AAV-8

[FIG. 3]

FIG. 3

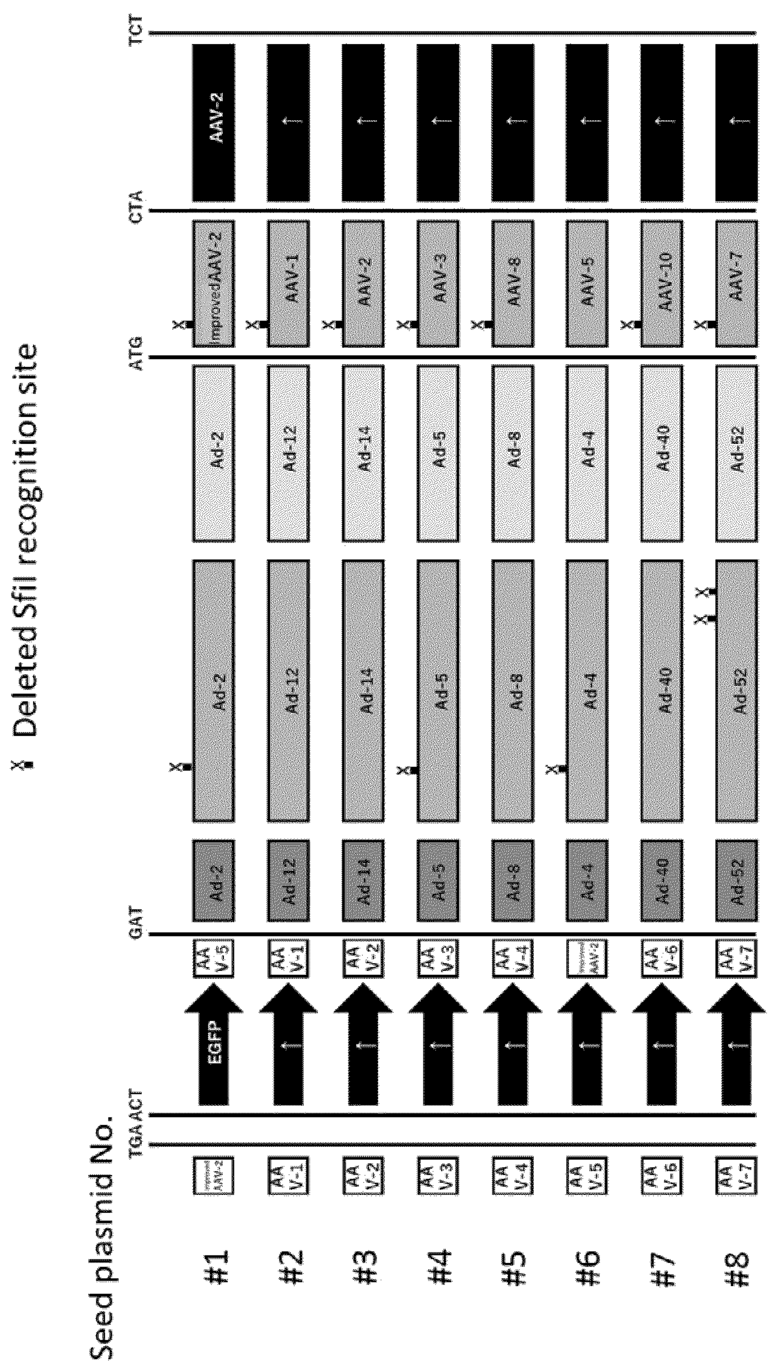
Deleted SfiI recognition site
Seed plasmid No.
TGA ACT
GAT
ATG
CTA
TCT
EGFP
#1 Improved AAV-2 AAV-5 Ad-2 Ad-2 Ad-2 Improved AAV-2 AAV-2
#2 AAV-1 AAV-1 Ad-12 Ad-12 Ad-12 AAV-1
#3 AAV-2 AAV-2 Ad-14 Ad-14 Ad-14 AAV-2
#4 AAV-3 AAV-3 Ad-5 Ad-5 Ad-5 AAV-3
#5 AAV-4 AAV-4 Ad-8 Ad-8 Ad-8 AAV-8
#6 AAV-5 Improved AAV-2 Ad-4 Ad-4 Ad-4 AAV-5
#7 AAV-6 AAV-6 Ad-40 Ad-40 Ad-40 AAV-10
#8 AAV-7 AAV-7 Ad-52 Ad-52 Ad-52 AAV-7

[FIG. 4]

FIG. 4

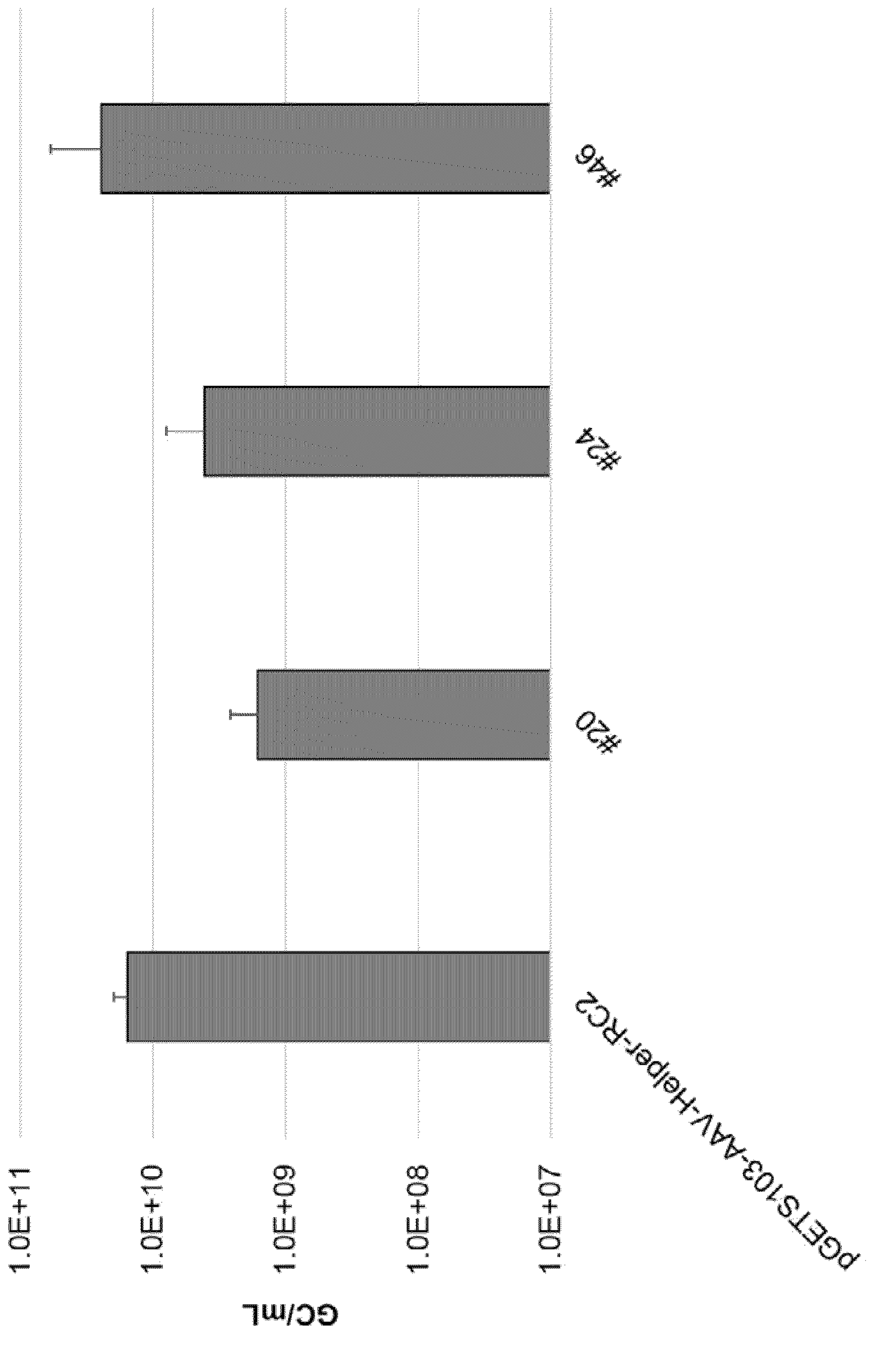
GC/mL
1.0E+11
1.0E+10
1.0E+09
1.0E+08
1.0E+07
pGETS103-AAV-Helper-RC2
#20
#24
#46

[FIG. 5]

FIG. 5

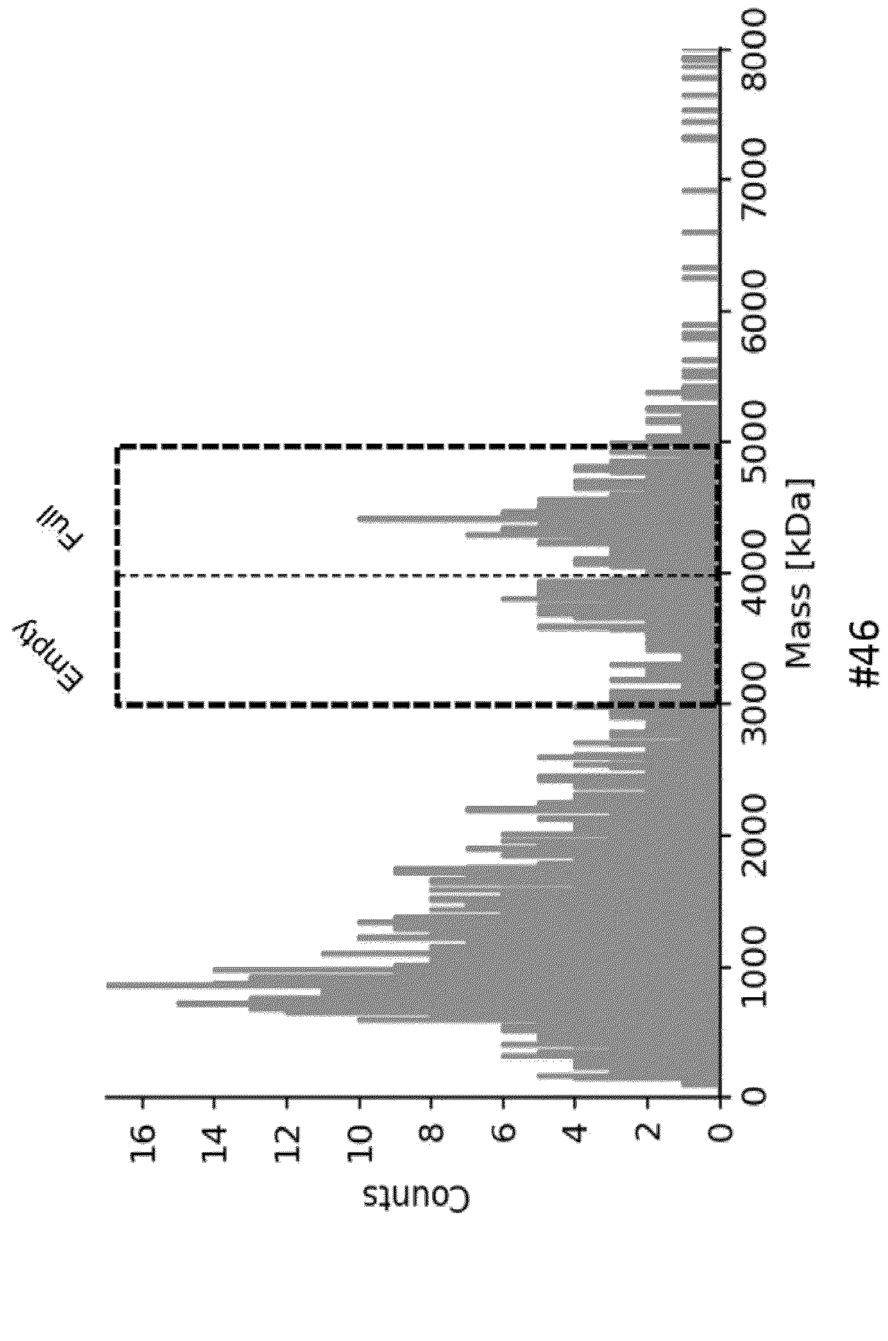
Empty
Full
Counts
0
2
4
6
8
10
12
14
16
0
1000
2000
3000
4000
5000
6000
7000
8000
Mass [kDa]
#46

[FIG. 6]

FIG. 6

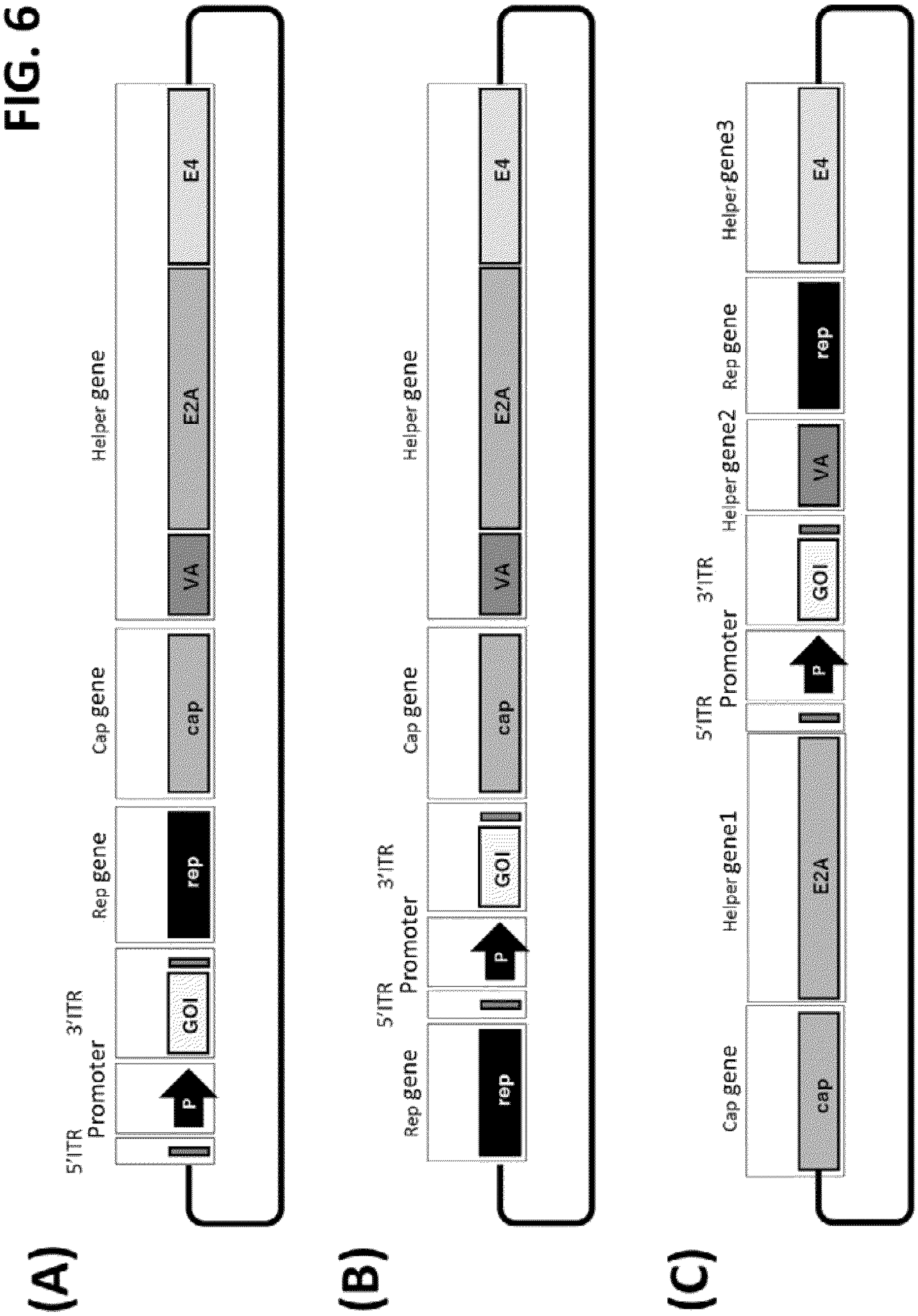
(A)
5'ITR
Promoter
3'ITR
Rep gene
Cap gene
Helper gene
P
GOI
rep
cap
VA
E2A
E4
(B)
Rep gene
5'ITR
Promoter
3'ITR
Cap gene
Helper gene
rep
P
GOI
cap
VA
E2A
E4
(C)
Cap gene
Helper gene1
5'ITR
Promoter
3'ITR
Helper gene2
Rep gene
Helper gene3
cap
E2A
P
GOI
VA
rep
E4

[FIG. 7]

FIG. 7

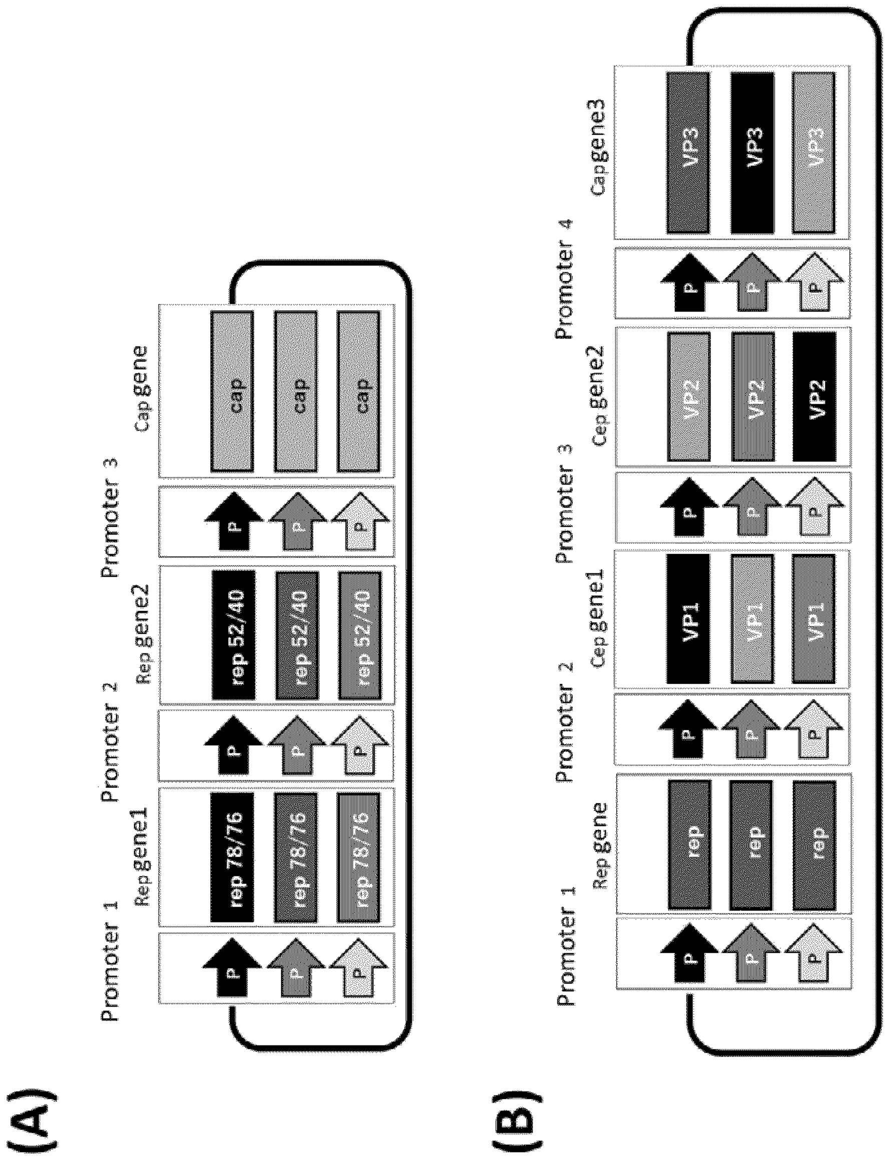
(A)
Promoter 1
Rep gene1
Promoter 2
Rep gene2
Promoter 3
Cap gene
P
rep 78/76
rep 52/40
cap
(B)
Promoter 1
Rep gene
Promoter 2
Cep gene1
Promoter 3
Cep gene2
Promoter 4
Cap gene3
rep
VP1
VP2
VP3

[FIG. 8]

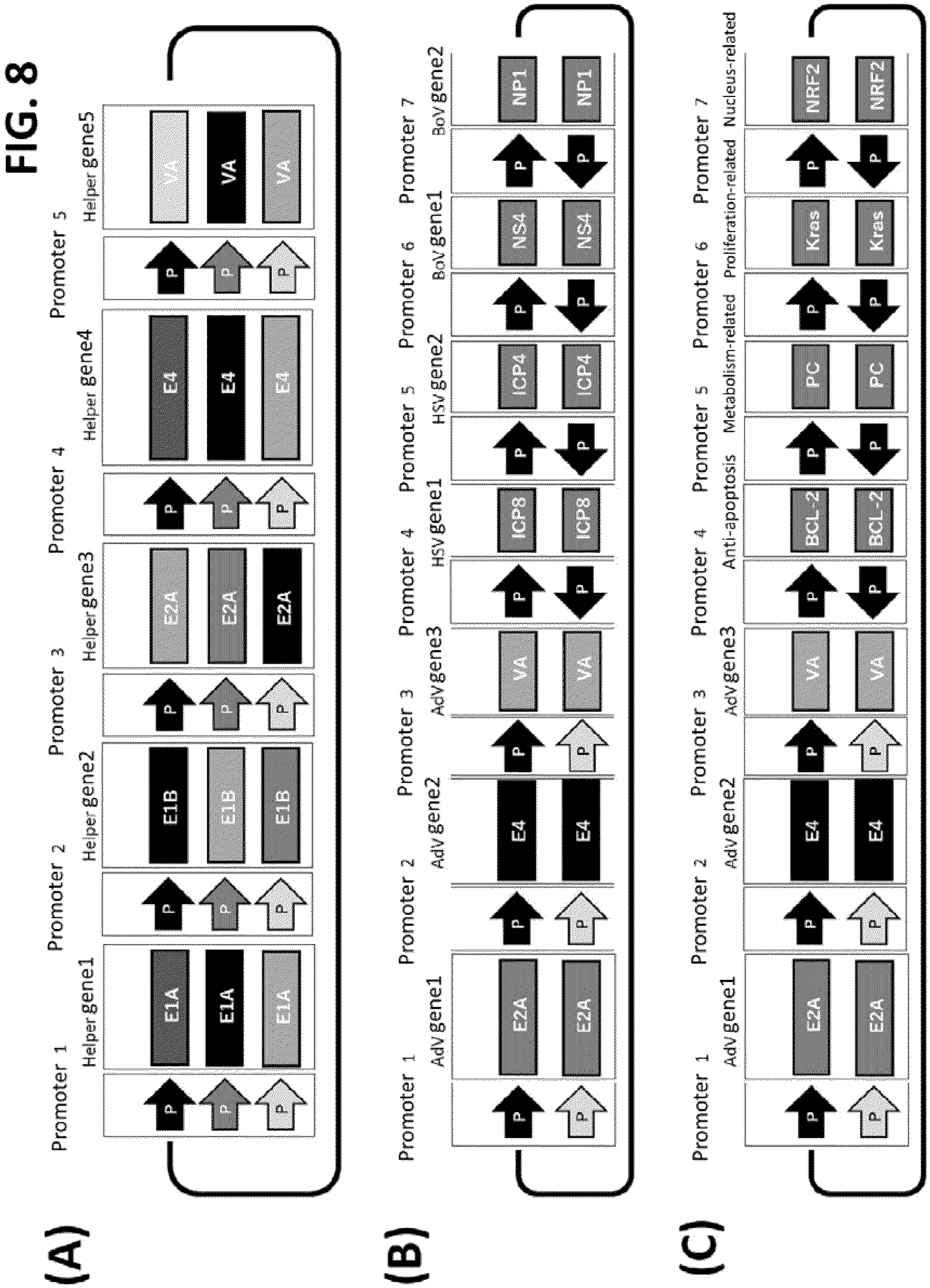
FIG. 8
(A)
Promoter 1
Helper gene1
Promoter 2
Helper gene2
Promoter 3
Helper gene3
Promoter 4
Helper gene4
Promoter 5
Helper gene5
E1A
E1B
E2A
E4
VA
P
(B)
Promoter 1
AdV gene1
Promoter 2
AdV gene2
Promoter 3
AdV gene3
Promoter 4
HSV gene1
Promoter 5
HSV gene2
Promoter 6
BoV gene1
Promoter 7
BoV gene2
E2A
E4
VA
ICP8
ICP4
NS4
NP1
(C)
Promoter 1
AdV gene1
Promoter 2
AdV gene2
Promoter 3
AdV gene3
Promoter 4
Anti-apoptosis
Promoter 5
Metabolism-related
Promoter 6
Proliferation-related
Promoter 7
Nucleus-related
E2A
E4
VA
BCL-2
PC
Kras
NRF2

[FIG. 9]

FIG. 9

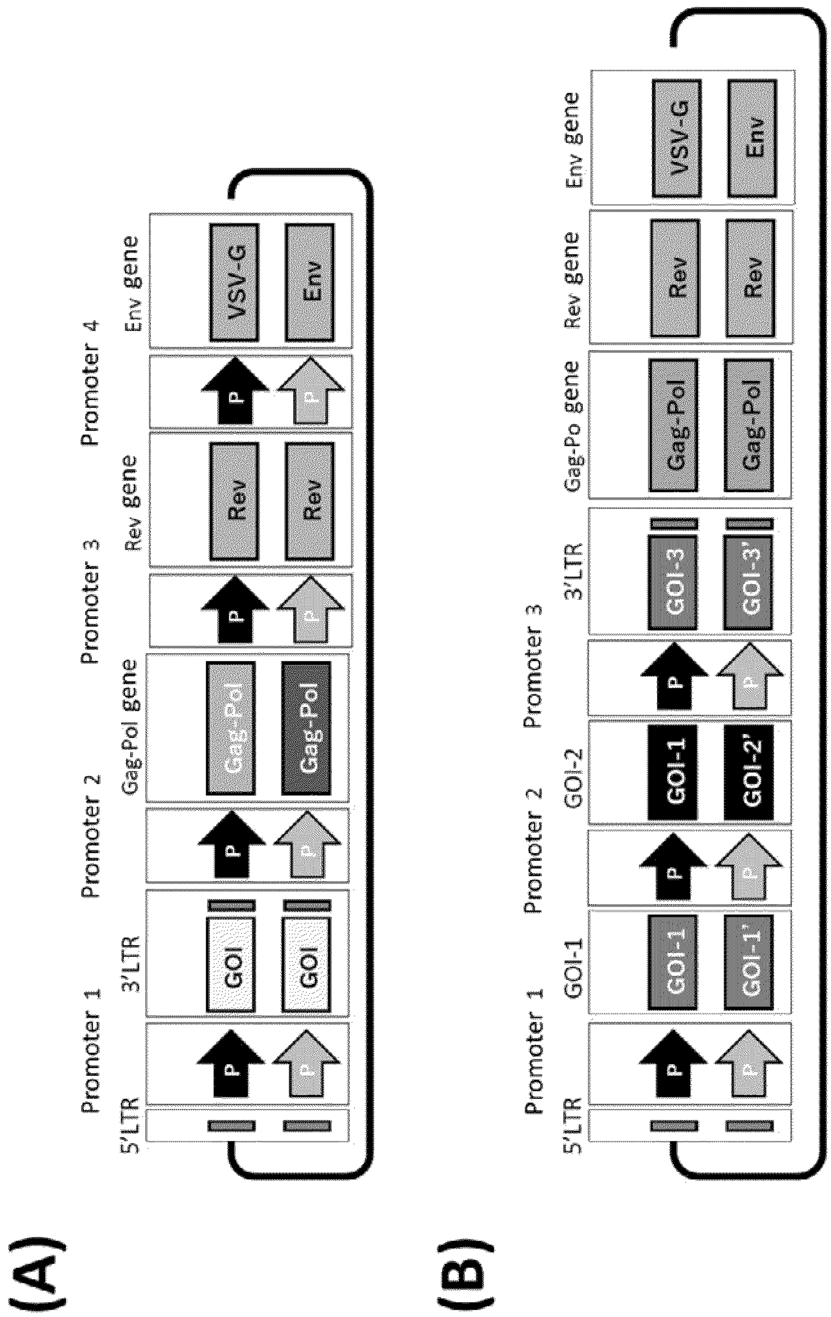
(A)
5'LTR
Promoter 1
3'LTR
Promoter 2
Gag-Pol gene
Promoter 3
Rev gene
Promoter 4
Env gene
P
GOI
Gag-Pol
Rev
VSV-G
Env
(B)
5'LTR
Promoter 1
GOI-1
Promoter 2
GOI-2
Promoter 3
3'LTR
Gag-Po gene
Rev gene
Env gene
GOI-1
GOI-1'
GOI-2'
GOI-3
GOI-3'

[FIG. 10]

FIG. 10

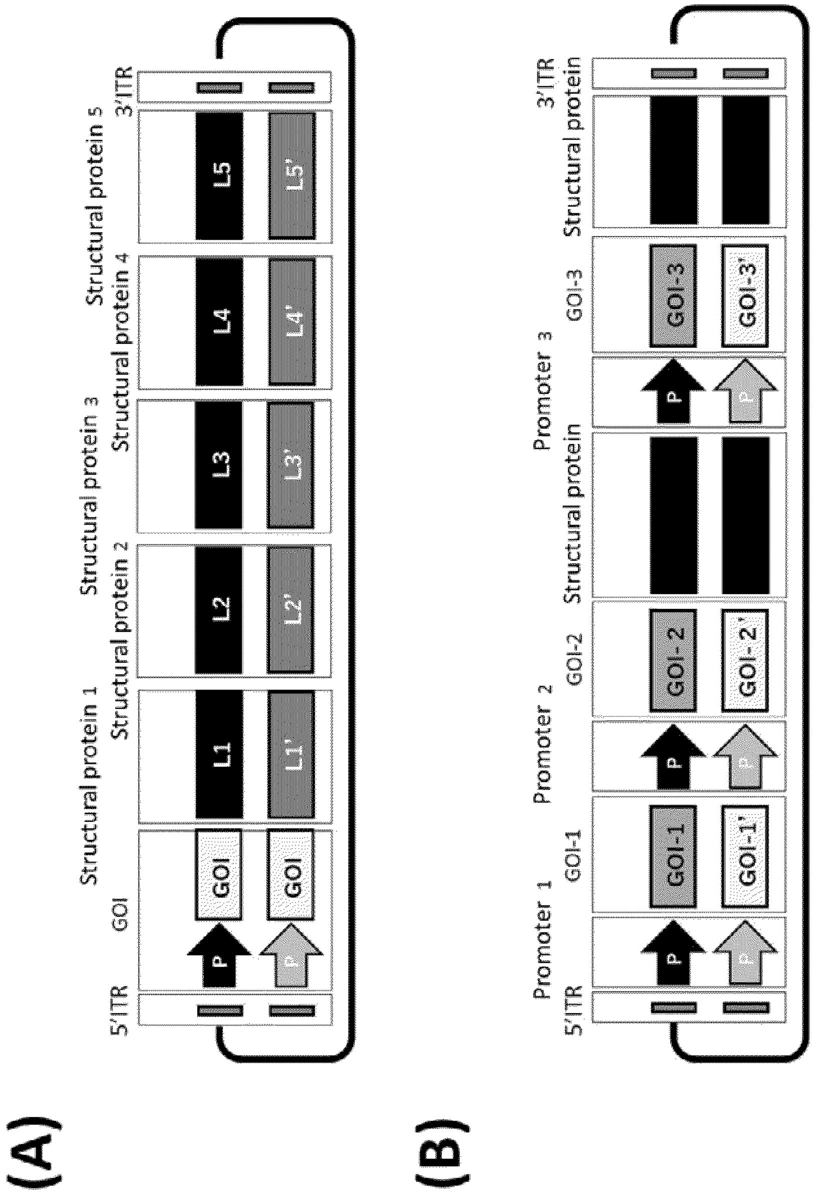
(A)
5'ITR
GOI
Structural protein 1
Structural protein 2
Structural protein 3
Structural protein 4
Structural protein 5
3'ITR
P
GOI
L1
L2
L3
L4
L5
L1'
L2'
L3'
L4'
L5'
(B)
Promoter 1
GOI-1
Promoter 2
GOI-2
Structural protein
Promoter 3
GOI-3
GOI-1'
GOI-2'
GOI-3'

[FIG. 11]

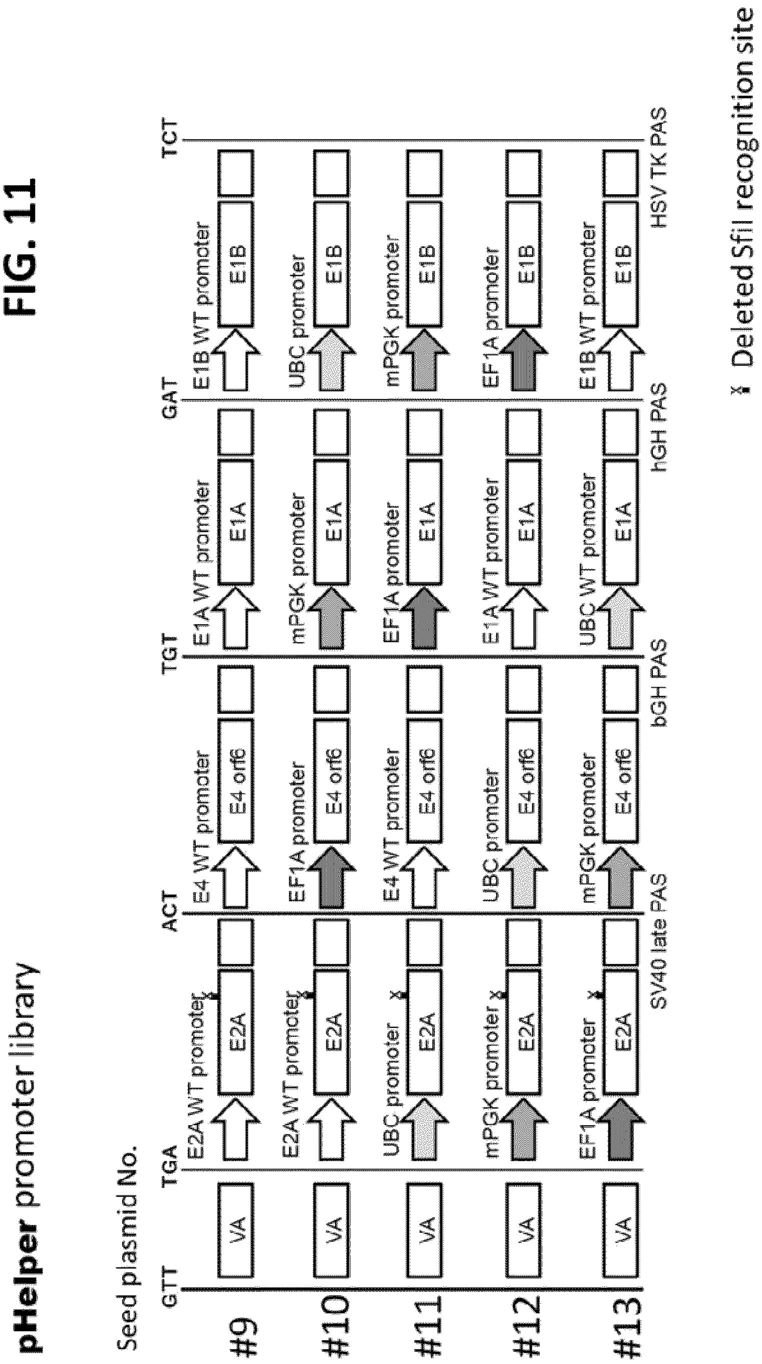
FIG. 11
pHelper promoter library
Seed plasmid No.
GTT
TGA
ACT
TGT
GAT
TCT
#9
#10
#11
#12
#13
VA
E2A WT promoter
UBC promoter
mPGK promoter
EF1A promoter
E2A
E4 WT promoter
EF1A promoter
UBC promoter
mPGK promoter
E4 orf6
E1A WT promoter
mPGK promoter
EF1A promoter
UBC WT promoter
E1A
E1B WT promoter
UBC promoter
mPGK promoter
EF1A promoter
E1B
SV40 late PAS
bGH PAS
hGH PAS
HSV TK PAS
Deleted SfiI recognition site

[FIG. 12]

FIG. 12

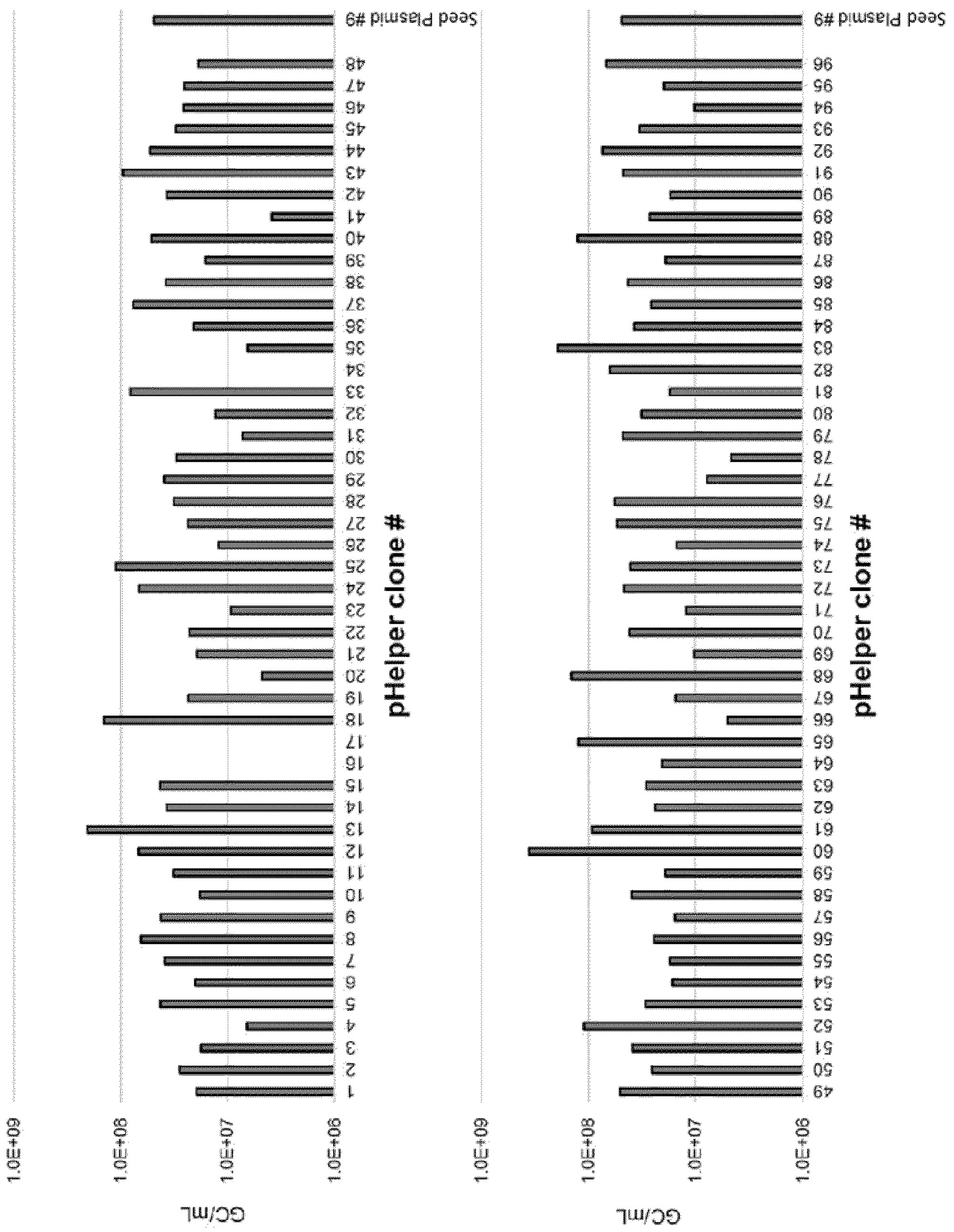
GC/mL
1.0E+06
1.0E+07
1.0E+08
1.0E+09
pHelper clone #
Seed Plasmid #9
GC/mL
1.0E+06
1.0E+07
1.0E+08
1.0E+09
pHelper clone #
Seed Plasmid #9

[FIG. 13]

FIG. 13

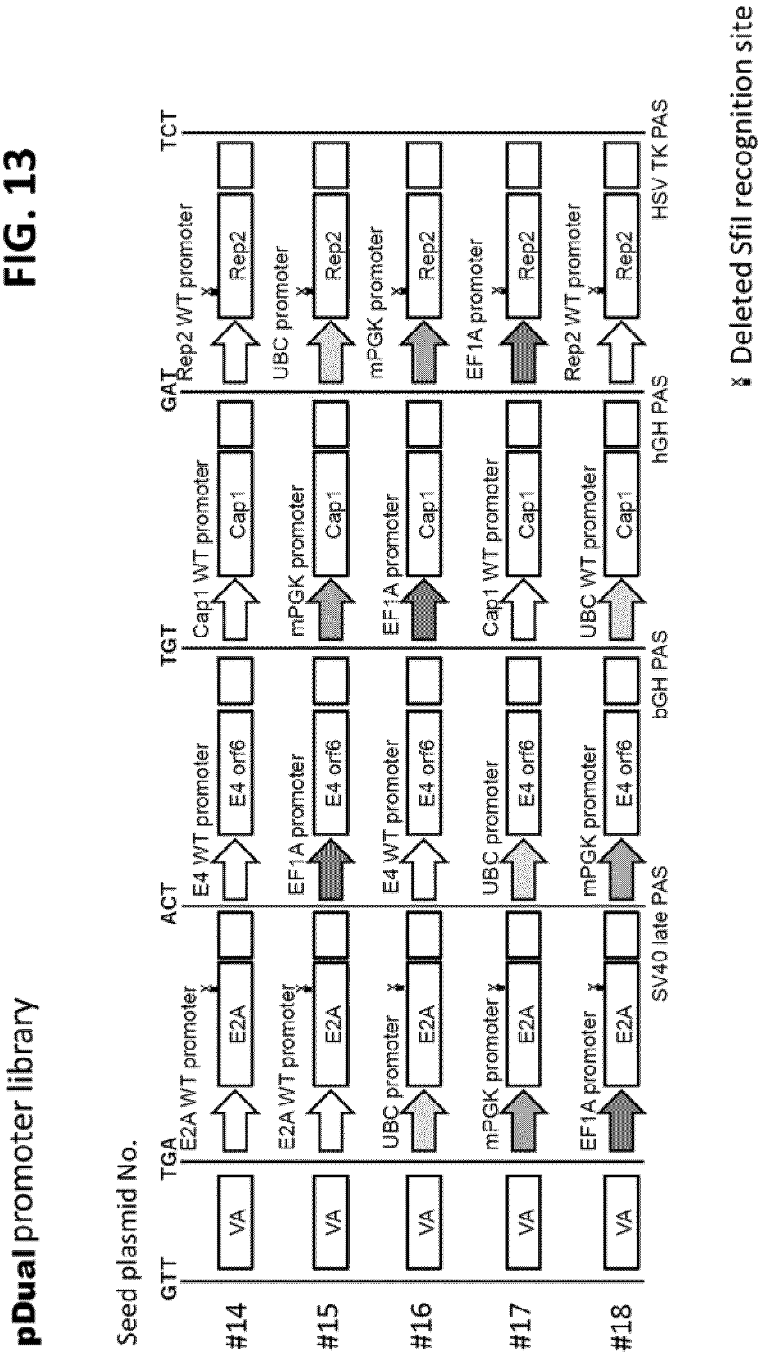
pDual promoter library
Seed plasmid No.
GTT
TGA
ACT
TGT
GAT
TCT
#14
#15
#16
#17
#18
VA
E2A WT promoter
UBC promoter
mPGK promoter
EF1A promoter
E2A
SV40 late PAS
E4 WT promoter
EF1A promoter
UBC promoter
mPGK promoter
E4 orf6
bGH PAS
Cap1 WT promoter
mPGK promoter
EF1A promoter
UBC WT promoter
Cap1
hGH PAS
Rep2 WT promoter
UBC promoter
mPGK promoter
EF1A promoter
Rep2
HSV TK PAS
Deleted SfiI recognition site

[FIG. 14]

FIG. 14

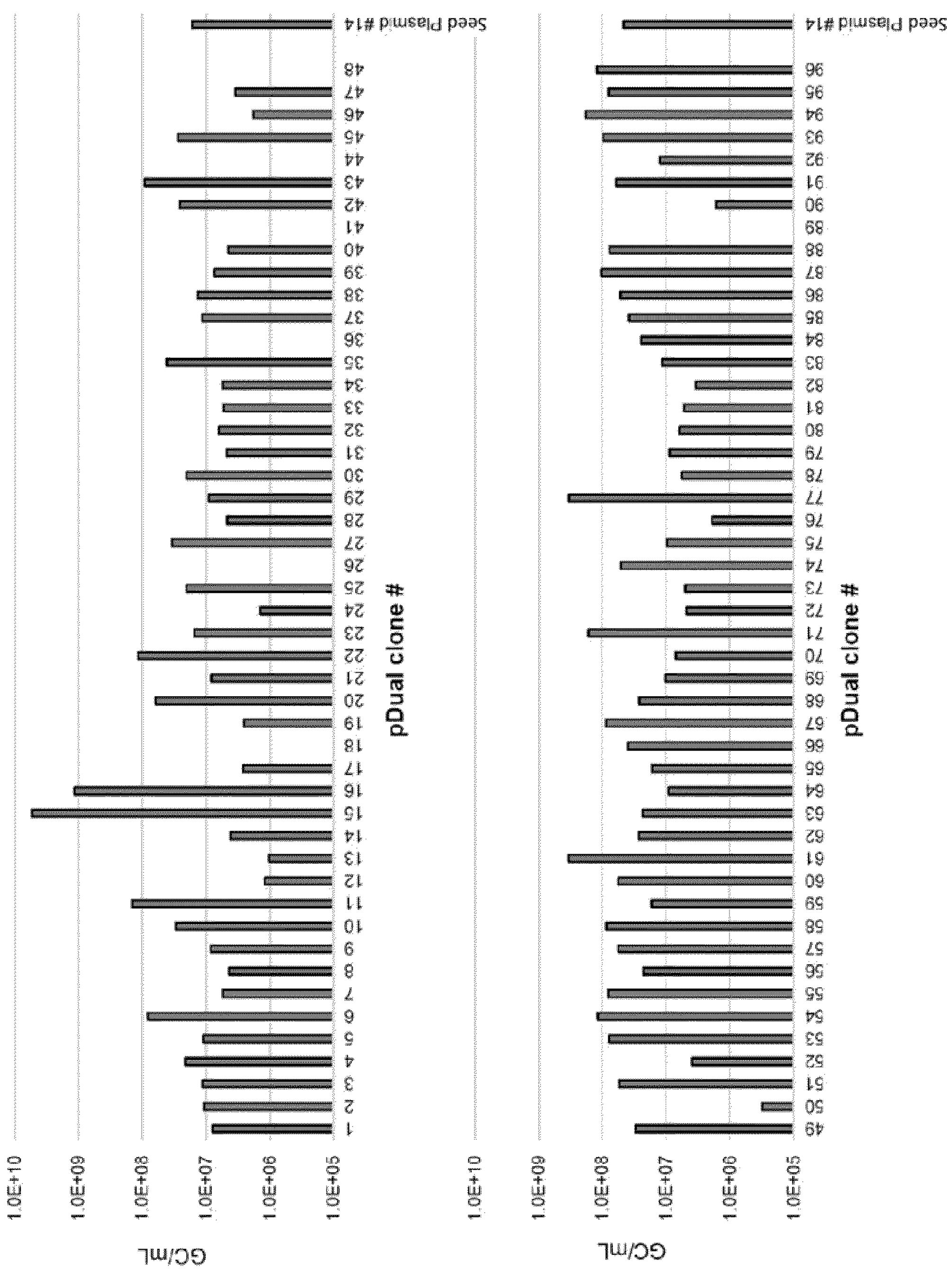
GC/mL
1.0E+10
1.0E+09
1.0E+08
1.0E+07
1.0E+06
1.0E+05
pDual clone #
Seed Plasmid #14

**INTERNATIONAL SEARCH REPORT**

International application No.
**PCT/JP2023/017130**

**A. CLASSIFICATION OF SUBJECT MATTER**

***C12N 15/86***(2006.01)i; ***A61K 35/76***(2015.01)i; ***A61K 48/00***(2006.01)i; ***A61P 43/00***(2006.01)i; ***C12N 5/10***(2006.01)i; ***C12N 7/01***(2006.01)i; ***C12N 15/12***(2006.01)i; ***C12N 15/37***(2006.01)i; ***C12N 15/48***(2006.01)i; ***C12N 15/52***(2006.01)i; ***C12N 15/63***(2006.01)i; ***C12N 15/66***(2006.01)i; ***C12N 15/861***(2006.01)i; ***C12N 15/863***(2006.01)i; ***C12N 15/864***(2006.01)i; ***C12N 15/867***(2006.01)i; ***C12N 15/869***(2006.01)i; ***C40B 40/08***(2006.01)i

FI: C12N15/86 Z ZNA; C12N15/66 Z; C12N15/63 100Z; C12N15/861 Z; C12N15/863 Z; C12N15/864 Z; C12N15/864 100Z; C12N15/867 Z; C12N15/869 Z; C12N15/37; C12N15/48; C12N15/12; C12N15/52 Z; C40B40/08; C12N5/10; C12N7/01; A61K48/00; A61P43/00 105; A61K35/76

According to International Patent Classification (IPC) or to both national classification and IPC

**B. FIELDS SEARCHED**

Minimum documentation searched (classification system followed by classification symbols)

C12N1/00-15/90; A61K38/00-51/12; C40B10/00-50/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed; Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| Y | JP 2021-502799 A (OXFORD GENETICS LIMITED) 04 February 2021 (2021-02-04)<br>claims 1, 8, 10, example 2, fig. 3 | 1-90 |
| Y | JP 2020-188763 A (JCR PHARMACEUTICALS COMPANY, LIMITED) 26 November 2020 (2020-11-26)<br>claim 1, examples | 1-90 |
| Y | WO 2020/203496 A1 (NATIONAL UNIVERSITY CORPORATION KOBE UNIVERSITY) 08 October 2020 (2020-10-08)<br>claims, abstract | 1-90 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 June 2023** | **04 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/017130**

**C. DOCUMENTS CONSIDERED TO BE RELEVANT**

| Category* | Citation of document, with indication, where appropriate, of the relevant passages | Relevant to claim No. |
|---|---|---|
| Y | TSUGE, Kenji et al. One step assembly of multiple DNA fragments with a designed order and orientation in Bacillus subtilis plasmid. Nucleic Acids Research. 2003, vol. 31, no. 21, article no. e133, DOI: 10.1093/nar/gng133<br>abstract | 1-90 |
| Y | ITAYA, Mitsuhiro, TSUGE, Kenji. Construction and Manipulation of Giant DNA by a Genome Vector. Methods in Enzymology. 20 May 2011, vol. 498, pp. 427-447, DOI: 10.1016/B978-0-12-385120-8.00019-X<br>p. 438, third paragraph to p. 443, second paragraph | 1-90 |
| A | JP 2002-528049 A (IMMUSOL INCORPORATED) 03 September 2002 (2002-09-03)<br>entire text, all drawings | 1-90 |
| A | US 2018/0155740 A1 (WUHAN INSTITUTE OF PHYSICS AND MATHEMATICS, CHINESE ACADEMY OF SCIENCES) 07 June 2018 (2018-06-07)<br>entire text, all drawings | 1-90 |
| A | JP 2021-510496 A (OXFORD GENETICS LIMITED) 30 April 2021 (2021-04-30)<br>entire text, all drawings | 1-90 |
| P, X | WO 2022/097646 A1 (SYNPLOGEN COMPANY, LIMITED) 12 May 2022 (2022-05-12)<br>claims | 1-90 |

Form PCT/ISA/210 (second sheet) (January 2015)

INTERNATIONAL SEARCH REPORT

International application No.
**PCT/JP2023/017130**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   “The form of Annex C/ST.25 text file” above shall read as “the form of ST.26.”.

**INTERNATIONAL SEARCH REPORT**
**Information on patent family members**

International application No.
**PCT/JP2023/017130**

| Patent document cited in search report | Publication date (day/month/year) | Patent family member(s) | Publication date (day/month/year) |
|---|---|---|---|
| JP 2021-502799 A | 04 February 2021 | US 2020/0277629 A1<br>claims 1, 8, 10, example 2, fig. 3<br>EP 3684790 A1<br>CN 111108116 A<br>KR 10-2020-0056429 A | |
| JP 2020-188763 A | 26 November 2020 | (Family: none) | |
| WO 2020/203496 A1 | 08 October 2020 | US 2022/0177871 A1<br>claims, abstract<br>EP 3951028 A1<br>KR 10-2021-0144816 A<br>CN 113728130 A | |
| JP 2002-528049 A | 03 September 2002 | US 2003/0096399 A1<br>whole document<br>EP 1097244 A1 | |
| US 2018/0155740 A1 | 07 June 2018 | CN 106544325 A<br>whole document | |
| JP 2021-510496 A | 30 April 2021 | US 2021/0163987 A1<br>whole document<br>EP 3697917 A1<br>CN 111511922 A<br>KR 10-2020-0120613 A | |
| WO 2022/097646 A1 | 12 May 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2001090392 A
- US 5908845 A
- WO 2015111248 A
- JP 2019198236 A
- WO 2020203496 A
- JP 2022076058 A


### Non-patent literature cited in the description


- **AUSUBEL et al.** Current Protocols in Molecular Biology. Wiley Interscience Publishers, 1995
- Molecular Cloning 2nd ed., Current Protocols in Molecular Biology. DNA Cloning 1: Core Techniques, A Practical Approach. Oxford University Press, 1995
- **SAMBROOK et al.** Molecular Cloning: A Laboratory Manual, # 3. Cold Spring Harbor Laboratory Press, 2001, vol. 1, 7.42-7.45
- **IMANAKA, T et al.** *J. Gen. Microbioi*, 1984, vol. 130, 1399-1408
- **TANAKA, T** ; **OGRA, M**. *FEBS Lett.*, 1998, vol. 422, 243-246
- **SWINFIELD, T. J et al.** *Gene*, 1990, vol. 87, 79-90
- **YANSURA, D** ; **HENNER, D**. *J. Pro. Natl. Acad. Sci, USA*, 1984, vol. 81, 439-443
- **ITAYA, M.** *Biosci. Biotechnol. Biochem.*, 1999, vol. 63, 602-604
- **ANAGNOSTOPOULOU, C.** ; **SPIZEN**. *J. J. Bacteriol.*, 1961, vol. 81, 741-746
- **IMANAKA, T et al.** *J. Gen. Microbioi.*, 1984, vol. 130, 1399-1408
- **TANAKA, T** ; **OGRA, M**. *FEBS Lett*, 1998, vol. 422, 243-246
- **E. W. MARTIN**. Remington's Pharmaceutical Sciences. Mark Publishing Company
- **SAMBROOK, J et al.** Molecular Cloning: A Laboratory Manual.. Cold Spring Harbor Laboratory Press, 1989
- **TSUGE et al.** *Scientific Reports*, vol. 5, 10655